# EUROPEAN PATENT APPLICATION

(11) **EP 4 455 161 A1**
(43) Date of publication of application: **30.10.2024**
(21) Application number: 23169571.9
(22) Date of filing: 24.04.2023
(51) Int. Cl.: C07K 16/18, A61P 25/28, A61K 39/395

(54) **HUMAN-DERIVED ANTI-COLLAPSIN RESPONSE MEDIATOR PROTEIN 2 (CRMP2) ANTIBODIES**

(71) Applicant: ONO Pharmaceutical Co., Ltd., Osaka-shi, Osaka 541-8526 (JP)
(72) Inventor: The designation of the inventor has not yet been filed
(74) Representative: J A Kemp LLP

(57) **Abstract**

Collapsin Response Mediator Protein 2 (CRMP2) is established as a therapeutic target in neurodegenerative disorders. Provided are novel human-derived anti-CRMP2 antibodies as well as fragments, derivatives and variants thereof. Polynucleotides, vectors, host cells and kits related to the CRMP2 specific antibodies are also provided. The antibodies, immunoglobulin chain(s), as well as binding fragments, derivatives and variants thereof can be used in pharmaceutical compositions for CRMP2 targeted therapy.

## Description

### FIELD OF THE INVENTION

The present invention generally relates to novel human-derived anti-Collapsin Response Mediator Protein 2 (CRMP2) antibodies and equivalent antigen-binding molecules, as well as to their use, in particular, to their use as medicament for the treatment of neurodegenerative disorders.

### BACKGROUND OF THE INVENTION

The pathogenesis of neurodegenerative disorders is highly complex and diverse. Neurodegenerative disorders are chronic disorders characterized by progressive loss of neurons in the brain and spinal cord, wherein a prominent and most prevalent group represents dementia-associated tauopathies, like Alzheimer's disease (AD), Pick's disease (PiD), progressive supranuclear palsy (PSP), and frontotemporal dementia (FTD). The underlying biology of these disorders includes for example aggregation of soluble amyloid species into insoluble amyloid plaques as observed in Alzheimer's disease (AD), and hyperphosphorylation of Tau with formation of intracellular neurofibrillary tangles as observed in AD, PiD, PSP, and FTD, as well as neuronal death along with a variety of related processes including neuroinflammation, synaptic and circuit dysfunction, mitochondrial and bioenergetic disorders. Among the earliest neuropathological changes in AD is the degeneration of neurons likely arising from the deposition of β-amyloid plaques as well as neurofibrillary tangles in the brain. Neuritic degeneration, including neuronal dysfunction and loss of functional synapses, can lead to cognitive and memory dysfunction, predominantly in elderly people.

As recently summarized by Yu et al. in "Novel Therapeutic Approaches for Alzheimer's Disease: An Updated Review". Int. J. Mol. Sci. 2021 Jul 30;22(15):8208. doi: 10.3390/ijms22158208, despite the profound and chronic effects of AD, current treatments are unable to achieve satisfactory therapeutic effects or stop disease progression. Today, only six drugs have been approved by the FDA for AD treatment: donepezil, rivastigmine, galantamine, tacrine, memantine, and aducanumab. The first four drugs are acetylcholinesterase inhibitors (AChEIs), while memantine is an N-methyl-D-aspartate receptor (NMDAR) antagonist, and aducanumab is a human anti-Aβ antibody that targets aggregated forms of Aβ . American and European guidelines list AChEIs as first-line pharmacotherapies for mild to moderate AD. However, AChEIs only show modest efficacy on cognitive deficits and non-significant efficacy on functional capacity in mild to moderate AD. Memantine shows very limited efficacy on cognitive symptoms without functional improvement. A variety of novel therapeutic approaches to AD, including pharmacological interventions and non-pharmacological interventions are investigated, including anti-Aβ therapy, anti-Tau therapy, anti-neuroinflammatory therapy, neuroprotective agents, and brain stimulation.

Therefore, there is still a need for novel drugs in the treatment for neurodegenerative diseases like tauopathies, in particular for AD.

### SUMMARY OF THE INVENTION

In accordance with the present invention, the solution of the above-defined problem is provided by the embodiments as characterized in the claims, disclosed in the description and illustrated in the Examples and Figures further below. Thus, the present invention relates to anti-CRMP2 antibodies, CRMP2-binding fragments and equivalent CRMP2 binding molecules that preferably display substantially the same binding characteristics and biological properties illustrated in the appended Examples and Figures with the lead antibody designated NI-504.3E7.

Since, as mentioned in the background section, current drug therapies for the treatment of AD as the most common neurodegenerative disease only showed varying or no significant success, the present inventors sought for alternative target proteins that potentially could play a role in the onset and/or progression of the disease, and wherein targeting one or more forms of the target protein, e.g., specific conformation, denatured vs. native-folded, monomer vs. multimer, phosphorylated vs. non-phosphorylated, etc., leads to a disease-modifying effect.

Eventually, after diligent work on several hypothesis and investigation of different kinds of disease models, the present inventors conceived a novel therapeutic strategy and selected Collapsin Response Mediator Protein 2 (CRMP2) as novel therapeutic target for neurodegenerative diseases such as AD.

Collapsin Response Mediator Proteins (CRMPs) represent a family of cytosolic proteins (CRMP1-5) that are expressed at high levels in the developing brain. CRMPs serve as signaling molecules involved in modulating microtubule polymerization and stabilization, actin bundling, and endocytosis, resulting in neuronal differentiation; see Brustovetsky et al., Cells 10 (2021), 2781. CRMP2 is a cytosolic protein implicated in axon guidance and neurite outgrowth via the Semaphorin 3A pathway. In contrast to other members of the CRMP family, CRMP2 retains a high level of expression in adults. CRMP2 is a phosphoprotein and while CRMP2 binds tubulin-heterodimers and promotes microtubule assembly and stabilization, phosphorylation of CRMP2 negatively regulates its ability to bind to and stabilize tubulin. Thus, phosphorylation affects CRMP2's affinity for assembled microtubules and tubulin dimers, which subsequently results in elongation or retraction of axons. In Brustovetsky et al., Cells 10 (2021), 2781 it is explained in more detail that increased CRMP2 phosphorylation correlates with decreased CRMP2 binding to dynamin-related protein 1 (Drp1), Miro 2, and Kinesin 1 light chain (KLC1). Switching between inactive (phosphorylated) and active (non-phosphorylated) CRMP2 is an ongoing physiologic adaptive mechanism for preventing abnormal neuronal sprouting. Overall, a balance exists between active and inactive CRMP2. Furthermore, CRMP2 has been reported to be crucial for neurogenesis and associated with numerous neurodegenerative disorders and as such has been suggested as specific marker of AD; see for example Soutar et al., Curr Alzheimer Res 6 (2009), 269-278.

However, so far CRMP2 had not been considered as a possible target in the suppression of pathological processes in the brain, probably because no pathological form of CRMP2 was known in this sense as for the known target proteins in neurodegeneration, e.g., Aβ and hyperphosphorylated Tau as well as their aggregates.

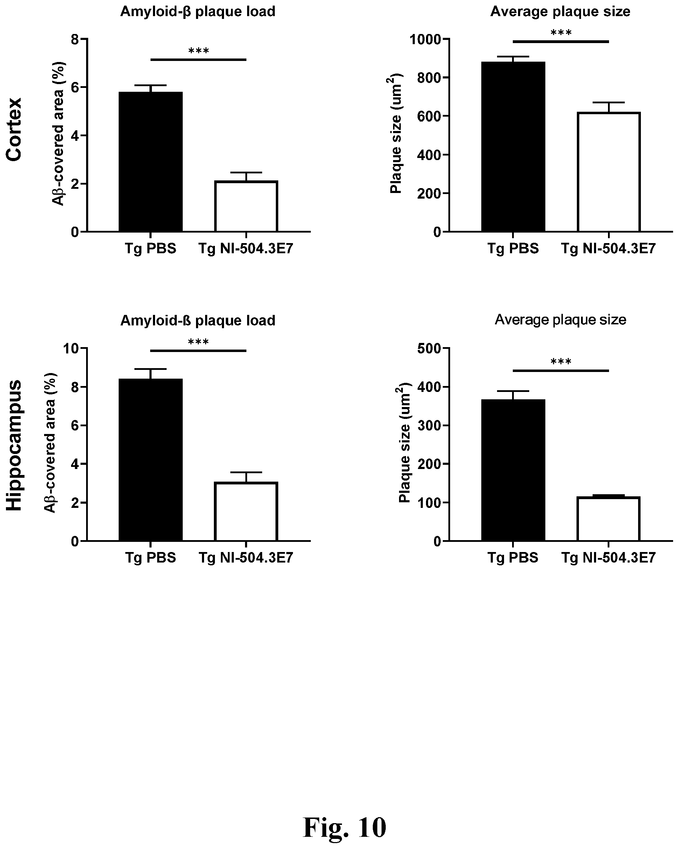

Accordingly, the results of the *in vivo* experiments carried out by the inventors are all the more surprising in that targeting of CRMP2 by means of anti-CRMP2 antibodies leads to significant therapeutic effects in various neurodegenerative disease animal models, one of which is illustrated in the Examples. In particular, the inventors could demonstrate that an anti-CRMP2 antibody, designated NI-504.3E7 reduces the load and average size of amyloid-β (Aβ) plaques by about 60% in TTBK1/APP transgenic mice; see Example 11 and Figure 10. In addition to reducing amyloid plaque load in a transgenic mouse model of AD, targeting CRMP2 has been shown to improve long term potentiation and cognitive function in aged mice; see Examples 13 and 14.

Characterization of antibody NI-504.3E7 in biochemical and *in vitro* cellular assays revealed that the antibody binds to human CRMP2 selectively over the other four CRMP members (Examples 2 and 3 as well as Figure 2), is capable of binding full length non-phosphorylated CRMP2 and phosphorylated CRMP2 (pCRMP2) with high affinity (Example 4), reduces the level of pCRMP2 in a concentration-dependent manner (Example 5 and Figure 4), and reverses the increases of spine density induced by pCRMP2 aggregates and monomeric CRMP, respectively, see Example 9 and Figure 8.

In view of these properties of the antibody, the inventors hypothesize, but without intending to be bound by theory that by interfering with the level of and ratio between, respectively, pCRMP2 and CRMP2 adverse effects of CRMP/CRMP2 in the brain due to alterations in the abundance and phosphorylation status of CRMP2 can be reversed.

For example, CRMP2s phosphorylation status is reportedly altered in different neuropathologies, including Huntington's disease (HD) and Alzheimer's disease (AD). In particular, high levels of phosphorylated CRMP2 have been reported in human AD brains in association with neurofibrillary tangles suggesting that accumulation of phosphorylated CRMP2 may be an early event in AD. It has been shown that not only the level of phosphorylated CRMP2 is increased, but that CRMP2 was hyperphosphorylated meaning that the relative level of phosphorylation of CRMP2 actually increases in brain areas associated with AD pathology; see Cole et al., J. Neurochem. 103 (2007), 1132-44.

Based on the experimental evidence the inventors believe, but again without intending to be bound by theory that a reduced amount of CRMP2 and an increased amount of (hyper)phosphorylated CRMP2 could not only be a marker of neurodegenerative diseases as hitherto described, but in fact may be actively involved in the onset and manifestation of the disease.

In this context, further experiments performed in accordance with the present invention revealed that exemplary antibody NI-504.3E7 is capable of detecting CRMP2 intracellularly as demonstrated in Example 12 with fully differentiated SH-SY5Y cells.

In addition, to exclude the possibility that the observed biological activities are dependent on the specific amino acid sequence of the variable region of antibody NI-504.3E7, several variants with alterations in the framework region and one in the CDRs have been constructed and tested. As shown in Table II, though some amino acid alterations affect the absolute EC₅₀ values of the antibody for CRMP2 and pCRMP2, all values remain in the nanomolar range for both CRMP and pCRMP2.

Accordingly, in a general aspect, the present invention relates to CRMP2 as a therapeutic target in the treatment of neurodegenerative disease and to any molecule capable of interfering with CRMP2 signaling, equivalent to and similar in kind as demonstrated for antibody NI-504.3E7, for use as a medicament, preferably for use in a method of preventing, delaying of progression or treating a neurodegenerative disorder or cognitive disorder and/or improving memory and learning ability. Preferably, such molecule is CRMP2 binding molecule which displays the binding characteristics and biological activities as demonstrated for antibody NI-504.3E7 in the mentioned biochemical and in vitro cellular assays. Such molecule may be screened for and obtained from compound libraries using the assays described in the Example, designed *in silico* or derived from antibody NI-504.3E7.

Nevertheless, because of its lack of immunogenicity and use in proof-of-concept, the use of a human or human-derived antibody is particularly preferred.

As illustrated in the Examples, within a complex antibody discovery process human monoclonal anti-CRMP2 antibodies have been cloned and identified; see Example 1. Those antibodies were tested for their binding specificity and affinity. As can be derived from Table I in Example 1, even though some of the cloned antibodies showed specific binding to a CRMP2-peptide with an EC₅₀ value of less than 20 nM in ELISA assays, remarkably, only three antibodies showed specific binding to the CRMP2-protein with an EC₅₀ value of less than 20 nM in ELISA assays. These three antibodies, *i.e*., antibodies NI-504.3E7, NI-504.10, and NI-504.12 further showed a binding signal in human brain tissue in immunohistochemical (IHC) assays, *i.e.*, only NI-504.3E7 and NI-504.12 with a strong binding signal in human hippocampal tissue at a concentration of 0.2 µg/mL. From these antibodies, only antibody NI-504.3E7 showed selective binding to CRMP2, *i.e.,* to both phosphorylated and non-phosphorylated CRMP2, wherein the other two antibodies also recognized CRMP1 and CRMP4.

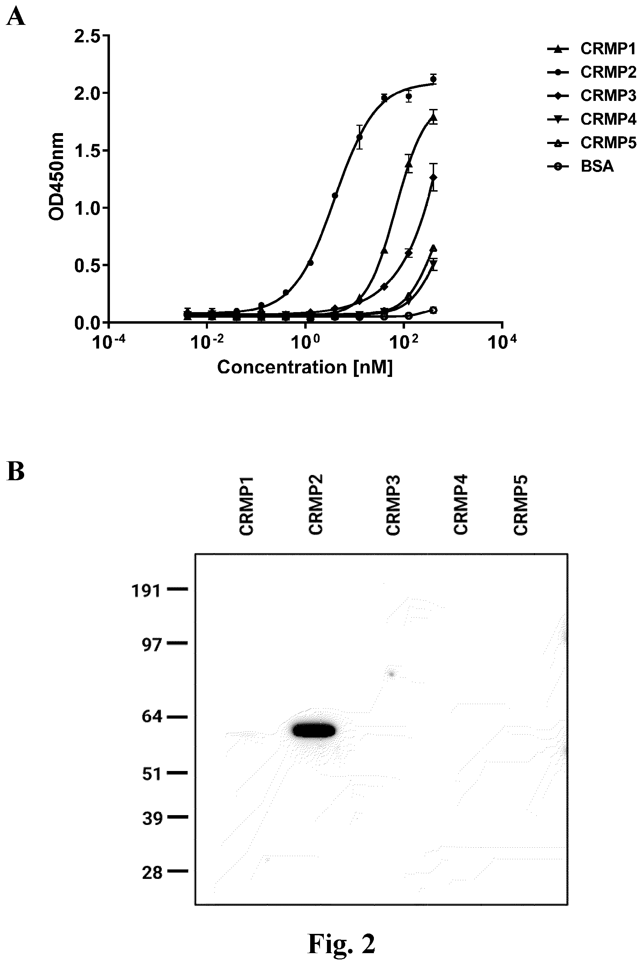

In more detail, antibody NI-504.3E7 has been shown to bind with high affinity, *i.e.,* with an EC₅₀ of 4 nM to full length CRMP2 as determined in ELISA assays, wherein only weak binding to CRMP1 was observed, *i.e.,* with an EC₅₀ of 69 nM as determined in ELISA assays. Binding of antibody NI-504.3E7 to CRMP3, CRMP4, and CRMP5 was very weak and no EC₅₀ value could be determined. Similar results have been obtained by Western Blot analysis, where it was shown that antibody NI-504.3E7 displays strong binding to full length CRMP2, weak binding to CRMP1, and no binding to CRMP3, CRMP4, and CRMP5; see Example 2 and Figure 2. Thus, the antibody does not substantially cross-react with other CRMP proteins.

Furthermore, antibody NI-504.3E7 has been shown to bind to full length non-phosphorylated CRMP2 and phosphorylated CRMP2 (pCRMP2) in ELISA assays, wherein it has been shown that antibody NI-504.3E7 specifically recognizes CRMP2 and pCRMP2 with an EC₅₀ in the low nanomolar range; see Example 4 and Table II.

In contrast, most antibodies available so far exclusively bind phosphorylated CRMP2. For example, anti-pCRMP2 antibody 3F4 recognizes triple- and dual-phosphorylated CRMP2, but not non-phosphorylated or single-phosphorylated CRMP2; see Uchida et al., Genes Cells 10 (2005), 165-79. Also, the commercially available polyclonal anti-phospho-CRMP-2 (pThr⁵⁰⁹) antibody (SAB4504698 from Sigma-Aldrich^{®}, available at Merck KGaA, Darmstadt, Germany), which was produced against a synthesized peptide derived from human CRMP2 around the phosphorylation site of Thr509, only binds pCRMP2. Same applies to antibody pCRMP-2 (Thr514) #9397 of Cell Signaling Technology, Inc, Leiden, The Netherlands. Furthermore, in WO 2011/007209 A1 an anti-CRMP2 antibody is described which is specific for pCRMP2 phosphorylated on tyrosine 479.

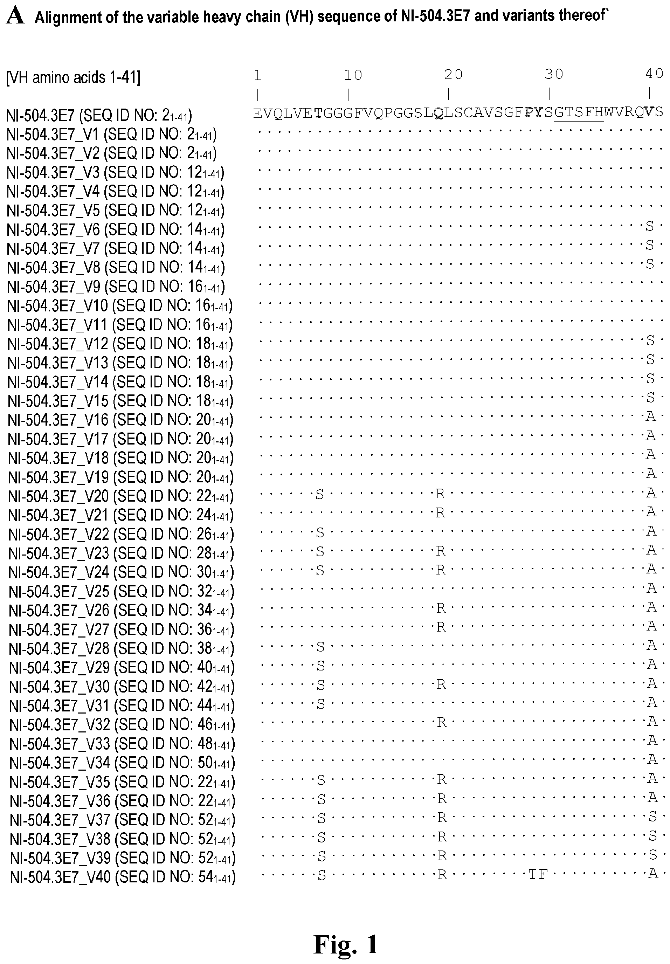

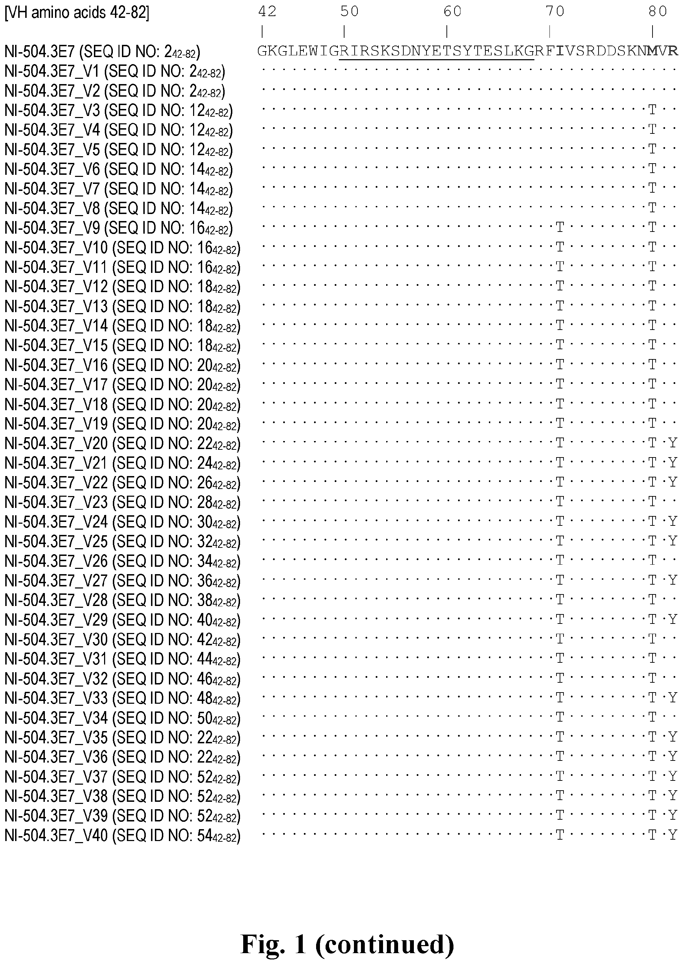

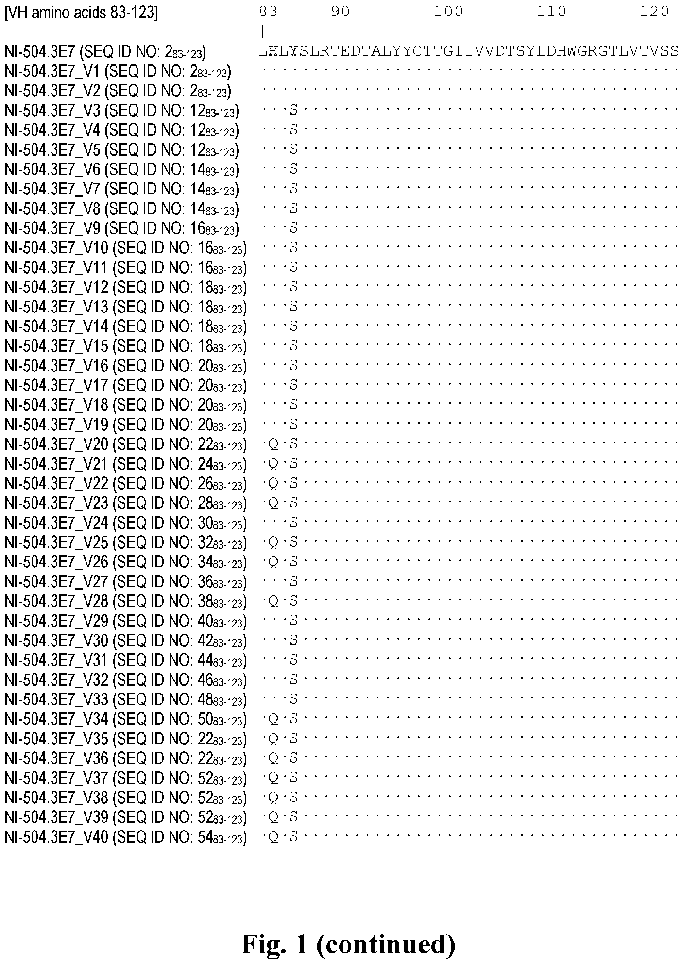

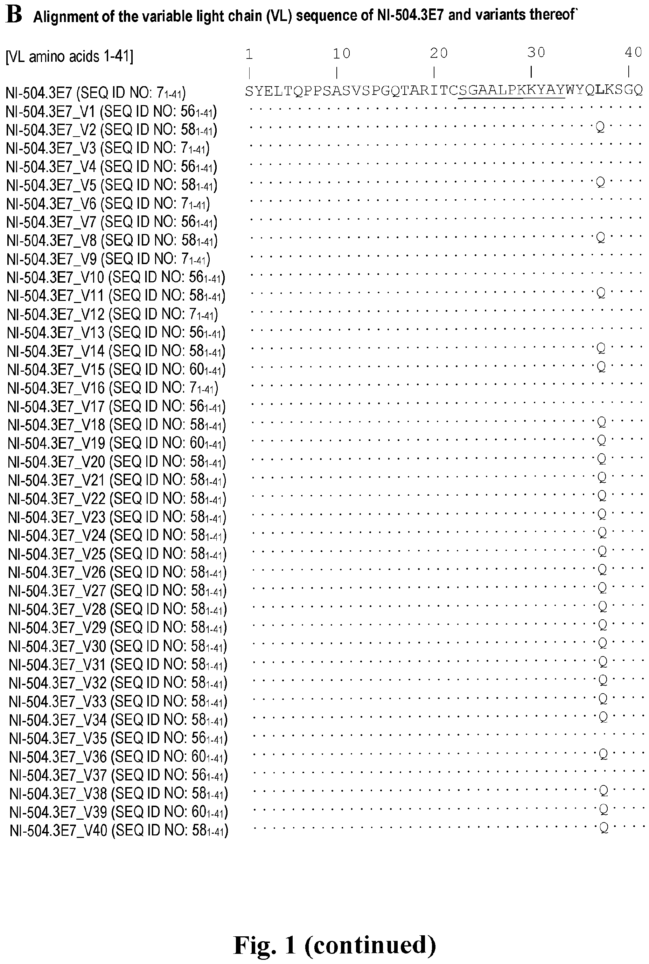

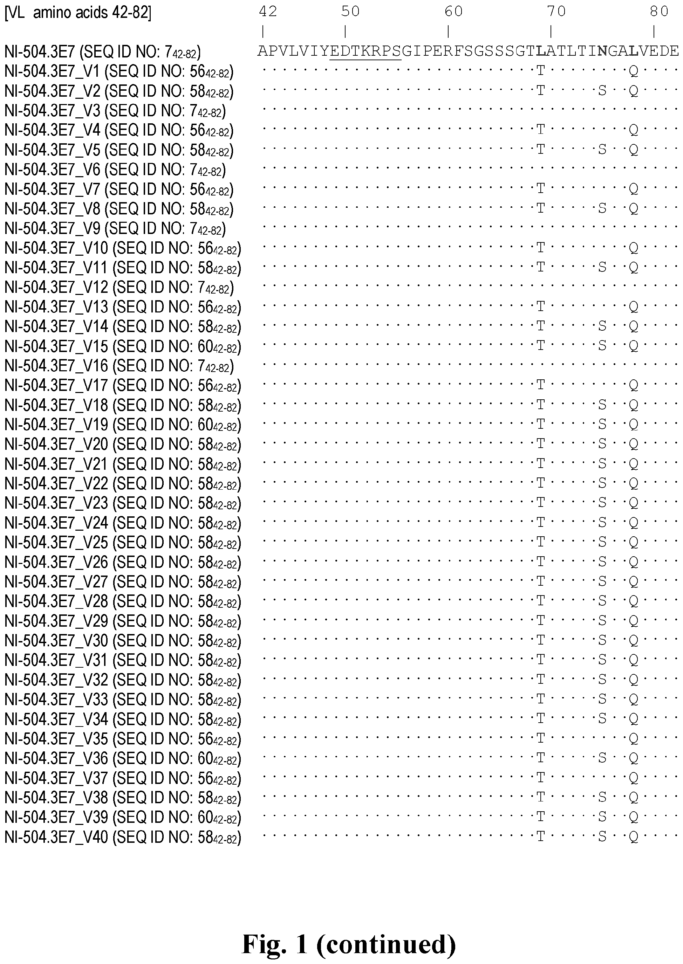

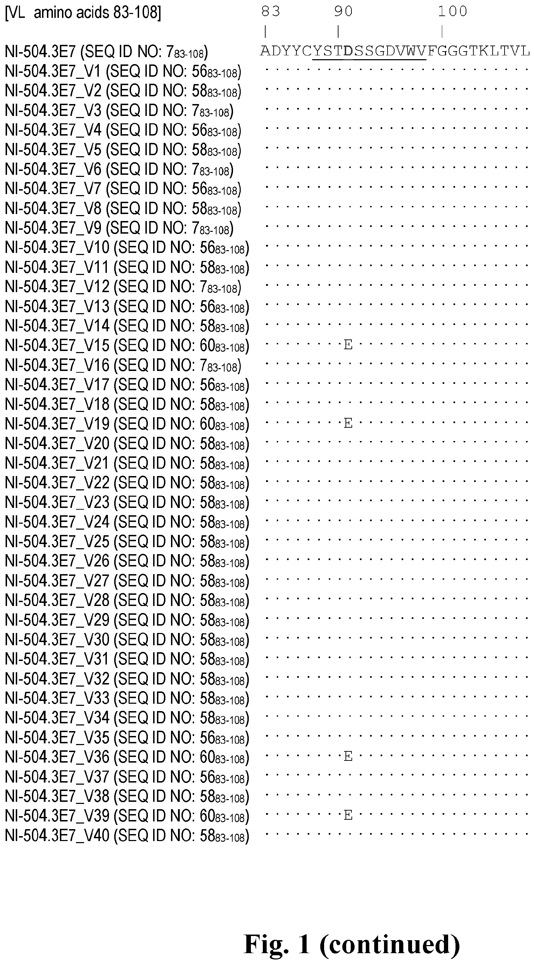

In addition to the original antibody NI-504.3E7, 40 variants (NI-504.3E7_V1 to NI-504.3E7_V40) of antibody NI-504.3E7 have been generated by exchanging amino acids in the variable heavy (VH) chain region, the variable light (VL) chain region, and/or within the complementarity determining regions (CDRs). The nucleotide and amino acid sequences of the variable regions (VH, VL) of antibody NI-504.3E7 and its variant VH and VL chains and CDRs are depicted in Table IV and V. In particular, three variant VL chains have been generated, *i.e.,* VL_3E7_var1, VL_3E7_var2, and VL_3E7_var3, wherein the variants comprise two, four, or five amino acid exchanges; see Table VII and the Alignment in Figure 1. Furthermore, 22 variant VH chains have been generated, *i.e.,* VH_3E7_var1, VH_3E7_var2, VH_3E7_var3, VH_3E7 _var4, VH_3E7 _var5, VH_3E7 _var6, VH_3E7_var6a, VH_3E7 _var6b, VH_3E7 _var6c, VH_3E7_var6d, VH_3E7_var6ab, VH_3E7_var6ac, VH_3E7 _var6ad, VH_3E7 _var6bc, VH_3E7_var6bd, VH_3E7_var6cd, VH_3E7_var6bcd, VH_3E7 _var6acd, VH_3E7 _var6abd, VH_3E7_var6abc, VH_3E7_var7, and VH_3E7 _var8, wherein the variants comprise two, three, four, five six, seven, eight, or 10 amino acid exchanges; see Table VII and the Alignment in Figure 1. The assignment which antibody variant (NI-504.3E7_V1 to NI-504.3E7_V40) comprises which variant VH and VL chain is shown in Table VI.

As shown in Example 4 and Table II, all variants maintained their ability to bind CRMP2 and pCRMP2. Furthermore, the results of the experiments performed with the original antibody NI-504.3E7 as described above have been confirmed with at least one exemplary variant.

Epitope mapping has been performed which, as shown in Example 3, revealed that antibody NI-504.3E7 binds to a linear epitope close to the C-terminus of CRMP2. In particular, antibody NI-504.3E7 binds CRMP2 peptides which comprise or consist of the amino acid sequence TPKTVTPASSAKTSP (SEQ ID NO: 62) or VTPASSAKTSPAKQQ (SEQ ID NO: 63) and the minimal epitope has been determined to comprise the amino acid sequence 516-ASSAK-520 (SEQ ID NO: 64).

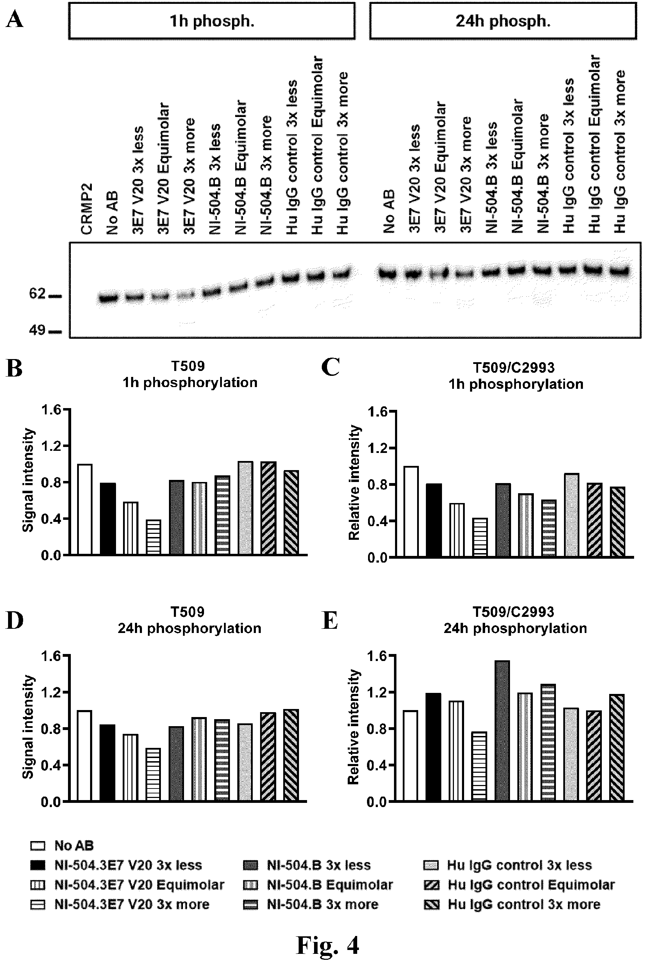

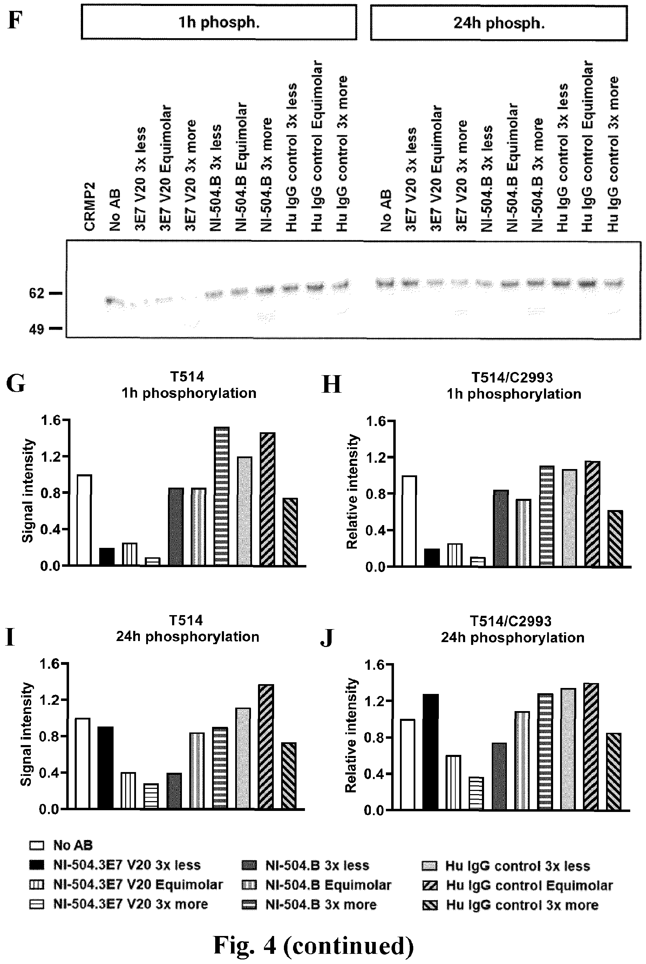

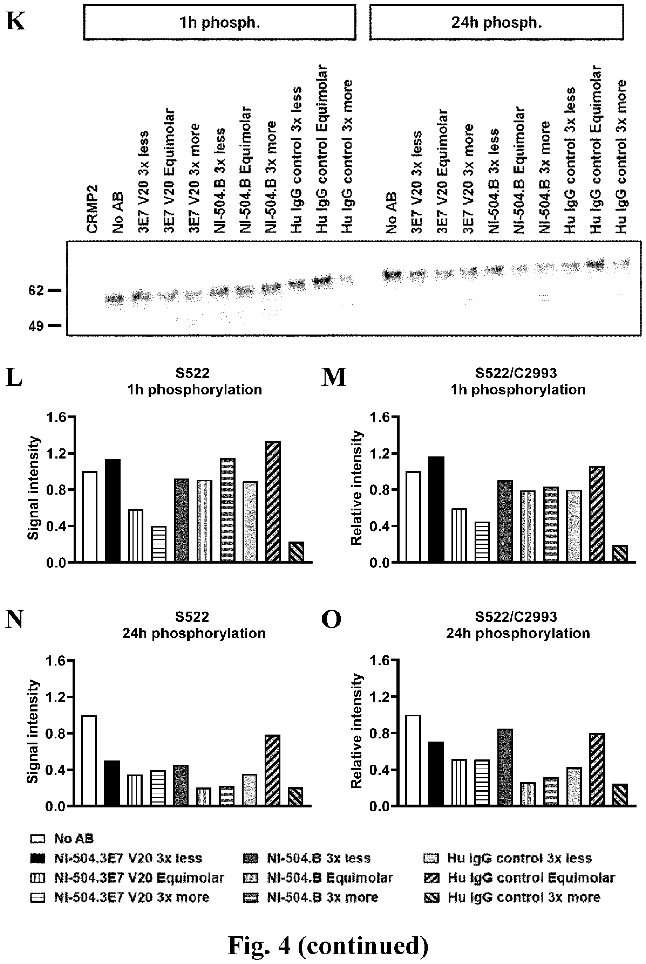

As mentioned above, antibody NI-504.3E7 reduces the level of pCRMP2 in a concentration-dependent manner; see Example 5 and Figure 4. Accordingly, without intending to be bound by theory, the therapeutic effect of the anti-CRMP2 antibody could be due to its ability to bind to CRMP2 and either preserving it and/or instead of neutralizing CRMP2 and/or inhibiting phosphorylation of CRMP2 and/or capturing phosphorylated CRMP2 leading to a reduced amount of hyperphosphorylated CRMP2 and/or a reduced amount of phosphorylated CRMP2 in general, which is excessively present in the brain of AD patients.

One explanation for this unique activity could be the epitope of antibody NI-504.3E7 (comprising 516-ASSAK-520, SEQ ID NO: 64), which is located at the C-terminus of CRMP2 and very close to the phosphorylation sites T514 and S522. This is supported by the fact that a corresponding anti-CRMP2 control antibody (NI-504.B), which binds to a different epitope which is not in proximity of the mentioned phosphorylation sites does not have this property, *i.e*., it does not reduce the level of pCRMP2 in a concentration-dependent manner; see Example 5 and Figure 4.

As further illustrated in the Examples, the cDNA sequences encoding the antibody NI-504.3E7 have been originally cloned from human memory B-cells. As mentioned above, in humans the phosphorylated as well as the non-phosphorylated form of CRMP2 is present. Furthermore, the ternary structure of the C-terminal region (490-572) of CRMP2 is flexible and somewhat random, altering its conformation upon posttranslational modification such as phosphorylation; see Nakamura et al., Front. Cell Neurosci. 14 (2020), 188. Accordingly, antibodies originally raised in the human body which are exposed to the natural environment and thus, to the flexible and altering ternary structure of the C-terminal region of CRMP2 might have specific binding characteristics which cannot be found in antibodies raised against an antigen, like the monoclonal anti-CRMP2 antibody 1B1 (sc-101348, Santa Cruz Biotechnology, Dallas, USA) and the polyclonal anti-CRMP2 antibody (ab36201) from abcam, Cambridge, UK, which both have been raised against a synthetic CRMP2 C-terminal peptide, and the polyclonal anti-CRMP2 antibody C2993 from Sigma-Aldrich^{®}, available at Merck KGaA, Darmstadt, Germany, which has been raised against non-phosphorylated full length CRMP2.

In fact, as described in Mileusnic and Rose, Journal of Neurochemistry 118 (2011), 616-635, doi: 10.1111/j.1471-4159.2011.07193.x, antibody ab36201, when administered to young chicks, induced amnesia for the passive avoidance task. Accordingly, this antibody negatively effects the cognitive function in chicks in contrast to the antibody of the present invention which improves the cognitive function in mice.

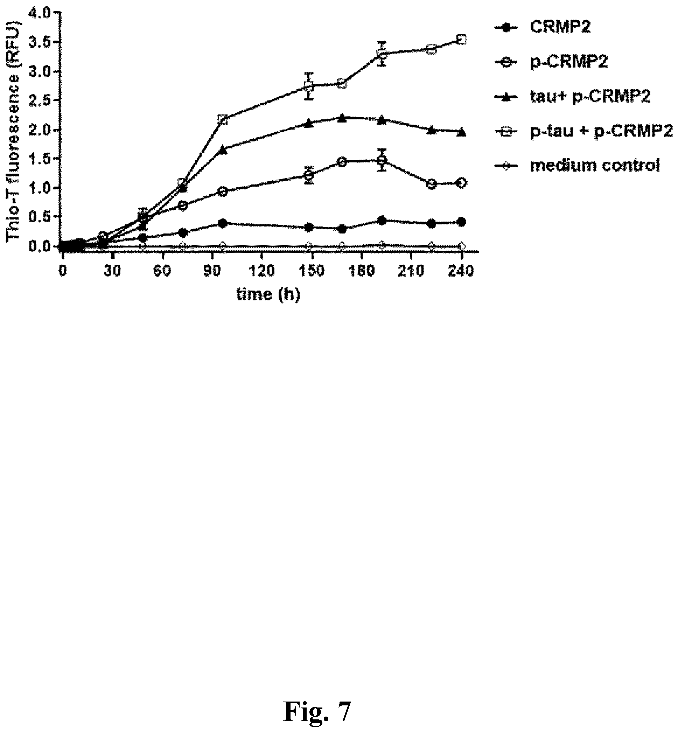

It is further known that hyperphosphorylated Tau is co-localized with phosphorylated CRMP2; see Takata et al., Am. J. Pathol 175 (2009), 17-24. In experiments made within the scope of the present invention it has now been shown that recombinant (p)Tau and (p)CRMP2 indeed co-aggregate in a ThioT assay (see Example 8 and Figure 7) and as mentioned before, it was also shown that antibody NI-503.3E7 reduces the amyloid plaque load in a transgenic AD mouse model. Accordingly, it is prudent to expect that antibody NI-504.3E7 is capable of interfering with coaggregation of pTau and pCRMP2. In this context, unless explicitly stated otherwise, the term "NI-504.3E7" refers to the original antibody NI-504.3E7 as well as to any variant thereof and is representative of equivalent CRMP2 specific binding molecules.

Extracellular neuritic plaques composed of amyloid-β (Aβ) protein and intracellular neurofibrillary tangles containing phosphorylated Tau protein, which are the two hallmark proteins of Alzheimer's disease (AD), and the separate neurotoxicity of these proteins in AD has been extensively studied and it is suggested that the coexistence of Aβ plaques and phosphorylated Tau is related to the mechanism by which Aβ facilitates the propagation of Tau aggregation in neuritic plaques. The interactions between Aβ and Tau mediate cognitive dysfunction in patients with AD; see Zhang et al., Int J Biol Sci. 17 (2021), 2181-2192. It is further known that therapeutics developed for the treatment of AD often target both, Aβ plaques and neurofibrillary tangles containing phosphorylated Tau protein. For example, a human anti-Aβ antibody has recently been shown to induce neutralization and removal of amyloid plaques from the brain and of neurotoxic oligomeric Aβ species from pre-synaptic axonal terminals, thereby protecting axons from amyloid damage resulting in remaining unphosphorylated microtubule-bound Tau within its physiological axonal localization, thereby preventing abnormal re-localization of Tau from axonal to somato-dendritic compartments in amyloid affected neurons, where Tau gets abnormally phosphorylated and becomes aggregation-prone. Moreover, extracellular species of pathological Tau are removed via phagocytosis of microglial cells or macrophages, which is observed by decreases in CSF levels of pathological phospho-Tau in aducanumab-treated patients; see, e.g., international application WO 2021/081101 A1 and Cummings et al. Alz. Res. Therapy 13 (2021), 98; Salloway and Cummings, Aducanumab, Amyloid Lowering, and Slowing of Alzheimer Disease, in Neurology 97 (2021), 543-544.

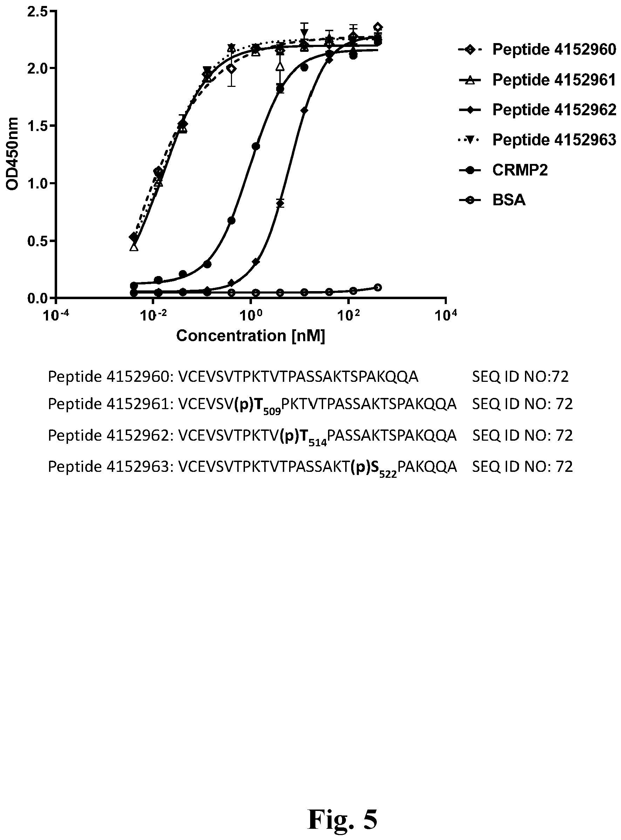

As mentioned above, CRMP2 is either phosphorylated or non-phosphorylated, wherein phosphorylation regulates neurite outgrowth. CRMP2 can be sequentially phosphorylated by GSK3β at S518, T514, and T509 but only after prior phosphorylation by Cdk5 at Ser522. It has been already shown in the Examples 2 and 4 that antibody NI-504.3E7 binds to full length phosphorylated CRMP2 and additional experiments have been performed to show that antibody NI-504.3E7 is capable of binding phosphorylated CRMP2 peptides. Experiments determining the binding specificity of antibody NI-504.3E7 using phosphorylated peptides showed that the affinity of antibody NI-504.3E7 to the CRMP2 peptide with phosphorylation at position T514 is about 300-fold lower than to the non-phosphorylated peptide and the peptides with phosphorylation at positions T509 and S522 suggesting that phosphorylation of T514 partially impairs binding of antibody NI-504.3E7 potentially due to the close proximity to the antibody minimal epitope 516-ASSAK-520; see Example 6 and Figure 5.

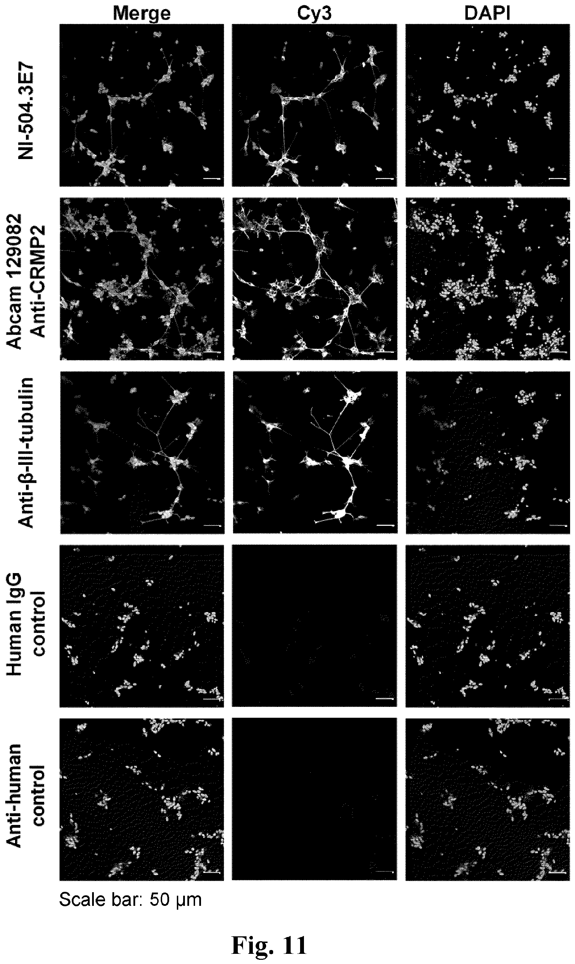

Antibody NI-504.3E7 has further been shown to bind human, murine and rat recombinant CRMP2 full length protein. It was also shown that antibody NI-504.3E7 is internalized in SH-SY5Y cells; see Example 12 and Figure 11.

As shown in Example 9, aggregated pCRMP2 and monomeric CRMP2 increase neuronal spine density, wherein the increase of spine density driven by CRMP2/pCRMP2 is reversed by NI-504.3E7 at two different concentrations as determined by an *ex vivo* hippocampal slice culture model. Such assay can be used as a proof-of-activity of the antibody, in particular as proof that the antibody interferes with CRMP2 signaling, which has not been described before.

Accordingly, the present invention relates to an antibody and equivalent CRMP2-binding molecules, which may be a binding fragment or derivative of NI-504.3E7 having substantially the same binding specificity and activity of antibody NI-504.3E7 meaning that the present invention relates to an antibody and equivalent CRMP2-binding molecules which (i) preferentially recognizes human CRMP2 over CRMP1, CRMP3, CRMP4 and CRMP5, which can be tested in ELISA assays and Western Blot analysis as described in Example 2, (ii) is capable of reducing the level of pCRMP2 in a concentration-dependent manner when subjecting CRMP2 to a phosphorylation assay, which can be determined as described in Example 5, and/or (iii) is capable of reversing the increase of neuronal spine density induced by CRMP2 as determined in an *ex vivo* hippocampal slice culture model, which can be tested as described in Example 9. Accordingly, the present invention relates to an antibody and equivalent CRMP2-binding molecules which may be a binding fragment or derivative of NI-504.3E7 having substantially the same binding specificity and activity of antibody NI-504.3E7 in terms of feature (i), or of feature (ii), or of feature (iii), or of features (i) and (ii), or of features (i) and (iii), or of features (ii) and (iii), or of features (i), (ii), and (iii).

The mentioned features can be easily determined in accordance with the experiments and assays disclosed in the appended Examples, wherein antibody NI-504.3E7 can be used as reference antibody. Typically, such equivalent CRMP2 binding molecule will compete with the corresponding reference antibody for binding CRMP2 and pCRMP2 at the same epitope and the peptide, respectively, mentioned above. Preferably, such equivalent CRMP2 binding molecule has the same epitope as the reference antibody.

As mentioned above, 40 variants (NI-504.3E7_V1 to NI-504.3E7_V40) of antibody NI-504.3E7 have been generated by exchanging amino acids in the variable heavy (VH) chain region, the variable light (VL) chain region, and/or within the complementarity determining regions (CDRs).

Thus, in one embodiment, the antibody of the present invention may be characterized by the complementarity determining regions (CDRs) or hypervariable regions of the variable heavy (VH) and variable light (VL) chain of antibody NI-504.3E7 comprising the amino acid sequence of SEQ ID NO: 2 (VH_3E7) and SEQ ID NO: 7 (VL_3E7) as shown in Tables IV and V.

In one embodiment, the antibody of the present invention may be characterized by the CDRs or hypervariable regions of the VH and VL chain of antibody NI-504.3E7_V1 comprising the amino acid sequence of SEQ ID NO: 2 (VH_3E7) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V2 comprising the amino acid sequence of SEQ ID NO: 2 (VH_3E7) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V3 comprising the amino acid sequence of SEQ ID NO: 12 (VH_3E7_var1) and SEQ ID NO: 7 (VL_3E7) as shown in Tables IV and V.

In one embodiment, the antibody of the present invention may be characterized by the CDRs or hypervariable regions of the VH and VL chain of antibody NI-504.3E7_V4 comprising the amino acid sequence of SEQ ID NO: 12 (VH_3E7_var1) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V5 comprising the amino acid sequence of SEQ ID NO: 12 (VH_3E7_var1) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V6 comprising the amino acid sequence of SEQ ID NO: 14 (VH_3E7_var2) and SEQ ID NO: 7 (VL_3E7) as shown in Tables IV and V.

In one embodiment, the antibody of the present invention may be characterized by the CDRs or hypervariable regions of the VH and VL chain of antibody NI-504.3E7_V7 comprising the amino acid sequence of SEQ ID NO: 14 (VH_3E7_var2) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V8 comprising the amino acid sequence of SEQ ID NO: 14 (VH_3E7_var2) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V9 comprising the amino acid sequence of SEQ ID NO: 16 (VH_3E7_var3) and SEQ ID NO: 7 (VL_3E7) as shown in Tables IV and V.

In one embodiment, the antibody of the present invention may be characterized by the CDRs or hypervariable regions of the VH and VL chain of antibody NI-504.3E7_V10 comprising the amino acid sequence of SEQ ID NO: 16 (VH_3E7_var3) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V11 comprising the amino acid sequence of SEQ ID NO: 16 (VH_3E7_var3) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V12 comprising the amino acid sequence of SEQ ID NO: 18 (VH_3E7_var4) and SEQ ID NO: 7 (VL_3E7) as shown in Tables IV and V.

In one embodiment, the antibody of the present invention may be characterized by the CDRs or hypervariable regions of the VH and VL chain of antibody NI-504.3E7_V13 comprising the amino acid sequence of SEQ ID NO: 18 (VH_3E7_var4) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V14 comprising the amino acid sequence of SEQ ID NO: 18 (VH_3E7_var4) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V15 comprising the amino acid sequence of SEQ ID NO: 18 (VH_3E7_var4) and SEQ ID NO: 60 (VL_3E7_var3) as shown in Tables IV and V.

In one embodiment, the antibody of the present invention may be characterized by the CDRs or hypervariable regions of the VH and VL chain of antibody NI-504.3E7_V16 comprising the amino acid sequence of SEQ ID NO: 20 (VH_3E7_var5) and SEQ ID NO: 7 (VL_3E7) as shown in Tables IV and V.

In one embodiment, the antibody of the present invention may be characterized by the CDRs or hypervariable regions of the VH and VL chain of antibody NI-504.3E7_V17 comprising the amino acid sequence of SEQ ID NO: 20 (VH_3E7_var5) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V18 comprising the amino acid sequence of SEQ ID NO: 20 (VH_3E7_var5) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V19 comprising the amino acid sequence of SEQ ID NO: 20 (VH_3E7_var5) and SEQ ID NO: 60 (VL_3E7_var3) as shown in Tables IV and V.

In one embodiment, the antibody of the present invention may be characterized by the CDRs or hypervariable regions of the VH and VL chain of antibody NI-504.3E7_V20 comprising the amino acid sequence of SEQ ID NO: 22 (VH_3E7_var6) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V21 comprising the amino acid sequence of SEQ ID NO: 24 (VH_3E7_var6a) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V22 comprising the amino acid sequence of SEQ ID NO: 26 (VH _3E7_var6b) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V23 comprising the amino acid sequence of SEQ ID NO: 28 (VH_3E7_var6c) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V24 comprising the amino acid sequence of SEQ ID NO: 30 (VH _3E7_var6d) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V25 comprising the amino acid sequence of SEQ ID NO: 32 (VH_3E7_var6ab) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V26 comprising the amino acid sequence of SEQ ID NO: 34 (VH_3E7_var6ac) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V27 comprising the amino acid sequence of SEQ ID NO: 36 (VH_3E7_var6ad) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V28 comprising the amino acid sequence of SEQ ID NO: 38 (VH_3E7_var6bc) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V29 comprising the amino acid sequence of SEQ ID NO: 40 (VH_3E7 _var6bd) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V30 comprising the amino acid sequence of SEQ ID NO: 42 (VH_3E7_var6cd) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V31 comprising the amino acid sequence of SEQ ID NO: 44 (VH_3E7 _var6bcd) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V32 comprising the amino acid sequence of SEQ ID NO: 46 (VH_3E7_var6acd) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V33 comprising the amino acid sequence of SEQ ID NO: 48 (VH_3E7 _var6abd) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V34 comprising the amino acid sequence of SEQ ID NO: 50 (VH_3E7_var6abc) and SEQ ID NO: 58 (VL_3E7 _var2) as shown in Tables IV and V.

In one embodiment, the antibody of the present invention may be characterized by the CDRs or hypervariable regions of the VH and VL chain of antibody NI-504.3E7_V35 comprising the amino acid sequence of SEQ ID NO: 22 (VH_3E7_var6) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V36 comprising the amino acid sequence of SEQ ID NO: 22 (VH_3E7_var6) and SEQ ID NO: 60 (VL_3E7_var3) as shown in Tables IV and V.

In one embodiment, the antibody of the present invention may be characterized by the CDRs or hypervariable regions of the VH and VL chain of antibody NI-504.3E7_V37 comprising the amino acid sequence of SEQ ID NO: 52 (VH_3E7_var7) and SEQ ID NO: 56 (VL_3E7_var1) as shown in Tables IV and V.

In one embodiment, the antibody of the present invention may be characterized by the CDRs or hypervariable regions of the VH and VL chain of antibody NI-504.3E7_V38 comprising the amino acid sequence of SEQ ID NO: 52 (VH_3E7_var7) and SEQ ID NO: 58 (VL_3E7_var2) as shown in Tables IV and V.

In one embodiment, the antibody of the present invention may be characterized by the CDRs or hypervariable regions of the VH and VL chain of antibody NI-504.3E7_V39 comprising the amino acid sequence of SEQ ID NO: 52 (VH_3E7_var7) and SEQ ID NO: 60 (VL_3E7_var3) as shown in Tables IV and V.

In one embodiment, the antibody of the present invention may be characterized by the CDRs or hypervariable regions of the VH and VL chain of antibody NI-504.3E7_V40 comprising the amino acid sequence of SEQ ID NO: 54 (VH_3E7_var8) and SEQ ID NO: 58 (VL_3E7_var2) as shown in Tables IV and V.

While the invention is illustrated and described by way of reference to the human-derived antibody originally obtained in the experiments performed in accordance with the present invention and described in the Examples it is to be understood that the present invention encompasses antibodies and equivalent CRMP2 binding molecules such as synthetic and biotechnological derivatives of an antibody NI-504.3E7, which means any engineered antibody or antibody-like CRMP2 binding molecule, synthesized by chemical or recombinant techniques, which retains one or more of the functional properties of the subject antibody, in particular which preferentially recognizes human CRMP2 over CRMP1, CRMP3, CRMP4 and CRMP5, which is capable of reducing the level of pCRMP2 in a concentration-dependent manner when subjecting CRMP2 to a phosphorylation assay, and/or which is capable of reversing the increase of neuronal spine density induced by CRMP2 as determined in an *ex vivo* hippocampal slice culture model. Thus, while the present invention may be described for the sake of conciseness by way of reference to an antibody or antibodies, unless stated otherwise synthetic and biotechnological derivatives thereof as well as equivalent CRMP2 binding molecules are meant and included within the meaning of the term "antibody".

As described above and demonstrated in the Examples, the present invention for the first time established CRMP2 and pCRMP2 as a therapeutic target in the treatment of a neurodegenerative disease. Thus, the present invention also relates to a molecule which is capable of targeting CRMP2 and pCRMP2 and interfering with CRMP2 signaling, respectively, for use as a medicament. In particular, the molecule is for use in a method for the prevention, delay of progression or the treatment of a neurodegenerative disorder, cognitive disorder and/or for improving memory and learning ability.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, the exemplary methods and materials are described below. All publications, patent applications, patents, and other references mentioned herein are incorporated by reference in their entirety. The materials, methods, and examples are illustrative only and not intended to be limiting.

Further embodiments of the present invention will be apparent from the description, Examples and claims that follow.

### BRIEF DESCRIPTION OF THE DRAWINGS

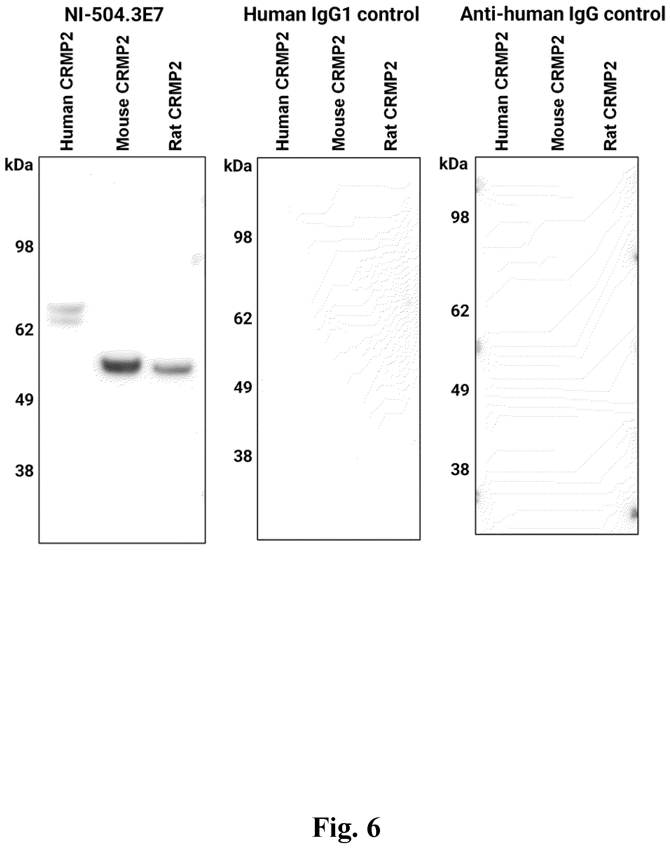

- **Fig. 1:**: Alignment of the amino acid sequences of the variable regions, *i.e.,* heavy chain (VH; **A**) and lambda light chain (VL; **B**) of anti-CRMP2 specific human antibody NI-504.3E7 to the 40 variants NI-504.3E7_V1 to V40, which comprise amino acid substitutions in the VH, VL and/or CDRs. Complementarity determining regions (CDRs) are indicated with the CDRs being underlined. The regions flanking the CDRs are known as the framework regions. The Kabat numbering scheme was used (cf. http://www.bioinf.org.uk/abs/; Kabat et al., U.S. Dept. of Health and Human Services, "Sequence of Proteins of Immunological Interest" (1983) as given in Table III, below. Unless otherwise specified, references to the numbering of specific amino acid residue positions in an antibody or CRMP2-binding fragment, variant, or derivative thereof of the present invention are according to the Kabat numbering system, which however is theoretical and may not equally apply to every antibody of the present invention. For example, depending on the position of the first CDR the following CDRs might be shifted in either direction. Accordingly, in case of any inadvertent errors or inconsistencies regarding indication of CDRs in Figure 1 and/or the sequence listing the person skilled in the art on the basis of the disclosure content of the present application, *i.e.,* the variable heavy (VH) and variable light (VL) chain amino acid sequences of antibody NI-504.3E7 and variants thereof is well in the position to determine the correct CDR sequences in accordance with Kabat, which shall be used for defining the claimed antibody and CRMP2-binding fragment thereof. Depicted are the variable heavy chain VH sequence of antibody NI-504.3E7 as set forth in SEQ ID NOs: 2 (**A**) and the light chain VL sequence of antibody NI-504.3E7 as set forth in SEQ ID NOs: 7 (**B**) which have been aligned to the VH and VL sequences of the 40 variants of NI.504.3E7. As further explained in the description, within CDRs and/or framework region conservative amino acid substitutions are preferred which take into account the physicochemical properties of the original amino acid either alone or with an adjacent amino acid as illustrated in Mirsky et al., Mol. Biol. Evol. 32 (2014) 806-819 at page 813, Figure 6 in particular the AB or LG model, for example such that the position of two amino acids is exchanged.
- **Fig. 2:**: The anti-CRMP2 antibody NI-504.3E7 of the present invention shows preferential binding to CRMP2 with a weak binding to CRMP1. **(A)** Binding activity of NI-504.3E7 was assessed on recombinant human CRMP proteins 1-5 by ELISA. Line depicts sigmoidal curve fit. Data is displayed as mean + SEM. **(B)** Binding specificity of NI-504.3E7 was assessed on recombinant human CRMP proteins 1-5 by Western Blot. Numbers indicate molecular weight in kilo Dalton (kDa).

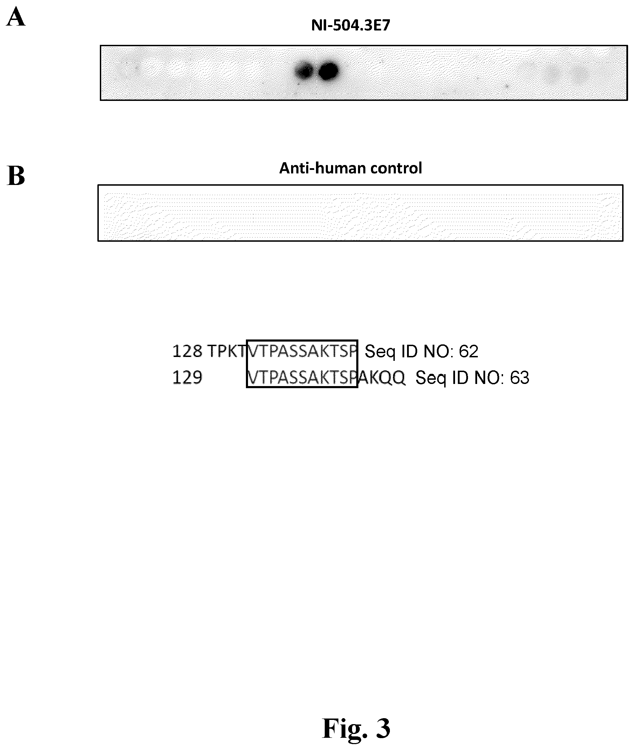

- **Fig. 3:**: (**A**) Epitope determination on antibody NI-504.3E7 binding. Epitope mapping using overlapping peptide array representing human CRMP2 protein showed that the anti-CRMP2 antibody NI-504.3E7 of the present invention bound to peptide 128 (SEQ ID NO: 62) and peptide 129 (SEQ ID NO: 63). (**B**) Anti-human control (secondary antibody control) shows absence of binding.
- **Fig. 4:**: The anti-CRMP2 antibody NI-504.3E7 of the present invention reduces the level of pCRMP2 in a concentration-dependent manner. Phosphorylation levels at three selected phospho-sites (T509 (**A-E**), T514 (**F-J**), S522 (**K-O**)) after 1h or 24h of phosphorylation in the presence of different concentrations of CRMP2-specific antibodies or a human IgG control (isotype control antibody) were analyzed in phosphorylation assays that were readout by Western Blots (**A, F, K**) (numbers indicate molecular weight in kilo Dalton (kDa)). Quantifications of the Western Blot analysis was performed based on the signal band intensity (**B-E, G-J, L-O**). In particular, signal band intensity was measured and displayed either directly or normalized to the total CRMP2 protein levels assessed with the commercial CRMP2 antibody C2993.
- **Fig. 5:**: The anti-CRMP2 antibody NI-504.3E7 of the present invention specifically binds CRMP2 and pCRMP2 peptides as determined by ELISA assay. The affinity of antibody NI-504.3E7 to the CRMP2 peptide with phosphorylation at position T₅₁₄ is ~300-fold lower than the non-phosphorylated peptide and the peptides with phosphorylation at positions T₅₀₉ and S₅₂₂. Phosphorylation of Threonine514, shows partially impaired binding of antibody NI-504.3E7. Unphosphorylated CRMP2 protein and BSA were used as positive and negative control, respectively. Line depicts sigmoidal curve fit. The sequences of the peptides bound by antibody NI-504.3E7 are indicated. Data is displayed as mean + SEM.
- **Fig. 6:**: The anti-CRMP2 antibody NI-504.3E7 of the present invention presents with orthologous protein binding on human, mouse, and rat recombinant CRMP2 protein. Binding specificity of NI-504.3E7 was detected on recombinant human CRMP2 (SUMO-CRMP2), mouse CRMP2 (CRMP2-His₆), and rat CRMP2 (CRMP2-His₆) proteins by Western Blot. Absence of binding on human IgG control (isotype control antibody) and anti-human control (secondary antibody control). Numbers indicate molecular weight in kilo Dalton (kDa).
- **Fig. 7:**: Recombinant CRMP2 and pCRMP2 aggregation is increased in the presence of recombinant Tau and pTau protein. Thio-T positive signal (RFU) indicates level of aggregation of the different proteins or protein combinations in a 240-hour aggregation assay. Medium was used as negative control. Data is displayed as mean + SEM.

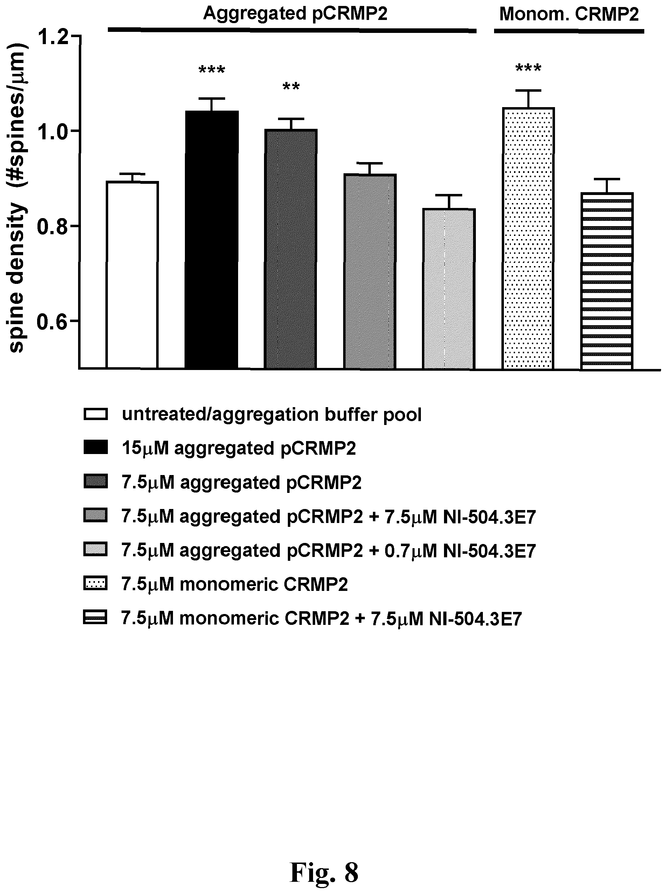

- **Fig. 8:**: Increase of spine density driven by CRMP2/phospho-CRMP2 is reversed by the anti-CRMP2 antibody NI-504.3E7 of the present invention at two different concentrations. pCRMP2 aggregates and CRMP2 monomer (monom. CRMP2) were evaluated on neuronal spine density in a hippocampal slice culture model. Dendritic spine density was assessed by threshold image analysis. Data is displayed as mean + SEM. Statistical analysis: One-way ANOVA *** p<0.01 ** p<0.01; Analysis by Kruskal-Wallis post-hoc test compared to untreated control.

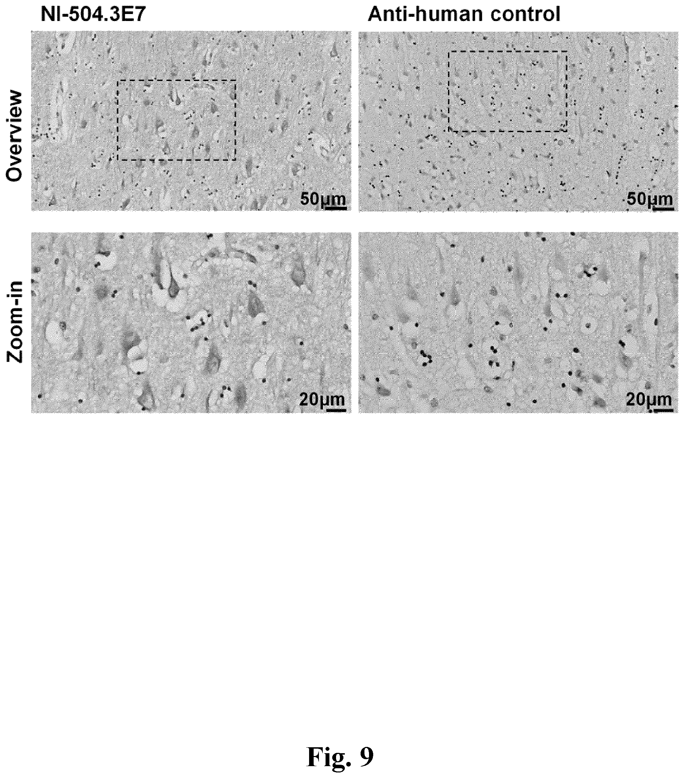

- **Fig. 9:**: The anti-CRMP2 antibody NI-504.3E7 of the present invention detects neuronal cells in the hippocampus of an Alzheimer's disease patient. **A)** Distinct neuronal cell staining is observed on Alzheimer's disease tissue with antibody NI-504.3E7, while absent in the anti-human control. **B)** Higher magnitude of the delineated area is shown as zoom-in.
- **Fig. 10:**: The anti-CRMP2 antibody NI-504.3E7 of the present invention reduces amyloid-beta plaques in female APPPS1/TTBK1 transgenic mice. Threshold-based image analysis was performed on cortical and hippocampal amyloid-beta histological staining. Number and size of amyloid-beta plaques were compared between NI-504.3E7 and PBS-injected APPPS1/TTBK1 transgenic mice. Mice receiving antibody NI-504.3E7 showed 63% reduced amyloid plaque load in cortex and hippocampus (**A, C**) and reduced plaque size (**B, D**). The reduction in average plaque size in female TTBK1/APP mice receiving antibody NI-504.3E7 was 30% in the cortex (**B**) and 68% in the hippocampus (**D**). Data is displayed as mean + SEM. Group size: n=7, Statistical analysis: Unpaired T-test ≤ 0.0001.
- **Fig. 11:**: The anti-CRMP2 antibody NI-504.3E7 of the present invention binds differentiated SH-SY5Y cells. Positive Cy3 signal was detected in cells stained with NI-504.3E7. SH-SY5Y cells stained with a commercial anti-CRMP2 antibody (Abcam 129082), and an anti-β-III-tubulin antibody were used as positive control. No signal was observed with human IgG control (isotype control antibody) and with an anti-human control (secondary antibody control). Nuclei were visualized with DAPI. Images were acquired with a confocal microscope (20x objective). Scale bar: 50 µM.

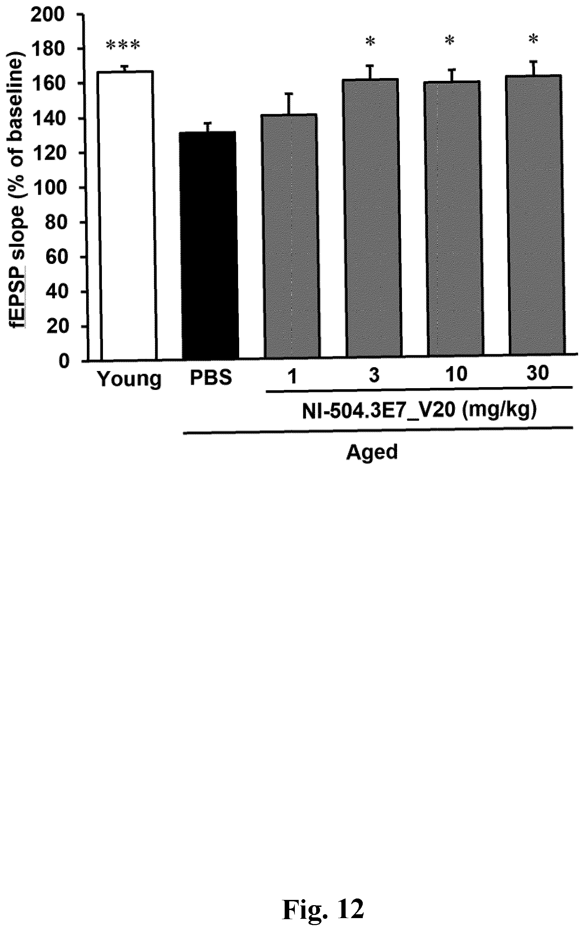

- **Fig. 12:**: The anti-CRMP2 antibody NI-504.3E7 of the present invention improves long-term potentiation (LTP) in aged C57Bl/6J mice. LTP analysis was performed in young mice and in a control population (aged mice receiving PBS) and the LTP signal was decreased in aged mice in comparison to young mice. After administration of the antibody to the aged mice, the LTP signal was increased again, thus showing that antibody NI-504.3E7 improves the LTP in aged mice. Data is displayed as mean + SEM. Group size: n=8, Statistical analysis: two-way ANOVA, * p<0.05, *** p<0.001 vs PBS group.

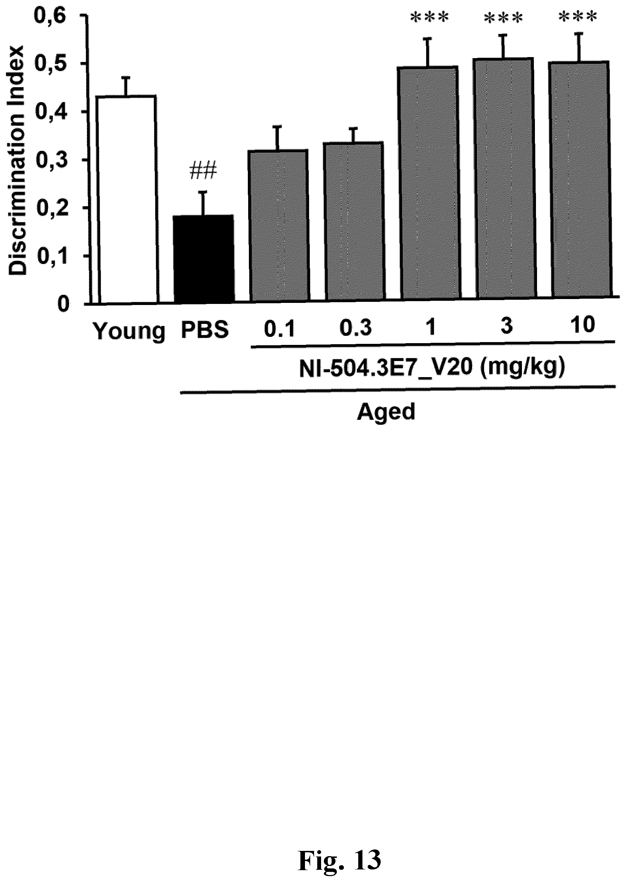

- **Fig. 13:**: The anti-CRMP2 antibody NI-504.3E7 of the present invention improves cognitive function in aged C57Bl/6J mice. The discrimination index was assayed on young mice and in a control population (aged mice receiving PBS) and the discrimination index was decreased in aged mice in comparison to young mice. After administration of the antibody, the discrimination index increased, thus showing that antibody NI-504.3E7 improves the cognitive function in aged mice. Data is displayed as mean + SEM. Group size: n=11 to 12, Statistical analysis: ** p <0.01 compared to the Young group (t-test), ### p <0.001 compared to the PBS group (Dunnett test).

### DETAILED DESCRIPTION OF THE INVENTION

The present invention relates to the embodiments as characterized in the claims, disclosed in the description and illustrated in the Examples and Figures further below. Generally, the present invention relates to recombinant, human-derived, preferably monoclonal antibodies that bind to human Collapsin Response Mediator Protein 2 (CRMP2) and to equivalent binding molecules like binding fragments and derivatives thereof. In particular, the present invention relates to an anti-CRMP2 antibody with unique binding characteristics, *i.e.,* which is capable of binding full length non-phosphorylated CRMP2 and phosphorylated CRMP2 (pCRMP2) and which preferably binds an epitope which comprises the amino acid sequence 516-ASSAK-520 (SEQ ID NO: 64).

For the avoidance of any doubt it is emphasized that the expressions "in some embodiments", "in a certain embodiment", "in certain instances", "in some instances", "in a further embodiment", "in one embodiment" and the like are used and meant such that any of the embodiments described therein are to be read with a mind to combine each of the features of those embodiments and that the disclosure has to be treated in the same way as if the combination of the features of those embodiments would be spelled out in one embodiment. The same is true for any combination of embodiments and features of the appended claims and illustrated in the Examples, which are also intended to be combined with features from corresponding embodiments disclosed in the description, wherein only for the sake of consistency and conciseness the embodiments are characterized by dependencies while in fact each embodiment and combination of features, which could be construed due to the (multiple) dependencies must be seen to be literally disclosed and not considered as a selection among different choices.

Unless otherwise stated, a term as used herein is given the definition as provided in the Oxford Dictionary of Biochemistry and Molecular Biology, Oxford University Press, 1997, revised 2000 and reprinted 2003, ISBN 0 19 850673 2; Second edition published 2006, ISBN 0-19-852917-1 978-0-19852917-0.

The term "derivative" refers to synthetic and biotechnological derivatives of an antibody meaning any engineered antibody and CRMP2 binding molecule, synthesized by chemical or recombinant techniques, which retain the functional properties of the parent human and human-derived antibody, respectively.

Furthermore, when reference is made to the anti-CRMP2 antibody of the present invention, equivalent binding molecules like binding fragments and derivatives thereof are also encompassed by this definition.

As explained herein, based on antibody NI-504.3E7, variant antibodies NI-504.3E7_V1 to NI-504.3E7_V40 have been generated. Thus, when reference is made to the antibody of the present invention, not only antibody NI-504.3E7, equivalent binding molecules like binding fragments and derivatives thereof and competing antibodies are included, but also the corresponding variants. In particular, the results of the experiments performed with the original antibody NI-504.3E7 have been confirmed with one or more of the variants.

Furthermore, regarding CRMP2 protein and anti-CRMP2 antibodies, their recombinant production in a host cell, purification, modification, formulation in a pharmaceutical composition and therapeutic use as well as terms and feature common in the art can be relied upon by the person skilled in art when carrying out the present invention as claimed; see, e.g., Antibodies A Laboratory Manual 2nd edition, 2014 by Cold Spring Harbor Laboratory Press, Cold Spring Harbor, New York, USA, wherein also antibody purification and storage; engineering antibodies, including use of degenerate oligonucleotides, 5'-RACE, phage display, and mutagenesis, immunoblotting protocols and the latest screening and labeling techniques are described.

As further illustrated in the Examples, the antibodies of the present invention have been originally isolated from human donors and are shown to specifically recognize at least human full length CRMP2, and optionally murine and rat full length CRMP2; see Example 7.

CRMP2 sequences are highly conserved across mammalian species and since antibody NI-504.3E7 binds to human, murine and rat CRMP2 it is prudent to expect that the antibody of the present invention binds to mammalian CRMP2 in general. Accordingly, in one embodiment, the antibody of the present invention is capable of specifically binding to mammalian CRMP2, preferably to recombinant full length mammalian CRMP2. In a preferred embodiment, the antibody of the present invention is capable of binding recombinant human CRMP2, in particular recombinant human full length CRMP2. In one embodiment, the antibody of the present invention is also capable to bind to murine and rat CRMP2, in particular to recombinant full length murine and rat CRMP2.

Furthermore, antibody NI-504.3E7 does not bind to *E. coli* cell lysates, confirming that there is no cross-reaction when recombinant CRMP2 is produced in *E. coli.*

As further shown in the Examples, the antibody of the present invention does not only specifically bind human, and optionally murine and rat full length CRMP2, but also binds selectively to CRMP2. In particular, it has been shown (see Example 2) that the antibody of the present invention binds to human full length CRMP2, but not to human full length CRMP1, CRMP3, CRMP4, and CRMP5 on a Western Blot. Accordingly, in one embodiment, the antibody of the present invention binds selectively to CRMP2, and preferably does not bind or does only show weak binding to CRMP1, CRMP3, CRMP4, and/or CRMP5. In one embodiment, the antibody of the present invention does not bind CRMP3, CRMP4, and CRMP5, but shows weak binding to CRMP1.

By "selectively or specifically binding CRMP2", "selectively or specifically recognizing CRMP2", "antibody selective or specific to/for CRMP2" is meant specifically, generally, and collectively, antibodies preferentially binding to the full length, either recombinant or native CRMP2 present for example in brain tissue, and optionally also to denatured CRMP2, over CRMP1, CRMP3, CRMP4, and CRMP5 as determined by ELISA, preferably by at least one order of magnitudes as illustrated in Example 2 and shown Figure 2A, and antibodies that do not substantially cross react with CRMP1, CRMP3, CRMP4, and CRMP5 as determined by Western blot as illustrated in Example 2 and shown Figure 2B. As mentioned, determination of the binding specificity of the antibody can be performed for example by Western Blot or ELISA assays as described in the Examples herein, or by an equivalent assay.

As mentioned above, CRMP2 is a phosphoprotein and can switch between the non-phosphorylated (active) and phosphorylated (not-active) form. As shown in Example 4 and in Table II, the antibody of the present invention binds full length non-phosphorylated CRMP2 and phosphorylated CRMP2 (pCRMP2) Accordingly, in one embodiment, the antibody of the present invention is capable of binding full length non-phosphorylated CRMP2 and pCRMP2 as determined by ELISA assay.

Thus, if not indicated otherwise, when reference is made to an anti-CRMP2 antibody, CRMP2 binding molecule and fragment or derivative thereof of the present invention, and to an antibody of the present invention specific to/for CRMP2, it is meant that this antibody, CRMP2 binding molecule and fragment, or derivative thereof also binds phosphorylated CRMP2 (pCRMP2). Thus, if not indicated otherwise, the terms "anti-CRMP2 antibody", and "CRMP2 binding molecule and fragment or derivative thereof" as well as "CRMP2 specific antibody" include corresponding antibodies and binding molecule and fragment or derivative thereof that bind to pCRMP2.

Antibodies with high affinity to CRMP2 and/or pCRMP2, preferably to CRMP2 and pCRMP2 are of particular interest and in order to obtain a measure of the binding affinity, the EC₅₀ of the antibodies was determined in the ELISA assay performed in Example 4. The term "EC₅₀", in the context of an *in vitro* or *in vivo* assay using an antibody or antigen-binding fragment thereof, refers to the concentration of an antibody or an antigen-binding fragment thereof that induces a response that is 50 % of the maximal response, *i.e.*, halfway between the maximal response and the baseline.

As can be derived from Table II, not only the binding affinity of antibody NI-504.3E7 has been determined, but also from 40 variants thereof (NI-504.3E7_V1 to NI-504.3E7_V40) which have amino acid substitutions in the VH and/or VL chains, and/or within the CDRs as explained in detail further below. In particular, antibody NI-504.3E7 specifically recognizes recombinant human full length CRMP2 and pCRMP2 with an EC₅₀ of 0.5 nM and 4.7 nM, respectively. The values for the variants NI-504.3E7_V1 to NI-504.3E7_V40 are listed in the following Table:

**Table II: Determination of the binding affinities of antibody NI-504.3E7 and variants thereof to full length recombinant CRMP2 and pCRMP2 by ELISA assay.**

| **Antibody** | **EC50 [nM] (CRMP2)** | **EC50 [nM] (pCRMP2)** |
|---|---|---|
| NI-504.3E7 | 0.5 | 4.7 |
| NI-504.3E7 V1 | 0.5 | 0.7 |
| NI-504.3E7 V2 | 0.3 | 0.8 |
| NI-504.3E7 V3 | 0.5 | 1.2 |
| NI-504.3E7 V4 | 0.5 | 1.3 |
| NI-504.3E7 V5 | 0.5 | 1.1 |
| NI-504.3E7 V6 | 0.5 | 1.5 |
| NI-504.3E7 V7 | 1 | 3.6 |
| NI-504.3E7_V8 | 0.5 | 1 |
| NI-504.3E7 V9 | 1.2 | 3.2 |
| NI-504.3E7_V10 | 0.9 | 2.2 |
| NI-504.3E7 V11 | 0.7 | 1.2 |
| NI-504.3E7 V12 | 0.6 | 1.9 |
| NI-504.3E7_V13 | 0.9 | 3 |
| NI-504.3E7_V14 | 0.6 | 2.5 |
| NI-504.3E7_V15 | 0.4 | 0.6 |
| NI-504.3E7_V16 | 0.3 | 0.7 |
| NI-504.3E7_V17 | 0.5 | 1.6 |
| NI-504.3E7_V18 | 0.5 | 1.5 |
| NI-504.3E7_V19 | 0.9 | 1.8 |
| NI-504.3E7_V20 | 1.2 | 13.7 |
| NI-504.3E7_V21 | 2.0 | 32.5 |
| NI-504.3E7_V22 | 2.3 | 47.0 |
| NI-504.3E7 V23 | 1.1 | 5.1 |
| NI-504.3E7_V24 | 1.4 | 46.4 |
| NI-504.3E7_V25 | 1.5 | 26.7 |
| NI-504.3E7_V26 | 0.6 | 3.5 |
| NI-504.3E7_V27 | 1.4 | 67.4 |
| NI-504.3E7_V28 | 1.1 | 8.1 |
| NI-504.3E7_V29 | 1.8 | 101.4 |
| NI-504.3E7_V30 | 0.2 | 0.6 |
| NI-504.3E7_V31 | 1.0 | 22.5 |
| NI-504.3E7_V32 | 0.3 | 0.9 |
| NI-504.3E7_V33 | 1.0 | 53.4 |
| NI-504.3E7_V34 | 1.6 | 8.9 |
| NI-504.3E7_V35 | 2.9 | 32.1 |
| NI-504.3E7_V36 | 2.8 | 99.9 |
| NI-504.3E7_V37 | 1.9 | 74.9 |
| NI-504.3E7_V38 | 1.2 | 52.4 |
| NI-504.3E7_V39 | 4.0 | 84.0 |
| NI-504.3E7_V40 | 4.1 | 89.2 |

In one embodiment, the antibodies of the present invention recognize CRMP2 with an EC₅₀ which is less than about 30 nM, preferably less than about 25 nM, preferably less than about 20 nM, preferably less than about 15 nM, preferably less than about 10 nM, preferably less than about 7.5 nM, preferably less than about 5 nM, preferably less than about 3 nM, and most preferably less than about 2 nM, In one embodiment, the antibodies of the present invention recognize pCRMP2 with an EC₅₀ which is less than about 120 mM, preferably less than about 110 mM, preferably less than about 100 mM, preferably less than about 90 mM, preferably less than about 80 nM, preferably less than about 70 nM, preferably less than about 60 nM, preferably less than about 50 nM, preferably less than about 40 nM, preferably less than about 30 mM, preferably less than about 25 nM, and most preferably less than about 20 nM. Preferably, the binding affinity to CRMP2 and pCRMP2 is within the same order of magnitude, or the binding affinity to pCRMP2 is one or two orders of magnitude lesser than the biding affinity to CRMP2. Most preferably, the antibodies of the present invention recognize CRMP2 and pCRMP2 with the EC₅₀ values mentioned above. Thus, an antibody that does not specifically bind to CRMP2/pCRMP2 is for example an antibody that binds CRMP2/pCRMP2 with an EC₅₀, which is higher than about 150 nM, more preferably higher than about 400 nM, and most preferably, the EC₅₀ is not even determinable. However, also depending on the antibody format, for example whether an IgG1, IgG4 or antibody fragments are used, like Fab fragments, the EC₅₀ values may deviate and may be for example higher than the values mentioned above and in the Examples. Accordingly, in this context the term "about" means a value which may differ from the value determined for the reference antibody in the Examples, the difference being preferably less than one order of magnitude and most preferably within the same order of magnitude, for example the EC₅₀ may be the reference value ± 10 nM.

The above-mentioned properties, *i.e.*, that antibody NI-504.3E7 shows preferential and selective binding to CRMP2 in comparison to CRMP1, CRMP3, CRMP4, and CRMP5, and that antibody NI-504.3E7 binds phosphorylated and non-phosphorylated CRMP2 has also been confirmed by analyzing the binding affinity of said antibody to corresponding CRMP and pCRMP peptides. In particular, antibody NI-504.3E7 preferentially binds a CRMP2 peptide (H-EVSVTPKTVTPASSAKTSPAKQQC-NH2 (SEQ ID NO: 70)) and pCRMP2 peptide containing three phosphor-sites T509, T514 and S522 (H-EVSV(pT)PKTV(pT)PASSAKT(pS)PAKQQC-NH2 (SEQ ID NO: 70)) over corresponding peptides derived from (p)CRMP1 ((EVPA(pT)PKYA(pT)PAPSAKS(pS)PSKHQC-NH2 (SEQ ID NO: 69)) and (p)CRMP4 (H-DLTT(pT)PKGG(pT)PAGSARG(pS)PTRPNC-NH2 (SEQ ID NO: 71) as determined by ELISA. Furthermore, antibody NI-504.3E7 preferentially binds a CRMP2 peptide (TVTPASSAKTSPAKQQAPPVRC-NH2 (SEQ ID NO: 67)) and a pCRMP2 peptide containing a single S522 phospho-site (H-TVTPASSAKT(pS)PAKQQAPPVRC-NH2 (SEQ ID NO: 67)) over corresponding peptides derived from (p)CRMP1 (H-YATPAPSAKS(pS)PSKHQPPPIRC-NH2 (SEQ ID NO: 66)) and (p)CRMP4 (H-GGTPAGSARG(pS)PTRPNPPVRNC-NH2 (SEQ ID NO: 68)).

As shown in Example 6, antibody NI-504.3E7 specifically binds the CRMP2 peptide VCEVSVTPKTVTPASSAKTSPAKQQA (SEQ ID NO: 72) and different pCRMP2 peptides containing each one phospho-site (T509, T514, or S522) (VCEVSV(p)T₅₀₉PKTVTPASSAKTSPAKQQA (SEQ ID NO: 72), VCEVSVTPKTV(p)T₅₁₄PASSAKTSPAKQQA (SEQ ID NO: 72), and VCEVSVTPKTVTPASSAKT(p)S₅₂₂PAKQQA (SEQ ID NO: 72), respectively) with different affinities. In particular, the affinity of antibody NI-504.3E7 to the CRMP2 peptide with phosphorylation at position T514 was shown to be about 300-fold lower than the non-phosphorylated peptide and the peptides with phosphorylation at positions T509 and S522. Accordingly, it one embodiment, the antibody of the present binds phosphorylated and non-phosphorylated CRMP2 peptides, wherein the phosphorylated peptides are preferably phosphorylated at T509, T514, and/or S522. Preferably, the antibody of the present invention binds less preferable a CRMP2 peptide with phosphorylation at position T514, preferably the one mentioned above, in comparison to a non-phosphorylated CRMP2 peptide and to CRMP2 peptides with phosphorylation at positions T509 and S522, preferably in comparison to the ones mentioned above.

It has been further shown in a phosphorylation assay as described in Example 5 that the antibody of the present invention reduces the level of pCRMP2 in a concentration-dependent manner. In fact, this experiment has been performed exemplarily with the variant NI-504.3E7_V20 to further support the fact that the variant antibodies remain the binding specificity and activity of the original antibody. As can be seen in Table II, the binding affinity (as represented by the EC₅₀ value) of NI-504.3E7_V20 towards CRMP2 and pCRMP2 is decreased in comparison to the original antibody. Accordingly, since this variant has sufficient activity to reduce the level of pCRMP2 in a phosphorylation assay, the other variants should be able to do so as well.

This activity was not observed for an anti-CRMP2 control antibody which binds a different epitope as described in detail above and also not for a human IgG control antibody. Accordingly, this feature seems to be unique for the antibody of the present invention. In particular, the Western Blot images showed that in comparison to a "no antibody control" and the "human IgG antibody control", the amount of T509 phosphorylated CRMP2, of T514 phosphorylated CRMP2, and of S522 phosphorylated CRMP2 decreased when subjecting the antibody of the present invention and CRMP2 to a phosphorylation assay for 1 h and for 24 h under appropriate conditions to phosphorylate T509, T514, and S522, respectively in the CRMP2 protein, *i.e.,* in the presence of glycogen synthase kinase-3 (GSK-3)β and cyclin-dependent kinase (Cdk) 5/p35. Furthermore, under most conditions, the decrease was dependent on the concentration of the subject antibody, *i.e.*, the higher the amount of the antibody of the present invention, the stronger was the decrease of phosphorylated CRMP2.

Thus, in one embodiment, the antibody of the present invention is capable of reducing the level of pCRMP2, preferably of T509 pCRMP2, T514 pCRMP2, and/or S522 pCRMP2, in a concentration-dependent manner when the antibody and the CRMP2 protein are both subjected to a phosphorylation assay, as compared to a control. Preferably, the phosphorylation assay is performed for 1 h or 24 h under appropriate conditions in the presence of GSK-3β and Cdk 5/p35 and Western Blot analysis is performed to analyze the results. Accordingly, in a preferred embodiment, the antibody of the present invention is capable of reducing the level of pCRMP2 in a concentration-dependent manner when subjecting the antibody and CRMP2 to a phosphorylation assay for 1h or 24h under appropriate conditions in the presence of glycogen synthase kinase-3 (GSK-3)β and Cdk 5/p35, as compared to a control and determined by Western Blot analysis, wherein preferably the pCRMP2 is selected from T509 pCRMP2, T514 pCRMP2 and S522 pCRMP2. In a preferred embodiment, a higher amount of the antibody leads to a stronger reduction of the level of pCRMP2, and a lower amount of the anybody leads to a lesser reduction of the level of pCRMP2.

There are different suitable controls, for example a "no antibody control", wherein no antibody is added to the phosphorylation assay, a control antibody which does not recognize CRMP2 or pCRMP2, for example a human IgG antibody control, or a CRMP2 antibody, which is known to not reduce the level of pCRMP2 in a phosphorylation assay.

Overlapping peptide arrays revealed that antibody NI-504.3E7 binds the linear peptides with the amino acid sequence TPKTVTPASSAKTSP (SEQ ID NO: 62) and VTPASSAKTSPAKQQ (SEQ ID NO: 63), with the consensus sequence VTPASSAKTSP (SEQ ID NO: 65) that are part of the C-terminal domain of CRMP2; see Example 3. The C-terminal domain extends from amino acid 490 to 572. Accordingly, in one embodiment, the antibody of the present invention binds to the C-terminal region of CRMP2 and pCRMP2. In particular, in one preferred embodiment, the antibody of the present invention binds to a peptide present in the C-terminal region of CRMP2 and pCRMP2, wherein the peptide is preferably TPKTVTPASSAKTSP (SEQ ID NO: 62) or VTPASSAKTSPAKQQ (SEQ ID NO: 63). Of course, the antibody also binds to peptides, which comprise said sequences.

Epitope mapping revealed that antibody NI-504.3E7 binds a linear peptide comprising the consensus sequence ASSAK (SEQ ID NO: 64). Accordingly, in one embodiment, the antibody of the present invention binds to an epitope which comprises or consists of the amino acid sequence ASSAK (SEQ ID NO: 64).

As shown in Example 9, antibody NI-504.3E7 is capable of reversing the increase of spine density driven by CRMP2/pCRMP2 at antibody concentrations of 7.5 µM and 0.7 µM. In particular, since it is known that CRMP2 is involved in axonal growth and since it has been shown by experiments performed within in the scope of the present invention that an anti-CRMP2 antibody acts on the spine density and preferably reduces the spine density, this feature can be used as a proof-of-activity of the antibody of the present invention and it further shows that the anti-CRMP2 antibody somehow interferes with CRMP2 signaling. Accordingly, the present invention also relates to a molecule capable of interfering with CRMP2 signaling for use as a medicament, preferably for use in a method for the prevention, delay of progression or the treatment of a neurodegenerative disorder, cognitive disorder and/or for improving memory and learning ability.

Furthermore, in one embodiment, the antibody of the present invention is capable of reversing the increase of neuronal spine density induced by CRMP2 as determined in an *ex vivo* hipocampal slice culture model, preferably at a concentration between 0.4 µM and 10 µM, preferably between 0.5 µM and 9 µM, preferably between 0.6 µM and 8 µM, and most preferably at 7.5 µM or 0.7 µM.

The increase of neuronal spine density is increased by pCRMP2 aggregates or CRMP2 monomers.

In one embodiment, the anti-CRMP2 antibodies and equivalent CRMP2 binding molecules demonstrate the immunohistochemical characteristics and *in vivo* activities of any one of the anti-CRMP2 antibodies illustrated in the Examples and Figures further below.

In particular, the antibodies, antigen-binding fragments, and derivatives thereof have at least one of the following properties (i) to (iii):
(i) detecting of neuronal cells in the hippocampus of an AD patient,
(ii) reducing of amyloid plaque load in female Tau tubulin kinase-1 (TTBK1)/ amyloid precursor protein (APP) transgenic mice, and/or
(iii) improving cognitive function in aged mice.

In more detail, IHC analysis showed that antibody NI-504.3E7 binds in hippocampal tissue, and in particular detects neuronal cells in samples of patients with tauopathies. In particular, antibody NI-504.3E7 binds in hippocampal tissue of an AD patient. Furthermore, it has been shown that antibody NI-504.3E7 binds in hippocampal tissue of a PSP patients, binds in hippocampal tissue of a PiD patient and detects neuronal cells in PiD hippocampal sections, detects neuronal cells in FTD-PD hippocampal sections, and detects neuronal cells FTLD-TDP hippocampal tissue. Further studies showed that antibody NI-504.3E7 reduced amyloid plaque load by 63% in cortex and hippocampus and amyloid plaque size by 30% in the cortex and by 68% in the hippocampus in a transgenic mouse model of Alzheimer's disease. In addition, in the cortex, a 50% reduction in the amount of plaques/area in female TTBK1/APP mice receiving antibody NI-504.3E7 was observed (area is defined by the whole cortex). It has been further shown in the experiments as described in Examples 13 and 14 that the antibody of the present invention improves the LTP and cognitive function in aged mice when administered at a dose of 0.3 mg/kg, 1 mg/kg, 3 mg/kg, or 10 mg/kg. In fact, these experiments have been performed exemplarily with the variant NI-504.3E7_V20 to further support the fact that the variant antibodies remain the activity of the original antibody as also explained above with regard to the phosphorylation assay.

Accordingly, in one embodiment, the antibody of the present invention detects neuronal cells in the hippocampus of a patient with a tauopathy, preferably of a patient with AD, PSP, PiD, FTD-PD and FTLD-TDP, most preferably AD. In one embodiment, the antibody of the present invention reduces the amyloid plaque load and/or average amyloid plaque size and/or the amount of plaques/area in a transgenic mouse model of Alzheimer's disease, preferably in female Tau tubulin kinase-1 (TTBK1)/ amyloid precursor protein (APP) transgenic mice. In a preferred embodiment, the plaque load is decreased in the cortex and/or in the hippocampus by 5% to 95%, preferably by 10% to 90%, preferably by 15% to 85%, preferably by 20% to 80%, preferably by 25% to 75%, preferably by 30% to 70%, preferably by 35% to 65%, preferably by 40% to 65%, preferably by 45% to 65%, preferably by 50% to 65%, preferably by 55% to 65%, more preferably by 60% to 65% and most preferably by 63%. In a further preferred embodiment, the average plaque size in the hippocampus is reduced by 5% to 95%, preferably by 10% to 90%, preferably by 15% to 85%, preferably by 20% to 80%, preferably by 25% to 75%, preferably by 30% to 70%, preferably by 35% to 70%, preferably by 40% to 70%, preferably by 45% to 70%, preferably by 50% to 70%, preferably by 55% to 70%, preferably by 60% to 70%, more preferably by 65% to 70% and most preferably by 68%. In a further preferred embodiment, the average plaque size in the cortex is reduced by 5% to 60%, preferably by 10% to 55%, preferably by 15% to 50%, preferably by 20% to 45%, preferably by 25% to 40%, and most preferably by 30%. In a further preferred embodiment, the amount of plaques/area is reduced by 20% to 70%, preferably by 25% to 65%, preferably by 30% to 60%, preferably by 35% to 55%, preferably by 40% to 50%, and most preferably by 50%. In one embodiment, the antibody of the present invention improves the cognitive function in aged mice, preferably when administered at a dose of 0.1 mg/kg to 10 mg/kg, preferably of 0.2 mg/kg to 10 mg/kg, preferably of 0.3 mg/kg to 10 mg/kg, preferably of 1 mg/kg to 10 mg/kg, and most preferably at a dose of 0.3 mg/kg, 1 mg/kg, 3 mg/kg, or 10 mg/kg, wherein the effect is most prominent at a dose of 1 mg/kg, 3 mg/kg, or 10 mg/kg.

The present invention also relates to an antibody or an antigen binding molecule in general which competes with the antibody of the present invention for specific binding to human CRMP2 and/or pCRMP2 and which retains one or more of the binding characteristics and functional features, respectively, as described for the antibody of the present invention. Preferably, the competing antibody and antigen binding molecule have at least one of the following properties (i) to (iii), preferably at least two of the following features (i) to (iii), *i.e.,* (i) and (ii), or (i) and (iii), or (ii) and (iii), most preferably all of the three following properties (i) to (iii):
(i) preferentially recognizes human CRMP2 over CRMP1, CRMP3, CRMP4 and CRMP5, which can be tested in ELISA assays and Western Blot analysis as described in Example 2,
(ii) is capable of reducing the level of pCRMP2 in a concentration-dependent manner when subjecting CRMP2 to a phosphorylation assay, which can be determined as described in Example 5,
(iii) is capable of reversing the increase of neuronal spine density induced by CRMP2 as determined in an *ex vivo* hippocampal slice culture model, which can be tested as described in Example 9.

In one embodiment, the present invention relates to an antibody, which recognize the same epitope than antibody NI-504.3E7, and which retains any one of, preferably all of the above-mentioned binding characteristics (i) to (iii). A competing antibody can be identified in a simple immunoassay showing the ability of one antibody to block the binding of another antibody to a target antigen *(e.g.,* an electrochemiluminescence assay, a competitive ELISA, a solid phase radioimmunoassay (SPRIA) or a blocking Western Blot).

The present invention is illustrated with anti-CRMP2 antibodies and antigen-binding fragments thereof which are characterized by comprising in their variable region, *i.e.,* binding domain, the variable heavy (VH) and variable light (VL) chain having the amino acid sequences depicted in Figure 1. The corresponding nucleotide and amino acid sequences are set forth in Table IV below. In particular, Figure 1 shows VH and VL chains of antibody NI-504.3E7 and 40 variants thereof (NI-504.3E7_V1 to NI-504.3E7_V40), in which amino acid substitutions have been performed in the VH chain, the VL chain, and/or the CDRs. The CDRs are underlined in the corresponding Figure and Table. Corresponding amino acid substitutions can be performed with conventional methods known in the art, for example PCR.

As always, the variable domains of each chain contain three hypervariable loops named complementarity determining regions (CDRs, CDR-1,-2, and -3). The CDRs are separated by structurally conserved regions called framework regions (FR-1,-2,-3, and -4) that form a "core" β-sheet structure displaying these loops on the surface of the variable domain. The length and composition of the CDR sequences are highly variable, especially in the CDR3. The CDRs are approximated to the paratope of the antibody that interacts with the antigen and therefore contains the antigen-binding residues. Accordingly, it is common to define an antibody by its six CDRs. Exemplary sets of CDRs in the above amino acid sequences of the VH and VL chains are depicted in Figure 1 and Tables IV and V. However, as discussed in the following, the person skilled in the art is well aware of the fact that in addition or alternatively CDRs may be used, which differ in their amino acid sequence from those set forth in any one of Figure 1 by one, two, three or even more amino acids, especially in case of CDR2 and CDR3. As mentioned in the Figure legend of Figure 1, the person skilled in the art can easily identify the CDRs according to common principles, for example as summarized in www.bioinf.org.uk/abs. In this context, while the CDRs of the antibodies depicted in Figure 1 are indicated according to Kabat *et al.* the person skilled in the art knows that a number of definitions of the CDRs are commonly in use, *i.e.* the (i) Kabat definition based on sequence variability, which is the most commonly used; (ii) Chothia definition based on the location of the structural loop regions; (iii) AbM definition as a compromise between the two used by Oxford Molecular's *AbM* antibody modelling software; and (iv) Contact definition that has been recently introduced by and is based on an analysis of the available complex crystal structures. This definition is likely to be the most useful for performing mutagenesis to modify the affinity of an antibody since these are residues which take part in interactions with the antigen. For lists of CDR residues making contact in each antibody with summary data for each CDR see, e.g., www.bioinf.org.uk/abs which also refers to antibody modelling software such as abYmod available at abymod.abysis.org. Table III below depicts the relation between the CDR positions defined by the different concepts.

**Table I: Different concepts of CDR definitions. ¹ some of these definitions (particularly for Chothia loops) vary depending on the individual publication examined; ² any of the numbering schemes can be used for these CDR definitions, except the contact definition uses the Chothia or Martin (Enhanced Chothia) definition; ³ the end of the Chothia CDR-H1 loop when numbered using the Kabat numbering convention varies between H32 and H34 depending on the length of the loop. (This is because the Kabat numbering scheme places the insertions at H35A and H35B.)**

| **Loop** | **Kabat** | **AbM** | **Chothia¹** | **Contact²** | **IMGT** |
|---|---|---|---|---|---|
| **L1** | L24--L34 | L24--L34 | L24--L34 | L30--L36 | L27--L32 |
| **L2** | L50--L56 | L50--L56 | L50--L56 | L46--L55 | L50--L51 |
| **L3** | L89--L97 | L89--L97 | L89--L97 | L89--L96 | L89--L97 |
| **H1** | H31--H35B (Kabat Numbering)³ | H26--H35B | H26--H32..34 | H30--H35B | H26-- H3 5B |
| **H1** | H31--H35 (Chothia Numbering) | H26--H35 | H26--H32 | H3 0--H3 5 | H26--H33 |
| **H2** | H50--H65 | H50--H58 | H52--H56 | H47--H58 | H51--H56 |
| **H3** | H95--H102 | H95--H102 | H95--H102 | H93--H101 | H93--H102 |

For the mentioned definitions see also Kontermann and Dübel (eds.), Antibody Engineering Vol. 2, DOI 10.1007/978-3-642-01147-4_3, # Springer-Verlag Berlin Heidelberg 2010, in particular Chapter 3, Protein Sequence and Structure Analysis of Antibody Variable Domains at pages 33-51 and Dondelinger et al., Front. Immunol. 9 (2018), 2278 specifically dealing with understanding the significance and implications of antibody numbering and antigen-binding surface/residue definition; see, *e.g.,* Dondelinger *etal.,* Fig. 4 and Fig. 6 illustrating the disparity in the classical CDR definitions according to Kabat *supra,* Chothia (Chothia and Lesk, J. Mol. Biol. 196 (1987), 901-917), Contact (MacCallum et al, J. Mol. Biol. 262 (1996), 732-745) and IMGT (IMGT^{®}, the international ImMunoGeneTics information system^{®}, www.imgt.org). The AbM definition is a compromise between the two used by Oxford Molecular's AbM antibody modelling software.

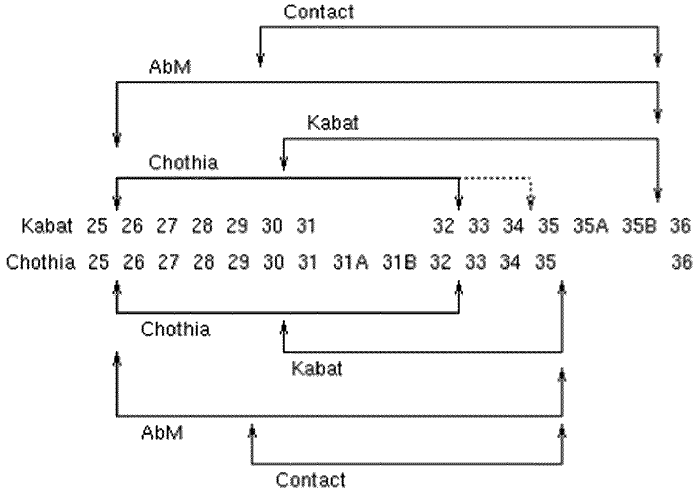

This above diagram illustrates the alternative definitions for CDR-H1 (VH-CDR1). The Kabat and Chothia numbering schemes are shown horizontally and the Kabat, Chothia, AbM and Contact definitions of the CDRs are shown with arrows above and below the two numbering schemes. Accordingly, based on the VH and V chain amino acid sequences provided for the two subject antibodies NI-504.3E7 as the corresponding variants thereof, their epitope and the assays described in the Examples the skilled person is able to design and arrive at equivalent antibodies and like CRMP2 binding molecules, which differ in one or more of their CDRs as defined by Kabat from the corresponding CDRs of the parent subject antibody but substantially retain the CDRs as defined by Chothia. During experiments performed within the scope of the present invention, it has been even proven that certain amino acid substitutions do not substantially alter the binding affinity of the original antibody NI-504.3E7. As indicated in Figure 1 and for example in Table VII, certain amino acid substitutions are present in the VH chain and/or the VL chain of the variant antibodies. Furthermore, in NI-504.3E7_V15, NI-504.3E7_V19, NI-504.3E7_V36, and NI-504.3E7_V39, there is even an amino acid substitution in the VL-CDR3. As shown in Example 4 and Table II, all variants maintained their capability to bind CRMP2 and pCRMP2. Furthermore, the results of the experiments performed with the original antibody NI-504.3E7 as described above have been confirmed with one or more of the 40 variants.

In one embodiment, the anti-CRMP2 antibody, antigen-binding fragment, or derivative thereof or CRMP2 binding molecule of the present invention comprises a variable heavy (VH) chain comprising VH complementarity determining regions (VH-CDRs) 1, 2, and 3, and/or a variable light (VL) chain comprising VL-CDRs 1, 2, and 3 as defined by Kabat, wherein
NI-504.3E7
   (a) VH-CDR1 comprises the amino acid sequence of SEQ ID NO: 3 as shown in Figure 1 or Table V or a variant thereof, wherein the variant comprises an amino acid sequence resulted from a partial alteration of SEQ ID NO: 3,
   (b) VH-CDR2 comprises the amino acid sequence of SEQ ID NO: 4 as shown in Figure 1 or Table V or a variant thereof, wherein the variant comprises an amino acid sequence resulted from a partial alteration of SEQ ID NO: 4,
   (c) VH-CDR3 comprises the amino acid sequence of SEQ ID NO: 5 as shown in Figure 1 or Table V or a variant thereof, wherein the variant comprises an amino acid sequence resulted from a partial alteration of SEQ ID NO: 5,
   (d) VL-CDR1 comprises the amino acid sequence of SEQ ID NO: 8 as shown in Figure 1 or Table V or a variant thereof, wherein the variant comprises an amino acid sequence resulted from a partial alteration of SEQ ID NO: 8,
   (e) VL-CDR2 comprises the amino acid sequence of SEQ ID NO: 9 as shown in Figure 1 or Table V or a variant thereof, wherein the variant comprises an amino acid sequence resulted from a partial alteration of SEQ ID NO: 9, and
   (f) VL-CDR3 comprises the amino acid sequence of SEQ ID NO: 10 as shown in Figure 1 or Table V or a variant thereof, wherein the variant comprises an amino acid sequence resulted from a partial alteration of SEQ ID NO: 10; or
[NI-504.3E7_V15, NI-504.3E7_V19, NI-504.3E7_V36, and NI-504.3E7_V39]
   (a) VH-CDR1 comprises the amino acid sequence of SEQ ID NO: 3 as shown in Figure 1 or Table V or a variant thereof, wherein the variant comprises an amino acid sequence resulted from a partial alteration of SEQ ID NO: 3,
   (b) VH-CDR2 comprises the amino acid sequence of SEQ ID NO: 4 as shown in Figure 1 or Table V or a variant thereof, wherein the variant comprises an amino acid sequence resulted from a partial alteration of SEQ ID NO: 4,
   (c) VH-CDR3 comprises the amino acid sequence of SEQ ID NO: 5 as shown in Figure 1 or Table V or a variant thereof, wherein the variant comprises an amino acid sequence resulted from a partial alteration of SEQ ID NO: 5,
   (d) VL-CDR1 comprises the amino acid sequence of SEQ ID NO: 8 as shown in Figure 1 or Table V or a variant thereof, wherein the variant comprises an amino acid sequence resulted from a partial alteration of SEQ ID NO: 8,
   (e) VL-CDR2 comprises the amino acid sequence of SEQ ID NO: 9 as shown in Figure 1 or Table V or a variant thereof, wherein the variant comprises an amino acid sequence resulted from a partial alteration of SEQ ID NO: 9, and
   (f) VL-CDR3 comprises the amino acid sequence of SEQ ID NO: 61 (VL_3E7_var3) as shown in Figure 1 or Table V or a variant thereof, wherein the variant comprises an amino acid sequence resulted from a partial alteration of SEQ ID NO: 61.

In a preferred embodiment, the variant CDR comprises one or more amino acid substitutions, preferably one, two, three or four, preferably one or two amino acid substitutions, most preferably one amino acid substitution. In a preferred embodiment, only the VL-CDR3 comprises one or more amino acid substitutions, preferably one, two, three or four, preferably one or two amino acid substitutions, most preferably one amino acid substitution.

In another preferred embodiment, the variant CDR comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 3, SEQ ID NO: 4, SEQ ID NO: 5, SEQ ID NO: 8, SEQ ID NO: 9, SEQ ID NO: 10, and SEQ ID NO: 61, respectively. More preferably, only the VL-CDR3 comprises a variant amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 61.

In addition, or alternatively the antibody or antigen-binding fragment thereof of the present invention can be characterized as follows:
NI-504.3E7
   (i) the VH chain comprises the amino acid sequence depicted in SEQ ID NO: 2 (VH_3E7) as shown in Figure 1 or Table IV or a variant thereof, wherein the variant comprises an amino acid sequence resulted from a partial alteration of SEQ ID NO: 2; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7.
NI-504.3E7_V1
   (i) the VH chain comprises the amino acid sequence depicted in SEQ ID NO: 2 (VH_3E7) as shown in Figure 1 or Table IV or a variant thereof, wherein the variant comprises an amino acid sequence resulted from a partial alteration of SEQ ID NO: 2; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V2
   (i) the VH chain comprises the amino acid sequence depicted in SEQ ID NO: 2 (VH_3E7) as shown in Figure 1 or Table IV or a variant thereof, wherein the variant comprises an amino acid sequence resulted from a partial alteration of SEQ ID NO: 2; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V3
   (i) the VH chain comprises the amino acid sequence depicted in SEQ ID NO: 12 (VH_3E7_var1) as shown in Figure 1 or Table IV or a variant thereof, wherein the variant comprises an amino acid sequence resulted from a partial alteration of SEQ ID NO: 12; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7.
NI-504.3E7_V4
   (i) the VH chain comprises the amino acid sequence depicted in SEQ ID NO: 12 (VH_3E7_var1) as shown in Figure 1 or Table IV or a variant thereof, wherein the variant comprises an amino acid sequence resulted from a partial alteration of SEQ ID NO: 12; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V5
   (i) the VH chain comprises the amino acid sequence depicted in SEQ ID NO: 12 (VH_3E7_var1) as shown in Figure 1 or Table IV or a variant thereof, wherein the variant comprises an amino acid sequence resulted from a partial alteration of SEQ ID NO: 12; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V6
   (i) the VH chain comprises the amino acid sequence depicted in SEQ ID NO: 14 (VH_3E7_var2) as shown in Figure 1 or Table IV or a variant thereof, wherein the variant comprises an amino acid sequence resulted from a partial alteration of SEQ ID NO: 14; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7.
NI-504.3E7_V7
   (i) the VH chain comprises the amino acid sequence depicted in SEQ ID NO: 14 (VH_3E7_var2) as shown in Figure 1 or Table IV or a variant thereof, wherein the variant comprises an amino acid sequence resulted from a partial alteration of SEQ ID NO: 14; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V8
   (i) the VH chain comprises the amino acid sequence depicted in SEQ ID NO: 14 (VH_3E7_var2) as shown in Figure 1 or Table IV or a variant thereof, wherein the variant comprises an amino acid sequence resulted from a partial alteration of SEQ ID NO: 14; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V9
   (i) the VH chain comprises the amino acid sequence depicted in SEQ ID NO: 16 (VH_3E7_var3) as shown in Figure 1 or Table IV or a variant thereof, wherein the variant comprises an amino acid sequence resulted from a partial alteration of SEQ ID NO: 16; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7.
NI-504.3E7_V10
   (i) the VH chain comprises the amino acid sequence depicted in SEQ ID NO: 16 (VH_3E7_var3) as shown in Figure 1 or Table IV or a variant thereof, wherein the variant comprises an amino acid sequence resulted from a partial alteration of SEQ ID NO: 16; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V11
   (i) the VH chain comprises the amino acid sequence depicted in SEQ ID NO: 16 (VH_3E7_var3) as shown in Figure 1 or Table IV or a variant thereof, wherein the variant comprises an amino acid sequence resulted from a partial alteration of SEQ ID NO: 16; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58
NI-504.3E7_V12
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7.
NI-504.3E7_V13
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V14
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V15
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60.
NI-504.3E7_V16
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7.
NI-504.3E7_V17
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V18
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V19
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60.
NI-504.3E7_V20
   (i) the VH chain comprises the amino acid sequence depicted in SEQ ID NO: 22 (VH_3E7_var6) as shown in Figure 1 or Table IV or a variant thereof, wherein the variant comprises an amino acid sequence resulted from a partial alteration of SEQ ID NO: 22; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V21
   (i) the VH chain comprises the amino acid sequence depicted in SEQ ID NO: 24 (VH_3E7_var6a) as shown in Figure 1 or Table IV or a variant thereof, wherein the variant comprises an amino acid sequence resulted from a partial alteration of SEQ ID NO: 24; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V22
   (i) the VH chain comprises the amino acid sequence depicted in SEQ ID NO: 26 (VH_3E7_var6b) as shown in Figure 1 or Table IV or a variant thereof, wherein the variant comprises an amino acid sequence resulted from a partial alteration of SEQ ID NO: 26; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V23
   (i) the VH chain comprises the amino acid sequence depicted in SEQ ID NO: 28 (VH_3E7_var6c) as shown in Figure 1 or Table IV or a variant thereof, wherein the variant comprises an amino acid sequence resulted from a partial alteration of SEQ ID NO: 28; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V24
   (i) the VH chain comprises the amino acid sequence depicted in SEQ ID NO: 30 (VH_3E7_var6d) as shown in Figure 1 or Table IV or a variant thereof, wherein the variant comprises an amino acid sequence resulted from a partial alteration of SEQ ID NO: 30; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V25
   (i) the VH chain comprises the amino acid sequence depicted in SEQ ID NO: 32 (VH_3E7_var6ab) as shown in Figure 1 or Table IV or a variant thereof, wherein the variant comprises an amino acid sequence resulted from a partial alteration of SEQ ID NO: 32; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V26
   (i) the VH chain comprises the amino acid sequence depicted in SEQ ID NO: 34 (VH_3E7_var6ac) as shown in Figure 1 or Table IV or a variant thereof, wherein the variant comprises an amino acid sequence resulted from a partial alteration of SEQ ID NO: 34; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V27
   (i) the VH chain comprises the amino acid sequence depicted in SEQ ID NO: 36 (VH_3E7_var6ad) as shown in Figure 1 or Table IV or a variant thereof, wherein the variant comprises an amino acid sequence resulted from a partial alteration of SEQ ID NO: 36; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V28
   (i) the VH chain comprises the amino acid sequence depicted in SEQ ID NO: 38 (VH_3E7_var6bc) as shown in Figure 1 or Table IV or a variant thereof, wherein the variant comprises an amino acid sequence resulted from a partial alteration of SEQ ID NO: 38; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V29
   (i) the VH chain comprises the amino acid sequence depicted in SEQ ID NO: 40 (VH_3E7_var6bd) as shown in Figure 1 or Table IV or a variant thereof, wherein the variant comprises an amino acid sequence resulted from a partial alteration of SEQ ID NO: 40; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V30
   (i) the VH chain comprises the amino acid sequence depicted in SEQ ID NO: 42 (VH_3E7_var6cd) as shown in Figure 1 or Table IV or a variant thereof, wherein the variant comprises an amino acid sequence resulted from a partial alteration of SEQ ID NO: 42; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V31
   (i) the VH chain comprises the amino acid sequence depicted in SEQ ID NO: 44 (VH_3E7_var6bcd) as shown in Figure 1 or Table IV or a variant thereof, wherein the variant comprises an amino acid sequence resulted from a partial alteration of SEQ ID NO: 44; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V32
   (i) the VH chain comprises the amino acid sequence depicted in SEQ ID NO: 46 (VH_3E7_var6acd) as shown in Figure 1 or Table IV or a variant thereof, wherein the variant comprises an amino acid sequence resulted from a partial alteration of SEQ ID NO: 46; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V33
   (i) the VH chain comprises the amino acid sequence depicted in SEQ ID NO: 48 (VH_3E7_var6abd) as shown in Figure 1 or Table IV or a variant thereof, wherein the variant comprises an amino acid sequence resulted from a partial alteration of SEQ ID NO: 48; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V34
   (i) the VH chain comprises the amino acid sequence depicted in SEQ ID NO: 50 (VH_3E7_var6abc) as shown in Figure 1 or Table IV or a variant thereof, wherein the variant comprises an amino acid sequence resulted from a partial alteration of SEQ ID NO: 50; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V35
   (i) the VH chain comprises the amino acid sequence depicted in SEQ ID NO: 22 (VH_3E7_var6) as shown in Figure 1 or Table IV or a variant thereof, wherein the variant comprises an amino acid sequence resulted from a partial alteration of SEQ ID NO: 22; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V36
   (i) the VH chain comprises the amino acid sequence depicted in SEQ ID NO: 22 (VH_3E7_var6) as shown in Figure 1 or Table IV or a variant thereof, wherein the variant comprises an amino acid sequence resulted from a partial alteration of SEQ ID NO: 22; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60.
NI-504.3E7_V37
   (i) the VH chain comprises the amino acid sequence depicted in SEQ ID NO: 52 (VH_3E7_var7) as shown in Figure 1 or Table IV or a variant thereof, wherein the variant comprises an amino acid sequence resulted from a partial alteration of SEQ ID NO: 52; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56.
NI-504.3E7_V38
   (i) the VH chain comprises the amino acid sequence depicted in SEQ ID NO: 52 (VH_3E7_var7) as shown in Figure 1 or Table IV or a variant thereof, wherein the variant comprises an amino acid sequence resulted from a partial alteration of SEQ ID NO: 52; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.
NI-504.3E7_V39
   (i) the VH chain comprises the amino acid sequence depicted in SEQ ID NO: 52 (VH_3E7_var7) as shown in Figure 1 or Table IV or a variant thereof, wherein the variant comprises an amino acid sequence resulted from a partial alteration of SEQ ID NO: 52; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60.
NI-504.3E7_V40
   (i) the VH chain comprises the amino acid sequence depicted in SEQ ID NO: 54 (VH_3E7_var8) as shown in Figure 1 or Table IV or a variant thereof, wherein the variant comprises an amino acid sequence resulted from a partial alteration of SEQ ID NO: 54; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.

In a preferred embodiment, the variant VH and VL, respectively, comprises one or more amino acid substitutions and/or deletions, preferably one, two, three, four, five, six, seven, eight, nine, or ten amino acid substitutions and/or deletions, preferably wherein the variant of the VL chain comprises one, two, three, four, or five, preferably two, four, or five amino acid substitutions and/or deletions, and/or the variant of the VH chain comprises one, two, three, four, five, six, seven, eight, nine, or ten, preferably two, three, four, five six, seven, eight, or ten amino acid substitutions and/or deletions. Most preferably, the variant VH and VL, respectively comprises one or two amino acid substitutions and/or deletions. For example, the variant VL comprises amino acid deletions in which the N-terminal amino acid is deleted by post-translational modification, in one embodiment, in which the first residue serine is deleted during signal peptide deletion.

In another preferred embodiment, the variant of the mentioned VH and VL chains, respectively comprises an amino acid sequence which is at least 90%, preferably 91%, preferably 92%, preferably 93%, preferably 94%, preferably 95%, preferably 96%, preferably 97%, preferably 98%, preferably 99% identical to the sequences set forth in SEQ ID NO: 2, SEQ ID NO: 7, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, respectively.

In these embodiments, preferably one or more of the CDRs according to the Kabat definition are maintained substantially unchanged. However, under the simplified assumption that the paratope corresponds to the CDRs, the Chothia definition of the CDRs may be used in addition or alternatively as they correlate very well with the structural loops present in the variable regions. Thus, in order to provide anti-CRMP2 antibodies equivalent to subject antibody NI-504.3E7 or to any one of the variants V1 to V40, preferably at least one or two of said one or more, preferably not more than two amino acid substitutions, and most preferably one amino acid substitution if made in the CDRs as defined according to Kabat are made outside the CDRs as defined by Chothia and/or IMGT and most preferably outside the overlap of the CDRs as defined according to Kabat and Chothia.

For example, regarding amino acid substitutions within the CDRs, variable heavy and light chain and framework amino acid sequences, respectively, preferably conservative amino acid substitutions are performed for example in accordance with the most frequently exchanged amino acids as analyzed and described by Mirsky et al., Mol. Biol. Evol. 32 (2014), 806-819; see Figure 6 at page 813 of Mirsky *et al.* In particular, within VH-CDR1, S may be substituted with T; within VH-CDR3, V may be substituted with E, T may be substituted with S and/or M may be substituted with V; within VL-CDR1, R may be substituted with K, R may be substituted with E, and/or T may be substituted; within VL-CDR2, S may be substituted with A and/or A may be substituted with G; and in VL-CDR3, P may be substituted with S. As mentioned, preferably amino acid substitutions are selected which belong to the same category in either or preferably both models LG and AB shown in Figure 6 of Mirsky *et al.* (2014), *supra,* with the LG model being preferred for the tendency to keep amino acid properties, and wherein the amino acid substitutions are selected preferably such that the physiochemical properties of the original amino acid is substantially maintained, *i.e.* hydrophobic, polar or charged property or for example that in case two or more amino acid substitutions are performed, they compensate each other so as to provide the physicochemical property of the surface all together.

Of course, besides theoretical considerations also experimental approaches exist for identifying CDR variants within a reasonable time and undue burden. For example, Tiller et al., in Front Immunol. 8 (2017), 986 describe facile affinity maturation of antibody variable domains using natural diversity mutagenesis. Indeed, already a few years earlier Rajpal et al., in PNAS 102 (2005), 8466-8471 reported a general method for greatly improving the affinity of antibodies by using combinatorial libraries and illustrated their method with anti-TNF-α antibody D2E7 (HUMIRA^{©}) identifying 38 substitutions in 21 CDR positions that resulted in higher affinity binding to TNF-α. More recently, Cannon et al., in PLOS Computational Biology, https://doi.org/10.1371/journal.pcbi.1006980 May 1, 2019 described experimentally guided computational antibody affinity maturation with *de novo* docking, modelling and rational design *in silico* affinity maturation, together with alanine scanning, that allowed fine-tuning the protein-protein docking model to subsequently enable the identification of two single-point mutations that increase the affinity of a hybridoma-derived antibody, AB 1 for its antigen murine CCL20.

Accordingly, though each antibody is unique and may have distinct features, nevertheless once a lead candidate has been provided the person skilled in the art in consideration of the teaching of the present invention as disclosed in the present application, as well as in view of the computational design and experimental approaches developed so far is able to arrive at equivalent anti-CRMP2 antibodies which keep the desired features of the antibody such as those described for the anti-CRM2 antibodies illustrated in the Examples and specifically defined in the claims. In this context, it is well understood that the variant antibody substantially maintains the binding specificity of the parent antibody, for example binding to the peptides/epitopes defined above; binding CRMP2 and pCRMP2, selectively binding to CRMP2 and not specifically to CRMP1, CRMP3, CRMP4, and CRMP5, reducing the level of pCRMP2 in a concentration-dependent manner in a phosphorylation assay, reversing the increase of neuronal spine density induced by CRMP2 as determined in an ex vivo hippocampal slice culture model, and/or that the variant antibody competes with the parent antibody, *i.e.,* with antibody NI-504.3E7 and any one of the variants NI-504.3E7_V1 to NI-504.3E7_V40, for binding to the respective epitope.

As known in the art, CDR3 of the variable heavy chain (VH-CDR3) seems to mainly determine antigen specificity; see, *e.g.,* Xu and Davis, Immunity 13 (2000), 37-45. In this context, it was noted that it is the diversity of heavy-chain CDR3s that drive specificity, whereas VH-CDR1 and VH-CDR2 residues are broadly cross-reactive and subject to improvement by somatic hypermutation; see Davis, Semin. Immunol. 16 (2004), 239-243. Accordingly, in one embodiment the antibody of the present invention, which has the immunological characteristics of the reference antibody and being capable of competing with its binding to CRMP2 at the respective epitope comprises in its variable region at least VH-CDR3 of the corresponding reference antibody or a VH-CDR3 which amino acid sequence is at least 90% identical to the reference VH-CDR3, preferably 95% identical and more preferably 96%, 97%, 98%, 99% or 100% identical. For example, a variant antibody of a reference antibody may retain VH-CDR3 of the reference (parent) antibody while VH-CDR1 and/or VH-CDR2 may contain one or more amino acid substitutions; see *supra.*

However, next to the theoretical and practical considerations made in the literature, experiments performed within the scope of the present invention have shown that the generated variant antibodies (NI-504.3E7_V1 to NI-504.3E7_V40) maintain the binding characteristics of the original antibody NI-504.3E7. For example, as shown in Table II, all variants still bind phosphorylated and non-phosphorylated CRMP2. Furthermore, the results of the experiments performed within the scope of the present invention have been confirmed with one or more of the variants.

Accordingly, in further preferred embodiment, amino acid substitutions are present in the VL-CDR3, preferably wherein the VL-CDR3 comprises one or more amino acid substitutions, preferably one, two, three or four, preferably one or two amino acid substitutions, most preferably one amino acid substitution. In a further preferred embodiment, the amino acid substitution is within the VL-CDR3, preferably at position 91 of the VL chain, and the amino acid substitution is most preferably D91E.

In another further preferred embodiment, the variant CDR comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 10, and SEQ ID NO: 61, respectively.

Accordingly, in one embodiment, as regards NI-504.3E7, which is characterized by
(a) VH-CDR1 comprises the amino acid sequence of SEQ ID NO: 3 as shown in Figure 1 or Table V or a variant thereof, wherein the variant comprises an amino acid sequence resulted from a partial alteration of SEQ ID NO: 3,
(b) VH-CDR2 comprises the amino acid sequence of SEQ ID NO: 4 as shown in Figure 1 or Table V or a variant thereof, wherein the variant comprises an amino acid sequence resulted from a partial alteration of SEQ ID NO: 4,
(c) VH-CDR3 comprises the amino acid sequence of SEQ ID NO: 5 as shown in Figure 1 or Table V or a variant thereof, wherein the variant comprises an amino acid sequence resulted from a partial alteration of SEQ ID NO: 5,
(d) VL-CDR1 comprises the amino acid sequence of SEQ ID NO: 8 as shown in Figure 1 or Table V or a variant thereof, wherein the variant comprises an amino acid sequence resulted from a partial alteration of SEQ ID NO: 8,
(e) VL-CDR2 comprises the amino acid sequence of SEQ ID NO: 9 as shown in Figure 1 or Table V or a variant thereof, wherein the variant comprises an amino acid sequence resulted from a partial alteration of SEQ ID NO: 9, and
(f) VL-CDR3 comprises the amino acid sequence of SEQ ID NO: 10 as shown in Figure 1 or Table V or a variant thereof, wherein the variant comprises an amino acid sequence resulted from a partial alteration of SEQ ID NO: 10,
the variant CDR comprises one or more amino acid substitutions, preferably one, two, three or four, preferably one or two amino acid substitutions, most preferably one amino acid substitution. In a preferred embodiment, the variant CDR is the VL-CDR3 and comprises one or more amino acid substitutions, preferably one, two, three or four, preferably one or two amino acid substitutions, most preferably one amino acid substitution. In a further preferred embodiment, the amino acid substitution is within the VL-CDR3, preferably at position 91 of the VL chain, and the amino acid substitution is most preferably D91E.

In one embodiment, as regards NI-504.3E7, the variant CDR comprises an amino acid sequence which is at least 90%, preferably 91%, preferably 92%, preferably 93%, preferably 94%, preferably 95%, preferably 96%, preferably 97%, preferably 98%, preferably 99% identical to the sequences set forth in SEQ ID NO: 3, SEQ ID NO: 4, SEQ ID NO: 5, SEQ ID NO: 8, SEQ ID NO: 9, and SEQ ID NO: 10, respectively. In a preferred embodiment, the variant CDR comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 10, respectively.

In another preferred embodiment, the variant of the mentioned VH and VL chains, respectively, comprises one or more amino acid substitutions, preferably one, two, three, four, five, six, seven, eight, nine, or ten amino acid substitutions, preferably wherein the variant of the VL chain comprises one, two, three, four, or five, preferably two, four, or five amino acid substitutions, and/or the variant of the VH chain comprises one, two, three, four, five, six, seven, eight, nine, or ten, preferably two, three, four, five six, seven, eight, or ten amino acid substitutions. In one embodiment, the variant of the mentioned VH and VL chains, respectively, comprises one or two amino acid substitutions.

In one embodiment, the variant of the listed VH chains comprises the amino acid substitutions T7S, Q19R, P28T, Y29F, V40S, V40A, I71T, M80T, R82Y, H84Q, and/or Y86S. In particular, in a preferred embodiment, the VH chain variant comprises the amino acid substitutions:
(i) M80T and Y86S,
(ii) V40S, M80T, and Y86S,
(iii) I71T, M80T, and Y86S,
(iv) V40S, I71T, M80T, and Y86S,
(v) V40A, I71T, M80T, and Y86S,
(vi) T7S, Q19R, V40A, I71T, M80T, R82Y, H84Q, and Y86S,
(vii) Q19R, V40A, I71T, M80T, R82Y, H84Q, and Y86S,
(viii) T7S, V40A, I71T, M80T, R82Y, H84Q, and Y86S,
(ix) T7S, Q19R, V40A, I71T, M80T, H84Q, and Y86S,
(x) T7S, Q19R, V40A, I71T, M80T, R82Y, and Y86S,
(xi) V40A, I71T, M80T, R82Y, H84Q, and Y86S,
(xii) Q19R, V40A, I71T, M80T, H84Q, and Y86S,
(xiii) Q19R, V40A, I71T, M80T, R82Y, and Y86S,
(xiv) T7S, V40A, I71T, M80T, H84Q, and Y86S,
(xv) T7S, V40A, I71T, M80T, R82Y, and Y86S,
(xvi) T7S, Q19R, V40A, I71T, M80T, and Y86S,
(xvii) T7S, V40A, I71T, M80T, and Y86S,
(xviii) Q19R, V40A, I71T, M80T, and Y86S,
(ixx) V40A, I71T, M80T, R82Y, and Y86S,
(xx) V40A, I71T, M80T, H84Q, and Y86S,
(xxi) T7S, Q19R, V40S, I71T, M80T, R82Y, H84Q, and Y86S, or
(xxii) T7S, Q19R, P28T, Y29F, V40A, I71T, M80T, R82Y, H84Q, and Y86S.

In one embodiment, the variant of the listed VL chains comprises the amino acid substitutions L37Q, L69T, N75S, L78Q, and/or D91E. In particular, in a preferred embodiment, the VL variant comprises the amino acid substitutions:
(i) L69T and L78Q,
(ii) L37Q, L69T, N75S, and L78Q, or
(iii) L37Q, L69T, N75S, L78Q, and D91E.

As shown in Table VII, the mentioned amino acid substitutions are present in variants of the VH chain of antibody NI-504.3E7 (VH_3E7_var1, VH_3E7_var2, VH_3E7_var3, VH_3E7_var4, VH_3E7 _var5, VH_3E7 _var6, VH_3E7_var6a, VH_3E7 _var6b, VH_3E7 _var6c, VH_3E7_var6d, VH_3E7_var6ab, VH_3E7_var6ac, VH_3E7 _var6ad, VH_3E7 _var6bc, VH_3E7 _var6bd, VH_3E7_var6cd, VH_3E7_var6bcd, VH_3E7 _var6acd, VH_3E7_var6abd, VH_3E7_var6abc, VH_3E7 _var7, VH_3E7_var8) and of variants in the VL chain of antibody NI-504.3E7 (VL_3E7_var1, VL_3E7_var2, VL_3E7_var3). A preferred assignment of variant VH and VL chains is depicted in Table VI.

In one embodiment, the variant of the mentioned VH and VL chains, respectively, comprises one or two amino acid substitutions, preferably any one of the ones listed above. In another preferred embodiment, the variant of the mentioned VH and VL chains, respectively comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 2, SEQ ID NO: 7, SEQ ID NO: 12, and SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, respectively.

As regards antibody NI-504.3E7, the variant of the mentioned VH and VL chains, respectively comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 2 and SEQ ID NO: 7, respectively.

Preferably however, the antibody of the present invention comprises in one or both of its immunoglobulin chains one, two or all three CDRs of the variable regions as set forth in Figure 1 and Table IV and V, or one, two or all three CDRs which are 90%, 91%, 92%, 93%, 94%, 95%, 96%, 97%, 98% or 99% identical to the CDRs of the variable regions as set forth in Figure 1 and Table V. In addition, or alternatively, one or more framework regions (FRs) from the FRs are 80% identical to the corresponding FRs (which are located between the CDRs depicted in Figure 1 and Table IV, preferably 85%, 90%, 95%, 96, 97%, 98%, 99% or 100% identical to the FRs, which is the sequence between the underlined CDRs in Figure 1 and Table IV. In some embodiments, 1, 2, 3, or all 4 FRs (each being at least 90%, 90-95%, and/or 95-99% identical to the FRs shown in Figure 1 and Table IV is/are present.

**Table II: Nucleotide and amino acid sequences of the variable regions (VH, VL) of antibody NI-504.3E7 as well as variant VH and VL chains of the present invention. Underlined, bold nucleotides or amino acids indicate the CDR coding regions in the variable chain sequence.**

| VH/VL chain | Nucleotide and amino acid sequence of the variable heavy (VH) and variable light (VL) chains |
|---|---|
| NI-504.3E7-VH | gaggtgcagctggtggagactgggggaggcttcgtccagccgggggggtccc tacaactctcctgtgccgtctctggattcccctacagtggcacttctttcca ctgggtccgccaggtttccgggaaagggctggagtggattggccgtattaga agtaaaagtgacaattatgaaacatcatatactgagtcactgaagggcaggt tcatcgtctccagagatgattcgaagaatatggtgcgtctgcacttgtacag tctgaggaccgaggacacggccctttattattgtactaccgggattatcgtg gtggatacgtcgtatttggaccactggggccggggaaccctggtcaccgtct cctcg SEQ ID NO: 1 |
| NI-504.3E7-VH | EVQLVETGGGFVQPGGSLQLSCAVSGFPYSGTSFHWVRQVSGKGLEWIGRIR SKSDNYETSYTESLKGRFIVSRDDSKNMVRLHLYSLRTEDTALYYCTTGIIV VDTSYLDHWGRGTLVTVSS SEQ ID NO: 2 |
| NI-504.3E7-VL | tcctatgagctgactcagccaccctcggcgtcagtgtccccaggacaaacgg ccaggatcacctgctctggagctgcattgccaaaaaaatatgcttattggta ccaactgaagtcaggccaggcccctgtgttggtcatctatgaagacactaaa cggccctccgggatccctgagagattctctggctccagctcagggactttgg ccaccctgactatcaatggggccctcgtggaggatgaagctgactactactg ttactcaacagacagcagtggtgatgtttgggtgttcggcggagggaccaag ttgaccgtccta SEQ ID NO: 6 |
| NI-504.3E7-VL | SYELTQPPSASVSPGQTARITCSGAALPKKYAYWYQLKSGQAPVLVIYEDTK RPSGIPERFSGSSSGTLATLTINGALVEDEADYYCYSTDSSGDVWVFGGGTK LTVL SEQ ID NO: 7 |
| VH_3E7_var1 | gaagttcaactggtagagaccggagggggatttgtgcaaccgggggggagct tgcaactgagctgcgcggtatccggctttccatactcggggacctcttttca ttgggtccgacaagtcagcggaaagggactggaatggattgggcgaatccga agcaaaagcgacaactatgagacgagctacaccgagagcttgaagggacgat tcatcgtgagcagggacgattctaagaatacggtaaggctgcacctaagcag cctgcgaaccgaagacaccgcgctatattactgcacgacggggatcattgtc gtcgacacctcatacttggaccactggggccgagggacgctagtgacggtct cgagc SEQ ID NO: 11 |
| VH_3E7_var1 | EVQLVETGGGFVQPGGSLQLSCAVSGFPYSGTSFHWVRQVSGKGLEWIGRIR SKSDNYETSYTESLKGRFIVSRDDSKNTVRLHLSSLRTEDTALYYCTTGIIV VDTSYLDHWGRGTLVTVSS SEQ ID NO: 12 |
| VH_3E7_var2 | gaagtgcaactggtggagaccggggggggatttgtacaaccgggggggagcc tacagctgagctgcgcggtatcgggattcccatactccggcacgagctttca |
| | ttgggtcaggcaatcgagcgggaaagggctggaatggatcgggaggattagg agcaagagcgacaactatgaaacatcatatacggagagcctgaagggaagat tcatagtgagccgggatgactcaaagaacaccgtgcgactgcatctgagcag cttgcggactgaggatacggctctatactattgcacgacggggatcattgtc gtcgatacgagctacctggatcactgggggaggggaacactcgtgacggtct cgagc SEQ ID NO: 13 |
| VH_3E7_var2 | EVQLVETGGGFVQPGGSLQLSCAVSGFPYSGTSFHWVRQSSGKGLEWIGRIR SKSDNYETSYTESLKGRFIVSRDDSKNTVRLHLSSLRTEDTALYYCTTGIIV VDTSYLDHWGRGTLVTVSS SEQ ID NO: 14 |
| VH_3E7_var3 | gaggtgcaactggtagaaaccggggggggattcgtgcaaccgggggggagct tacaattaagctgcgcggtatcgggcttcccctatagcggaacatcattcca ttgggtccggcaagtctctgggaaggggctggaatggattggacggattcgg agcaagagcgacaattacgagacatcatataccgaatcattaaaagggcgat ttaccgtatcaagagacgattcgaaaaatacggtgcggctacacctatcatc gctgagaacggaggataccgcactatactattgcacaacggggataattgtc gtcgacacaagctacttggatcattgggggcgagggacacttgtgacggtct cgagc SEQ ID NO: 15 |
| VH_3E7_var3 | EVQLVETGGGFVQPGGSLQLSCAVSGFPYSGTSFHWVRQVSGKGLEWIGRIR SKSDNYETSYTESLKGRFTVSRDDSKNTVRLHLSSLRTEDTALYYCTTGIIV VDTSYLDHWGRGTLVTVSS SEQ ID NO: 16 |
| VH_3E7_var4 | gaggtgcaactggtggagaccgggggaggatttgtgcaacccggcgggagcc tccaactgagctgcgcggtctccggcttcccatactctgggacgagcttcca ctgggtccggcaatcgagcgggaaaggcctggaatggatcggccggattaga tcgaagagcgacaactatgaaacgagctatacggaatcactgaagggacgat tcacggtatcgcgggatgactctaaaaataccgtgaggctacacctatcatc cttgcggaccgaggacaccgcgctatattactgcacgacggggataattgtc gtcgatacgagctacttagaccattgggggagaggcaccttggtaacggtct cgagc SEQ ID NO: 17 |
| VH_3E7_var4 | EVQLVETGGGFVQPGGSLQLSCAVSGFPYSGTSFHWVRQSSGKGLEWIGRIR SKSDNYETSYTESLKGRFTVSRDDSKNTVRLHLSSLRTEDTALYYCTTGIIV VDTSYLDHWGRGTLVTVSS SEQ ID NO: 18 |
| VH_3E7_var5 | gaagtccaactggtcgagactggggggggctttgtgcaaccggggggatcgc tgcaactatcatgcgccgtcagcggattcccatactctggaacatcatttca ttgggtccgacaagcgagcggaaaggggctggaatggatcggacgcatacga tctaagagcgacaattacgaaacatcttatacggaatcgctcaaagggcgat ttactgtgagccgggacgactcgaagaacacggtcaggctgcaccttagctc actgagaacggaagatacggcgctatactactgcactactgggatcatagtc gtcgatacgagctatctggaccactgggggaggggcacgctggtcacggtct cgagc SEQ ID NO: 19 |
| VH_3E7_var5 | EVQLVETGGGFVQPGGSLQLSCAVSGFPYSGTSFHWVRQASGKGLEWIGRIR SKSDNYETSYTESLKGRFTVSRDDSKNTVRLHLSSLRTEDTALYYCTTGIIV VDTSYLDHWGRGTLVTVSS SEQ ID NO: 20 |
| VH_3E7_var6 | gaggtgcaactggtggaatcggggggcggattcgtgcaaccgggcgggagct tgcgactatcatgcgcggttagcggatttccatatagcgggacatcatttca ctgggtccggcaagcaagcggaaaagggctggaatggattggccgcattcgg agcaaatccgacaactatgagacgagctatacggaatcattgaaaggaagat tcacggtatcgagggatgactctaaaaacactgtatacctgcaactgagcag cttgcgaacggaagacaccgcgctatactactgcacgacggggatcatagtc |
| | gtcgatacctcatacctggaccactggggccggggaacactggtcacggtct cgagc SEQ ID NO: 21 |
| VH_3E7_var6 | EVQLVESGGGFVQPGGSLRLSCAVSGFPYSGTSFHWVRQASGKGLEWIGRIR SKSDNYETSYTESLKGRFTVSRDDSKNTVYLQLSSLRTEDTALYYCTTGIIV VDTSYLDHWGRGTLVTVSS SEQ ID NO: 22 |
| VH_3E7_var6a | gaggtgcaactggtggagaccggagggggatttgtgcaacctggaggatcac tgcggctaagctgcgcggtatccggcttcccttatagcggaacatccttcca ctgggtccggcaagcatccggcaagggcttggaatggatcgggcggattaga tcgaagagcgacaattacgaaacatcttataccgagagcctgaaggggagat ttacggtaagcagggacgactcgaaaaatactgtatacctgcaactgagcag cctgaggaccgaagacacggccttatactactgcacaaccggcattatcgtc gtcgacactagctatctggatcattgggggcggggaacactcgtgacggtct cgagc SEQ ID NO: 23 |
| VH_3E7_var6a | EVQLVETGGGFVQPGGSLRLSCAVSGFPYSGTSFHWVRQASGKGLEWIGRIR SKSDNYETSYTESLKGRFTVSRDDSKNTVYLQLSSLRTEDTALYYCTTGIIV VDTSYLDHWGRGTLVTVSS SEQ ID NO: 24 |
| VH_3E7_var6b | gaagtgcaattagtggagagcgggggcggattcgtgcaaccgggggggagcc ttcaactgagctgcgctgtgagcggatttccttatagcggaacatcatttca ttgggtccgacaagcctccgggaagggactggaatggatcgggaggattagg agcaaatccgacaattacgaaacaagctacactgaatccctgaagggccgat ttaccgtgagccgagacgattcaaagaatacggtatacctgcaactatcatc actgcggactgaggataccgcgctatactactgcacaacggggataatagtc gtcgacacatcatacctcgaccattgggggagggggacgctggtcacggtct cgagc SEQ ID NO: 25 |
| VH_3E7_var6b | EVQLVESGGGFVQPGGSLQLSCAVSGFPYSGTSFHWVRQASGKGLEWIGRIR SKSDNYETSYTESLKGRFTVSRDDSKNTVYLQLSSLRTEDTALYYCTTGIIV VDTSYLDHWGRGTLVTVSS SEQ ID NO: 26 |
| VH_3E7_var6c | gaggtccaactggttgaaagcggagggggattcgtgcaacccggaggaagcc tgagattgagctgcgccgtatcgggattcccatactcgggaacatcctttca ctgggtccggcaagcaagcgggaagggccttgaatggattgggcgcattcga tcgaaatcggataactatgaaacgagctataccgaatcgctgaaggggcgat tcacggttagcagagatgactccaaaaacacggtgcggctgcaactatcatc gctgcggaccgaggacaccgcgctatactattgcacgacggggataatcgtc gtcgacacgagctacctggaccactgggggaggggcacgctggtaacggtct cgagc SEQ ID NO: 27 |
| VH_3E7_var6c | EVQLVESGGGFVQPGGSLRLSCAVSGFPYSGTSFHWVRQASGKGLEWIGRIR SKSDNYETSYTESLKGRFTVSRDDSKNTVRLQLSSLRTEDTALYYCTTGIIV VDTSYLDHWGRGTLVTVSS SEQ ID NO: 28 |
| VH_3E7_var6d | gaggtccagctggtagagagcgggggcggcttcgtgcaaccgggaggatcgc tgaggctgagctgcgccgtatcgggctttccttactccggcacttcattcca ctgggtccggcaagcatctgggaaagggcttgaatggatcgggcggatacgg agcaaatccgacaattatgagacttcatatacggaatccctgaaggggagat ttacggtatcgagggatgattcaaagaataccgtatatctgcacctgagctc gctgcgcacggaagacaccgcattatactactgcaccactggaatcattgtc gtcgatacaagctacttggatcattgggggagggggacgctggtaacggtct cgagc SEQ ID NO: 29 |
| VH_3E7_var6d | EVQLVESGGGFVQPGGSLRLSCAVSGFPYSGTSFHWVRQASGKGLEWIGRIR SKSDNYETSYTESLKGRFTVSRDDSKNTVYLHLSSLRTEDTALYYCTTGIIV VDTSYLDHWGRGTLVTVSS SEQ ID NO:30 |
| VH_3E7_var6ab | gaggtgcaactggtcgagacgggaggaggatttgtgcaaccgggggggagcc ttcaactatcatgcgctgtgagcggattcccctattccgggacgagctttca ctgggtcagacaagcgagcggaaaagggctggaatggatcgggaggattcga agcaagagcgacaactatgagacatcatacactgaatcactgaaggggagat tcaccgtatctcgcgacgactcgaagaacacggtatacctgcaattaagctc gctgcggacggaggacacggcgctatactattgcacgacgggaattatagtc gtcgacacatcatatcttgatcattgggggcggggaaccctggtaacggtct cgagc SEQ ID NO: 31 |
| VH_3E7_var6ab | EVQLVETGGGFVQPGGSLQLSCAVSGFPYSGTSFHWVRQASGKGLEWIGRIR SKSDNYETSYTESLKGRFTVSRDDSKNTVYLQLSSLRTEDTALYYCTTGIIV VDTSYLDHWGRGTLVTVSS SEQ ID NO: 32 |
| VH_3E7_var6ac | gaggtgcagctggttgagactggcggaggatttgtgcaaccgggggggagcc tgcggctatcatgcgcggtatcgggattcccatactctgggacaagtttcca ctgggtccgacaggcttcgggaaaggggctggaatggataggaaggataaga tctaagagcgataactatgagacatcatacacggaatcgctgaaagggcgat tcaccgtatctcgggacgatagcaagaacaccgtgagactgcaactatcaag cctacgaacggaggatacggcgctatactactgcacaaccgggataatcgtc gtcgacacatcatatttggatcattggggccgggggacgctggtaacggtct cgagc SEQ ID NO: 33 |
| VH_3E7_var6ac | EVQLVETGGGFVQPGGSLRLSCAVSGFPYSGTSFHWVRQASGKGLEWIGRIR SKSDNYETSYTESLKGRFTVSRDDSKNTVRLQLSSLRTEDTALYYCTTGIIV VDTSYLDHWGRGTLVTVSS SEQ ID NO: 34 |
| VH_3E7_var6ad | gaggtgcaactcgtggagaccggcgggggatttgtacaaccggggggatcgc tgcggctatcatgcgccgtgagcggattcccctactcggggacatcatttca ctgggtccgacaagcctccgggaaggggctggaatggattgggcggatacga tcgaagagcgacaattacgagacatcatacactgaatccctgaaagggcgat ttaccgtgagccgagacgactccaagaatacggtatacctgcatttgagctc gctaaggacggaggatacggcattatactactgcacaactggaataatagtc gtcgacacgagctacttagaccactgggggaggggaacgctggtaacggtct cgagc SEQ ID NO: 35 |
| VH_3E7_var6ad | EVQLVETGGGFVQPGGSLRLSCAVSGFPYSGTSFHWVRQASGKGLEWIGRIR SKSDNYETSYTESLKGRFTVSRDDSKNTVYLHLSSLRTEDTALYYCTTGIIV VDTSYLDHWGRGTLVTVSS SEQ ID NO: 36 |
| VH_3E7_var6bc | gaggtgcaactggtggaatctggagggggcttcgtgcaacccggaggatcgc tacaacttagctgcgccgtgagcggatttccatactcgggcacatcattcca ctgggtccggcaagcgagcgggaagggactggaatggatagggcggataaga tcgaaatcggacaattatgagacgagctacaccgaatccctaaagggaagat tcaccgtatcgagagacgattcaaagaacaccgtaagactgcaactaagctc gctgagaactgaagacactgcactatactactgcacaacggggataatagtc gtcgacacatcatatttggaccactgggggaggggaactttggtaacggtct cgagc SEQ ID NO: 37 |
| VH_3E7_var6bc | EVQLVESGGGFVQPGGSLQLSCAVSGFPYSGTSFHWVRQASGKGLEWIGRIR SKSDNYETSYTESLKGRFTVSRDDSKNTVRLQLSSLRTEDTALYYCTTGIIV VDTSYLDHWGRGTLVTVSS SEQ ID NO: 38 |
| VH_3E7_var6bd | gaggtgcagctggtggagagcgggggcggattcgtgcaacccggaggatcgc tacaactgagctgcgccgtgagcggatttccttattcgggcacgagctttca ctgggtccgacaagcgagcggaaaaggcctggaatggattgggaggatacgg agcaaatccgacaattatgagacatcatacacggagagcctgaaggggagat ttaccgtgagcagggacgattcgaagaatacggtatacctgcacctgagctc tctgcggaccgaggacacggcgctatattattgcactacggggataatcgtc gtcgacacatcatacctggaccactgggggagggggacgctagtgacggtct cgagc SEQ ID NO: 39 |
| VH_3E7_var6bd | EVQLVESGGGFVQPGGSLQLSCAVSGFPYSGTSFHWVRQASGKGLEWIGRIR SKSDNYETSYTESLKGRFTVSRDDSKNTVYLHLSSLRTEDTALYYCTTGIIV VDTSYLDHWGRGTLVTVSS SEQ ID NO: 40 |
| VH_3E7_var6cd | gaggtacagctggtggaatcgggcgggggatttgtgcaaccggggggctcgc tgcgcctgagctgcgcggtatccggcttcccttactctggcacatccttcca ttgggtccgccaagcatctgggaaaggactggaatggattgggagaatccga tcgaaatccgataactatgagacgagctataccgaatcattgaaggggcgat tcaccgtgagcagggatgactctaaaaacaccgtgaggctgcatctatcgag cctacggaccgaggatacggctctatattactgcacgaccgggatcatagtc gtcgatacctcatacttggatcactgggggcggggaacattggtcacggtct cgagc SEQ ID NO: 41 |
| VH_3E7_var6cd | EVQLVESGGGFVQPGGSLRLSCAVSGFPYSGTSFHWVRQASGKGLEWIGRIR SKSDNYETSYTESLKGRFTVSRDDSKNTVRLHLSSLRTEDTALYYCTTGIIV VDTSYLDHWGRGTLVTVSS SEQ ID NO: 42 |
| VH_3E7_var6bcd | gaggtgcaactcgtggaatcgggggggggatttgtgcaaccgggcggatcgc tgcaactatcatgcgccgtgagcggatttccctactctggaacatcatttca ctgggtccgacaagcgagcggaaagggattggaatggattgggcggataaga tcgaagagcgataactacgagacatcatataccgaatcactgaaggggcgat ttaccgtgagccgagacgactcgaagaatactgttcggctgcacctgagctc gctaaggacggaggacaccgcgctatattactgcacgaccggcataatagtc gtcgacacaagctacttggatcactgggggaggggcacgctggtaacggtct cgagc SEQ ID NO: 43 |
| VH_3E7_var6bcd | EVQLVESGGGFVQPGGSLQLSCAVSGFPYSGTSFHWVRQASGKGLEWIGRIR SKSDNYETSYTESLKGRFTVSRDDSKNTVRLHLSSLRTEDTALYYCTTGIIV VDTSYLDHWGRGTLVTVSS SEQ ID NO: 44 |
| VH_3E7_var6acd | gaggtgcagctggtggagacgggggggggattcgtgcaaccggggggctcgc tgcggctatcatgcgcggtatcgggatttccatatagcgggacatcctttca ttgggtccgacaagcatctggaaaggggctggaatggattgggaggattaga tcaaagagcgataattacgaaacttcatacacggaatcgctgaaagggagat tcaccgtgagccgagacgactcaaagaacactgtgcgcctgcatctatcatc gctgagaaccgaggatacggcgctatattattgcaccaccgggatcattgtc gtcgacacttcatacttggaccactgggggcggggaacgcttgtgacggtct cgagc SEQ ID NO: 45 |
| VH_3E7_var6acd | EVQLVETGGGFVQPGGSLRLSCAVSGFPYSGTSFHWVRQASGKGLEWIGRIR SKSDNYETSYTESLKGRFTVSRDDSKNTVRLHLSSLRTEDTALYYCTTGIIV VDTSYLDHWGRGTLVTVSS SEQ ID NO: 46 |
| VH_3E7_var6abd | gaagtacagctggtggagacggggggaggatttgtgcaaccgggagggagcc tgcaactatcatgcgccgtatccggcttcccatactcggggacatcattcca ttgggtcaggcaagcaagcggcaaagggctggaatggattgggcggattaga tcaaaatccgacaattacgagacgagctacaccgaaagcctgaagggccgat |
| | ttaccgtatcgcgggacgattcgaagaacacggtatacctgcaccttagctc actgaggacggaggacacggccttatactattgcacgaccgggattatcgtc gtcgacacatcatacttggatcattgggggcgaggcacactcgttaccgtct cgagc SEQ ID NO: 47 |
| VH_3E7_var6abd | EVQLVETGGGFVQPGGSLQLSCAVSGFPYSGTSFHWVRQASGKGLEWIGRIR SKSDNYETSYTESLKGRFTVSRDDSKNTVYLHLSSLRTEDTALYYCTTGIIV VDTSYLDHWGRGTLVTVSS SEQ ID NO: 48 |
| VH_3E7_var6abc | gaagtccaacttgtggaaactggggggggatttgtgcaaccgggagggagcc tacaactgagctgcgcggtctccggcttcccctactcggggacatcctttca ctgggtccgacaagcatccgggaaggggctggaatggattggacgcatacgg agcaaatctgacaattacgagacatcatacactgaatcgctaaagggccgat ttacggtctcgcgggacgattcgaagaataccgtccggctgcaactatcgag cctgcggaccgaggataccgcgctatattattgcaccacgggaattattgtc gtcgatactagctatctggatcactgggggagggggacgctggtcacggtct cgagc SEQ ID NO: 49 |
| VH_3E7_var6abc | EVQLVETGGGFVQPGGSLQLSCAVSGFPYSGTSFHWVRQASGKGLEWIGRIR SKSDNYETSYTESLKGRFTVSRDDSKNTVRLQLSSLRTEDTALYYCTTGIIV VDTSYLDHWGRGTLVTVSS SEQ ID NO: 50 |
| VH_3E7_var7 | gaggtgcagctggtcgaatctggagggggatttgtgcaacctggcggaagtt tgcggctgagctgcgccgtgagcggattcccatattcggggacgagctttca ctgggtccggcaatcgagcgggaaggggcttgaatggataggacggattcgg agcaaatccgacaactacgaaacatcatataccgagagcctgaaggggagat tcacggtatcgagagatgattcgaagaacactgtatatctgcaactatcatc actgagaacggaggacacggcgctatattactgcacaaccggaattatagtc gtcgacacatcatacctcgaccactgggggcgaggcacgctggttacggtct cgagc SEQ ID NO: 51 |
| VH_3E7_var7 | EVQLVESGGGFVQPGGSLRLSCAVSGFPYSGTSFHWVRQSSGKGLEWIGRIR SKSDNYETSYTESLKGRFTVSRDDSKNTVYLQLSSLRTEDTALYYCTTGIIV VDTSYLDHWGRGTLVTVSS SEQ ID NO: 52 |
| VH_3E7_var8 | gaggtgcaactggtggaatccggcgggggattcgtgcaaccggggggatcgc tgcgactatcatgcgccgtaagcggatttacatttagcgggacatcatttca ctgggtccggcaagcaagcggaaaggggctggaatggattggacggattagg agcaaatcggataactatgagacaagctatacggaatcgctgaaagggagat ttaccgtatcgcgcgatgactcgaagaacaccgtatacctgcaactatcgag cttgcggaccgaggacaccgcactatactattgcacaactggaataatcgtc gtcgacacatcatacctagaccactggggaaggggcacactggtcacggtct cgagc SEQ ID NO: 53 |
| VH_3E7_var8 | EVQLVESGGGFVQPGGSLRLSCAVSGFTFSGTSFHWVRQASGKGLEWIGRIR SKSDNYETSYTESLKGRFTVSRDDSKNTVYLQLSSLRTEDTALYYCTTGIIV VDTSYLDHWGRGTLVTVSS SEQ ID NO: 54 |
| VL_3E7_var1 | tcctatgaactgacacagcctccttccgcaagcgtatctcctgggcagaccg cacggattacatgcagtggggccgcgttgcctaaaaagtacgcctactggta tcaactgaaatcagggcaagccccagtcttagtgatttacgaggacactaag cggcctagtggcatacccgaaagattcagtggcagcagctctgggactactg caactttgacaatcaatggtgcccaagtggaggatgaggctgactactattg ttatagcactgactccagtggcgacgtctgggtgtttggaggcggtaccaag ctgacagttctg SEQ ID NO: 55 |
| VL_3E7_var1 | SYELTQPPSASVSPGQTARITCSGAALPKKYAYWYQLKSGQAPVLVIYEDTK RPSGIPERFSGSSSGTTATLTINGAQVEDEADYYCYSTDSSGDVWVFGGGTK LTVL SEQ ID NO: 56 |
| VL_3E7_var2 | tcgtatgagctaacccagccacccagtgcgagcgtgtcgccaggccagaccg ccagaattacctgtagcggtgctgcactacccaaaaagtacgcctattggta tcaacagaaaagcggacaggccccagtcctagttatctacgaagatactaaa cgacccagcggaatccccgagcggttcagtggctcatcgtcaggaactacag ctactttgacaatttctggagctcaggtcgaggacgaagctgattactactg ttactccactgacagcagcggcgacgtgtgggtgttcggaggcggtaccaag ctgaccgttctg SEQ ID NO:57 |
| VL_3E7_var2 | SYELTQPPSASVSPGQTARITCSGAALPKKYAYWYQQKSGQAPVLVIYEDTK RPSGIPERFSGSSSGTTATLTISGAQVEDEADYYCYSTDSSGDVWVFGGGTK LTVL SEQ ID NO: 58 |
| VL_3E7_var3 | agctacgaactgacccaaccaccatccgcatctgttagcccaggtcaaactg cgcggatcacttgcagtggggctgcgcttccaaagaagtatgcctactggta tcaacaaaagtccggtcaagcaccagttttggttatttatgaggatacaaaa aggccatcagggataccagagagattttcagggtcttctagcgggactacgg cgacacttacaatttccggcgctcaagtcgaggacgaggccgattactattg ttattcgacggaatcatctggcgacgtctgggtcttcggaggcggtaccaag ttgactgtttta SEQ ID NO: 59 |
| VL_3E7_var3 | SYELTQPPSASVSPGQTARITCSGAALPKKYAYWYQQKSGQAPVLVIYEDTK RPSGIPERFSGSSSGTTATLTISGAQVEDEADYYCYSTESSGDVWVFGGGTK LTVL SEQ ID NO: 60 |

**Table III: Amino acid sequences of the three CDRs of the VH and VL chain of antibody NI-504.3E7 as well as of the variant CDR of the variant VL chain VL_3E7_var3 of the present invention.**

| CDR | Amino acid sequence of CDR1-3 of the variable heavy (VH) and variable light (VL) chains of antibody NI-504.3E7 as well as variants thereof | |
|---|---|---|
| NI-504.3E7-VH CDR1 | GTSFH | SEQ ID NO: 3 |
| NI-504.3E7-VH CDR2 | RIRSKSDNYETSYTESLKG | SEQ ID NO: 4 |
| NI-504.3E7-VH CDR3 | GIIWDTSYLDH | SEQ ID NO: 5 |
| NI-504.3E7-VL CDR1 | SGAALPKKYAY | SEQ ID NO: 8 |
| NI-504.3E7-VL CDR2 | EDTKRPS | SEQ ID NO: 9 |
| NI-504.3E7-VL CDR3 | YSTDSSGDVWV | SEQ ID NO: 10 |
| VL_3E7_var3 CDR3 | YSTESSGDVWV | SEQ ID NO: 61 |

**Table IV: Combination of VH and VL chains of the antibodies of the present invention, i.e., NI-504.3E7 and variants thereof (NI-504.3E7_V1 to NI-504.3E7_V40)**

| **Variant Name** | **Light Chain** | **Heavy Chain** |
|---|---|---|
| NI-504.3E7_O | VL_3E7 | VH_3E7 |
| NI-504.3E7_V1 | VL_3E7_var1 | VH_3E7 |
| NI-504.3E7_V2 | VL_3E7_var2 | VH_3E7 |
| NI-504.3E7_V3 | VL_3E7 | VH_3E7_var1 |
| NI-504.3E7_V4 | VL_3E7_var1 | VH_3E7_var1 |
| NI-504.3E7_V5 | VL_3E7_var2 | VH_3E7_var1 |
| NI-504.3E7_V6 | VL_3E7 | VH_3E7_var2 |
| NI-504.3E7_V7 | VL_3E7_var1 | VH_3E7_var2 |
| NI-504.3E7_V8 | VL_3E7_var2 | VH_3E7_var2 |
| NI-504.3E7_V9 | VL_3E7 | VH_3E7_var3 |
| NI-504.3E7_V10 | VL_3E7_var1 | VH_3E7_var3 |
| NI-504.3E7_V11 | VL_3E7_var2 | VH_3E7_var3 |
| NI-504.3E7_V12 | VL_3E7 | VH_3E7_var4 |
| NI-504.3E7_V13 | VL_3E7_var1 | VH_3E7_var4 |
| NI-504.3E7_V14 | VL_3E7_var2 | VH_3E7_var4 |
| NI-504.3E7_V15 | VL_3E7_var3 | VH_3E7_var4 |
| NI-504.3E7_V16 | VL_3E7 | VH_3E7_var5 |
| NI-504.3E7_V17 | VL_3E7_var1 | VH_3E7_var5 |
| NI-504.3E7_V18 | VL_3E7_var2 | VH_3E7_var5 |
| NI-504.3E7_V19 | VL_3E7_var3 | VH_3E7_var5 |
| NI-504.3E7_V20 | VL_3E7_var2 | VH_3E7_var6 |
| NI-504.3E7_V21 | VL_3E7_var2 | VH_3E7_var6a |
| NI-504.3E7_V22 | VL_3E7_var2 | VH_3E7_var6b |
| NI-504.3E7_V23 | VL_3E7_var2 | VH_3E7_var6c |
| NI-504.3E7_V24 | VL_3E7_var2 | VH_3E7_var6d |
| NI-504.3E7_V25 | VL_3E7_var2 | VH_3E7_var6ab |
| NI-504.3E7_V26 | VL_3E7_var2 | VH_3E7_var6ac |
| NI-504.3E7_V27 | VL_3E7_var2 | VH_3E7_var6ad |
| NI-504.3E7_V28 | VL_3E7_var2 | VH_3E7_var6bc |
| NI-504.3E7_V29 | VL_3E7_var2 | VH_3E7_var6bd |
| NI-504.3E7_V30 | VL_3E7_var2 | VH_3E7_var6cd |
| NI-504.3E7_V31 | VL_3E7_var2 | VH_3E7_var6bcd |
| NI-504.3E7_V32 | VL_3E7_var2 | VH_3E7_var6acd |
| NI-504.3E7_V33 | VL_3E7_var2 | VH_3E7_var6abd |
| NI-504.3E7_V34 | VL_3E7_var2 | VH_3E7_var6abc |
| NI-504.3E7_V35 | VL_3E7_var1 | VH_3E7_var6 |
| NI-504.3E7_V36 | VL_3E7_var3 | VH_3E7_var6 |
| NI-504.3E7_V37 | VL_3E7_var1 | VH_3E7_var7 |
| NI-504.3E7_V38 | VL_3E7_var2 | VH_3E7_var7 |
| NI-504.3E7_V39 | VL_3E7_var3 | VH_3E7_var7 |
| NI-504.3E7_V40 | VL_3E7_var2 | VH_3E7_var8 |

**Table V: Overview of substitutions in the engineered variant VH and VL chains relativ to the sequence of original NI504.3E7 (VL_3E7 and VH_3E7).**

| Chain | Chain name | Position that differs between engineered chains | | | | | | | | | |
|---|---|---|---|---|---|---|---|---|---|---|---|
| L | VL_3E7 | L37 | L69 | N75 | L78 | D91 | | | | | |
| L | VL_3E7_var1 | L | T | N | Q | D | | | | | |
| L | VL_3E7_var2 | Q | T | S | Q | D | | | | | |
| L | VL_3E7 var3 | Q | T | S | Q | E | | | | | |
| H | VH_3E7 | T7 | Q19 | P28 | Y29 | V40 | I71 | M80 | R82 | H84 | Y86 |
| H | VH_3E7_var1 | T | Q | P | Y | V | I | T | R | H | S |
| H | VH_3E7_var2 | T | Q | P | Y | S | I | T | R | H | S |
| H | VH_3E7_var3 | T | Q | P | Y | V | T | T | R | H | S |
| H | VH_3E7_var4 | T | Q | P | Y | S | T | T | R | H | S |
| H | VH_3E7_var5 | T | Q | P | Y | A | T | T | R | H | S |
| H | VH_3E7_var6 | S | R | P | Y | A | T | T | Y | Q | S |
| H | VH_3E7_var6a | T | R | P | Y | A | T | T | Y | Q | S |
| H | VH_3E7_var6b | S | Q | P | Y | A | T | T | Y | Q | S |
| H | VH_3E7_var6c | S | R | P | Y | A | T | T | R | Q | S |
| H | VH_3E7_var6d | S | R | P | Y | A | T | T | Y | H | S |
| H | VH_3E7_var6ab | T | Q | P | Y | A | T | T | Y | Q | S |
| H | VH_3E7_var6ac | T | R | P | Y | A | T | T | R | Q | S |
| H | VH_3E7_var6ad | T | R | P | Y | A | T | T | Y | H | S |
| H | VH_3E7_var6bc | S | Q | P | Y | A | T | T | R | Q | S |
| H | VH_3E7_var6bd | S | Q | P | Y | A | T | T | Y | H | S |
| H | VH_3E7_var6cd | S | R | P | Y | A | T | T | R | H | S |
| H | VH_3E7_var6bcd | S | Q | P | Y | A | T | T | R | H | S |
| H | VH_3E7_var6acd | T | R | P | Y | A | T | T | R | H | S |
| H | VH_3E7_var6abd | T | Q | P | Y | A | T | T | Y | H | S |
| H | VH_3E7_var6abc | T | Q | P | Y | A | T | T | R | Q | S |
| H | VH_3E7_var7 | S | R | P | Y | S | T | T | Y | Q | S |
| H | VH_3E7_var8 | S | R | T | F | A | T | T | Y | Q | S |

In a further additional or alternative embodiment of the present invention the anti-CRMP2 antibody, antigen-binding fragment, synthetic or biotechnological variant thereof can be optimized to have appropriate binding affinity to the target and stability properties. Therefore, at least one amino acid in the CDR or variable region, which is prone to modifications selected from the group consisting of glycosylation, oxidation, deamination, peptide bond cleavage, iso-aspartate formation and/or unpaired cysteine is substituted by a mutated amino acid that lacks such alteration or wherein at least one carbohydrate moiety is deleted or added chemically or enzymatically to the antibody, see, *e.g.* Liu et al., J. Pharm. Sci. 97 (2008), 2426-2447; Beck et al., Nat. Rev. Immunol. 10 (2010), 345-352; Haberger et al., MAbs. 6 (2014), 327-339.

An immunoglobulin or its encoding cDNA may be further modified. Thus, in a further embodiment, the method of the present invention comprises any one of the step(s) of producing a chimeric antibody, murinized antibody, single-chain antibody, Fab-fragment, bi-specific antibody, fusion antibody, labeled antibody or an analog of any one of those. Corresponding methods are known to the person skilled in the art and are described, *e.g.,* in Harlow and Lane "Antibodies, A Laboratory Manual", CSH Press, Cold Spring Harbor (1988) First edition; Second edition by Edward A. Greenfield, Dana-Farber Cancer Institute © 2014, ISBN 978-1-936113-81-1. For example, Fab and F(ab')2 fragments may be produced recombinantly or by proteolytic cleavage of immunoglobulin molecules, using enzymes such as papain (to produce Fab fragments) or pepsin (to produce F(ab')2 fragments). F(ab')2 fragments contain the variable region, the light chain constant region (CL) and the CH1 domain of the heavy chain. Such fragments are sufficient for use, for example, in immunodiagnostic procedures involving coupling the immunospecific portions of immunoglobulins to detecting reagents such as radioisotopes.

In one embodiment, the antibody of the present invention may thus be provided in a format selected from the group consisting of a single chain Fv fragment (scFv), an F(ab') fragment, an F(ab) fragment, and an F(ab')₂ fragment, an Fd, an Fv, a single-chain antibody, a disulfide-stabilized Fv (dsFv), and a nanobody and/or which is a chimeric murine-human or a murinized antibody.

As mentioned above, CRMP2 has been shown to be co-localized with hyperphosphorylated Tau and thus, is present in the corresponding intracellular neurofibrillary tangles. Thus, in one embodiment, it may be beneficial to use recombinant Fab (rFab) and scFvs of the anti-Tau antibody, which might more readily penetrate a cell membrane.

Immunotherapy approaches using different antibody formats such as scFv, single-domain antibody fragments (VHHs or sdAbs), bispecific antibodies, intrabodies and nanobodies have shown therapeutic efficacy in several animal models of Alzheimer's disease (AD), Parkinson disease (PD), dementia with Lewy bodies (DLB), frontotemporal dementia (FTD), Huntington disease (HD), transmissible spongiform encephalopathies (TSEs) and multiple sclerosis (MS). It has been demonstrated that recombinant antibody fragments may neutralize toxic extra- and intracellular misfolded proteins involved in the pathogenesis neurodegenerative diseases and thus represent a promising tool for the development of antibody-based immunotherapeutics for those diseases; see review of Manoutcharian et al., Curr Neuropharmacol 15 (2017), 779-788. The perceived advantages of using small Fab and scFv engineered antibody formats which lack the effector function include more efficient passage across the blood-brain barrier and minimizing the risk of triggering inflammatory side reactions. Furthermore, besides scFv and single-domain antibodies retain the binding specificity of full-length antibodies, they can be expressed as single genes and intracellularly in mammalian cells as intrabodies, with the potential for alteration of the folding, interactions, modifications, or subcellular localization of their targets; *see* for review, *e.g.,* Miller and Messer, Molecular Therapy 12 (2005), 394-401. Furthermore, alternative CRMP2 binding molecules are provided, *e.g.,* designed ankyrin repeat proteins (DARPins); see amyloid-β peptide-specific DARPins described as a novel class of potential therapeutics for Alzheimer disease by Hanenberg et al., J Biol Chem. 289 (2014), 27080-27089. However, as illustrated in the Examples, in accordance with the present invention IgG antibodies are preferably used.

The five primary classes of immunoglobulins are IgG, IgM, IgA, IgD and IgE. These are distinguished by the type of heavy chain found in the molecule. IgG molecules have heavy chains known as gamma-chains; IgMs have mu-chains; IgAs have alpha-chains; IgEs have epsilon-chains; and IgDs have delta-chains; see for review, *e.g.,* Schroeder et al., Structure and function of immunoglobulins. J. Allergy Clin. Immunol. 125 (2010), S41-S52. Furthermore, different subclasses exist, wherein the IgAs are further divided into subclasses IgA1 and IgA2, and wherein IgGs are further divided into subclasses IgG1, IgG2, IgG3, and IgG4. Furthermore, two types of light chain, kappa (κ) and lambda (λ) exist.

In principle, the antibodies of the present invention may be of any kind of class and subclass, respectively, and may comprise any kind of light chain, as long as the binding specificity towards CRMP2, for example as indicated in Table I and illustrated in the appended Examples for the corresponding reference antibody remains unaffected in kind. However, preferably complete IgG antibodies are used, wherein the antibody comprises a constant domain. Accordingly, in one embodiment, the immunoglobulin heavy and/or light chain constant domain present in the antibody of the present invention is of the IgG type, the IgM type, the IgA type, the IgD type or the IgE type, preferably of the IgG type. In one embodiment, the immunoglobulin heavy and/or light chain constant domain present in the antibody of the present invention is of the IgA1, IgA1, IgG1, IgG2, IgG3, or IgG4 subclass, preferably of the IgG1, IgG2, IgG3, or IgG4 subclass and most preferably of the IgG2 subclass. In one embodiment, the light chain is a kappa (κ) or lambda (λ) light chain.

The constant domain may be native, *i.e.*, originally cloned together with the variable domain or heterologous, for example, a murine constant domain in case animal studies are envisaged. In the present case, the original isolated antibody was an IgG2 antibody, wherein the recombinant human-derived antibody of the present invention is preferably of the IgG4 or IgG1 type, preferably of the IgG1 type. Thus, preferably, the constant domain is of human origin with a different IgG subtype, *e.g.,* IgG2 versus IgG4, or IgG2 versus IgG1, or a different allotype and allele, respectively, compared to the constant domain of the antibody as naturally occurred in human. The definition of "allotypes" requires that antibody reagents are available to determine the allotypes serologically. If the determination is only done at the sequence level, the polymorphisms have to be described as "alleles". This does not hinder to establish a correspondence with allotypes if the correspondence allele-allotype has been experimentally proven, or if the individual sequence is identical to a sequence for which it has been demonstrated.

In a preferred embodiment of the present invention, the constant domain is heterologous to at least one of the CDRs and the VH and VL chains, respectively, *e.g.* an immunoglobulin heavy chain constant domain and/or immunoglobulin light chain constant domain, preferably of the IgG type. In addition, or alternatively, the heterologous part of the antibody may be a mammalian secretory signal peptide. Put in other words, in one embodiment the anti-CRMP2 antibody and CRMP2 binding fragment, or derivative thereof of the present invention is a (i) fusion protein comprising a polypeptide sequence which is heterologous to the VH region and/or VL region, or at least one CDR; and/or (ii) a non-natural variant of a polypeptide derived from an immunoglobulin, said non-natural variant comprising a heavy chain constant region that comprises one or more amino acid deletions, substitutions, and/or additions relative to a wild type polypeptide. For example, the human constant domain of the recombinant human-derived antibody of the present invention may be of a different IgG isotype than the constant domain of the parent antibody as naturally produced by the memory B cell or of a different allotype, for example to avoid or reduce immunogenicity which can happen as a result of allo-immunization; see, *e.g.,* for review Jefferis and Lefranc, MAbs 1 (2009), 332-338. Accordingly, in one embodiment, if the parent antibody is of the IgG1 subclass, the human constant domain of the recombinant human-derived antibody of the present invention is of the IgG2, the IgG3, or the IgG4 subclass. In one embodiment, if the parent antibody is of the IgG2 subclass, the human constant domain of the recombinant human-derived antibody of the present invention is of the IgG1, the IgG3, or the IgG4 subclass. In one embodiment, if the parent antibody is of the IgG3 subclass, the human constant domain of the recombinant human-derived antibody of the present invention is of the IgG1, the IgG2, or the IgG4 subclass. In one embodiment, if the parent antibody is of the IgG3 subclass, the human constant domain of the recombinant human-derived antibody of the present invention is of the IgG1, the IgG2, or the IgG4 subclass. In one embodiment, if the parent antibody is of the IgG4 subclass, the human constant domain of the recombinant human-derived antibody of the present invention is of the IgG1, the IgG2, or the IgG3 subclass. In a preferred embodiment, the parent antibody is of the IgG2 subclass, and thus, the human constant domain of the recombinant human-derived antibody of the present invention is of the IgG1, the IgG3, or the IgG4 subclass, preferably of the IgG1 or IgG4 subclass, and most preferably of the IgG1 subclass or isotype.

In an alternative or additional embodiment, if the parent antibody comprises a kappa (κ) light chain, the light chain of the recombinant human-derived antibody of the present invention can be changed to a lambda (λ) light chain or if the parent antibody comprises a lambda (λ) light chain, the light chain of the recombinant human-derived antibody of the present invention can be changed to a kappa (κ) light chain.

There are not only the above-mentioned four subclasses of IgGs but human heavy and light chain genes also exhibit extensive structural polymorphism(s) and, being closely linked, are inherited as a haplotype. Allotypic variants can be immunogenic and provoke antibody responses as a result of allo-immunization. Thus, switching the allotype can be of particular interest to provide non-immunogenic antibody therapeutics. So far, extensive allotypes (polymorphisms) are known, but focus is put on the serologically defined allotypes. Allotypes of IgG proteins are defined by the expression of unique epitope(s) recognized by unique serologic reagent(s). Allotypes expressed on the constant region of IgG heavy chain are designated as Gm (Genetic marker) together with the subclass, e.g., G1m, and the allotype number (or letter), *e.g.,* G1m1 [or G1m(a)], G3m5 [or G3m(b1)]. Human immunoglobulin allotypes are listed in Table 1 of Jefferis and Lefrance, mAbs 1 (2009), 1-7 and in Fig. 1A of Irani et al., Molecular Immunology 67 (2015), 171-182, which content is herein incorporated by reference. Accordingly, in one embodiment, the antibodies of the present invention are of any one of the following allotypes, but not limited thereto: G1m1, G1m2, G1m3, G1m17, G2m23, G3m21, G3m28, G3m11, G3m5, G3m13, G3m14, G3m10, G3m15, G3m16, G3m6, G3m24, G3m26, G3m27, A2m1, A2m2, A2m3, Em1, Km1, Km2, and Km3. In one embodiment, if the parent antibody is of any one of the above-listed allotypes, the recombinant human-derived antibody of the present invention is of any one of the above-mentioned allotypes, but the one of the parent antibody.

The four subclasses, IgG1, IgG2, IgG3, and IgG4, which are highly conserved, differ in their constant region, particularly in their hinges and upper CH2 domains. These regions are involved in binding to both IgG-Fc receptors (FcgR) and C1q. As a result, the different subclasses have different effector functions, both in terms of triggering FcgR-expressing cells, resulting in phagocytosis or antibody-dependent cell-mediated cytotoxicity, and activating complement. The Fc regions also contain a binding epitope for the neonatal Fc receptor (FcRn), responsible for the extended half-life, placental transport, and bidirectional transport of IgG through mucosal surfaces. However, FcRn is also expressed in myeloid cells, where it participates in both phagocytosis and antigen presentation together with classical FcgR and complement. How these properties, IgG-polymorphisms and post-translational modification of the antibodies in the form of glycosylation, affect IgG-function is described in Vidarsson et al., Front. Immunol. 5 (2014), 520. doi:10.3389/fimmu.2014.00520 and de Taeye et al., Antibodies 2019, 8, 30; doi:10.3390/antib8020030. Preferably, the immunoglobulin heavy and/or light chain constant domain present in the antibody of the present invention is of the IgG type.

Accordingly, in certain embodiments of the present invention a specific IgG type is preferred, for example the IgG1 isotype and/or the constant region of the antibody, or antigen-binding fragment, variant, or derivative thereof has been altered so as to provide desired biochemical characteristics. In particular, in one embodiment the Fc portion of the antibody may be mutated to alter, *i.e.* to decrease or increase immune effector function or to increase its half-life using techniques known in the art. Thus, in one embodiment the Fc portion of the antibody is mutated to decrease immune effector function and in another embodiment the Fc portion of the antibody is mutated to increase immune effector function. In another embodiment, the antibody is mutated to increase its half-life.

For example, it may be that constant region modifications consistent with the instant invention moderate complement binding and thus reduce the serum half-life and nonspecific association of a conjugated cytotoxin. Other modifications of the constant region may be used to modify disulfide linkages or oligosaccharide moieties that allow for enhanced tissue antigen interaction due to increased antigen specificity or antibody flexibility. Furthermore, mutations in the Fc region can be made that lead to enhanced antibody dependent cell-mediated cytotoxicity (ADCC) or antibody-dependent cellular phagocytosis (ADCP) via increasing FcyRIIIa binding and/or decreasing FcyRIIIb binding and via increasing FcyRIIa binding and/or FcyRIIIa binding, respectively. For example, the GASDALIE Fc mutant (G236A/S239D/A330L/I332E) exhibits a higher affinity for FcγRIIIa. Another possibility is the enhancement of complement-dependent cytotoxicity (CDC) via increasing C1q binding and/or hexamerization.

In other embodiments, certain antibodies for use in the diagnostic and treatment methods described herein have a constant region, *e.g.,* an IgG heavy chain constant region, which is altered to eliminate glycosylation, referred to elsewhere herein as aglycosylated or "agly" antibodies. Such "agly" antibodies may be prepared enzymatically as well as by engineering the consensus glycosylation site(s) in the constant region. It is believed that "agly" antibodies have a reduced effector function and thus an improved safety and stability profile *in vivo.* Methods of producing aglycosylated antibodies, having desired effector function are found for example in international application WO 2005/018572 A2, which is incorporated by reference in its entirety. A further approach to reduce the effector function of antibodies is the reduction of FcyR and C1q binding by mutations in the Fc region.

A summary is for example given in the review of Wang et al., Protein Cell 9 (2018), 63-73, wherein Table 1 provides examples of modifications to modulate antibody effector function and the half-life of an antibody and which mutations described therein are herein incorporated by reference. The resulting physiological profile, bioavailability and other biochemical effects of the modifications, such as recognizing and binding CRMP2, biodistribution and serum half-life, may easily be measured and quantified using well know immunological techniques without undue experimentation.

As mentioned, in some instances inflammatory responses should be avoided for which reason effector functions of the constant domain of the antibody may be attenuated or eliminated altogether. For example, IgG4 antibodies are potential candidates for immunotherapy when reduced effector functions are desirable. IgG4 Abs are dynamic molecules able to undergo a process known as Fab arm exchange (FAE). This results in functionally monovalent, bispecific antibodies (bsAbs) with unknown specificity and hence, potentially, reduced therapeutic efficacy. However, the stabilizing S228P mutation can prevent IgG4 FAE and thus, restore the therapeutic utility of IgG4 antibodies; see Silva et al., J Biol Chem 290 (2015), 5462-5469. Furthermore, recombinant human IgG antibodies (hIgGs) completely devoid of binding to Fcy receptors (FcyRs) and complement protein C1q, and thus with abolished immune effector functions, are of use for various therapeutic applications. It was found that the combination of Leu234Ala and Leu235Ala (commonly called LALA mutations) or the SPLE mutation eliminated FcyRIIa binding and were shown to eliminate detectable binding to FcyRI, IIa, and IIIa for both IgG1 and IgG4 and that the LALA-PG mutation was an improvement over LALA mutations alone in that they nullified Fc function in mouse and human IgG; for corresponding review see, *e.g.,* Saunders, Front. Immunol. 10 (2019), 1296.doi: 10.3389/fimmu.2019.01296 and Schlothauer et al., Protein Engineering, Design and Selection 29 (2016), 457-466.

Another early approach to reduce effector function is to mutate the glycosylation site at N297 with mutations such as N297A, N297Q, and N297G. The half-life of an antibody can be increased via introducing the following mutations M252Y/S254T/T256E or M428L/N434S; see Wang *et al.* 2018.

As shown in Examples 13 and 14, administration of antibody NI-504-3E7 led to improve long term potentiation (LTP) and cognitive function in aged mice. Same was observed for antibody NI-504.3E7 comprising the LALA-PG mutation. Thus, IgG1 effector functions does not seem to be required or decisive for the observed biological effect of the antibody. This is consistent with the finding that antibody NI-504.3E7 is capable of detecting CRMP2 intracellularly as demonstrated in Example 12 with fully differentiated SH-SY5Y cells, where no effector function is needed. Accordingly, in one embodiment, the antibody of the present invention has a reduced or no Fc function. In one embodiment, the antibody of the present invention is of the IgG4 class or IgG1 class having any of the above-mentioned mutations. In a preferred embodiment, the antibody of the present invention is of the IgG4 class, preferably comprising the S228P mutation. In another preferred embodiment, the antibody of the present invention is of the IgG1 subclass having the LALA mutation, and most preferably the LALA-PG mutation.

The present invention also relates to the antibody or antigen-binding fragment of the present invention which comprises a heterologous polypeptide. For example, an antibody polypeptide of the invention may comprise, consist essentially of, or consist of a fusion protein. Fusion proteins are chimeric molecules which comprise, for example, an immunoglobulin CRMP2-binding domain with at least one target binding site, and at least one heterologous portion, *i.e.* a portion with which it is not naturally linked in nature. The amino acid sequences may normally exist in separate proteins that are brought together in the fusion polypeptide or they may normally exist in the same protein but are placed in a new arrangement in the fusion polypeptide. Fusion proteins may be created, for example, by chemical synthesis, or by creating and translating a polynucleotide in which the peptide regions are encoded in the desired relationship.

The term "heterologous" as applied to a polynucleotide or a polypeptide, means that the polynucleotide or polypeptide is derived from a distinct entity from that of the rest of the entity to which it is being compared. For instance, as used herein, a "heterologous polypeptide" to be fused to an antibody, or an antigen-binding fragment, variant, or analog thereof is derived from a non-immunoglobulin polypeptide of the same species, or an immunoglobulin or non-immunoglobulin polypeptide of a different species.

As mentioned above, chimeric molecules constitute fusion proteins. The antibody of the present invention may be a chimeric rodent-human antibody, preferably a chimeric murine-human antibody, which are particularly useful for diagnostic methods and studies in animals. In one embodiment, the antibody of the present invention is a chimeric murine-human antibody, preferably, wherein the antibody comprises a murine constant region, most preferably a murine IgG2a constant region. The production of chimeric antibodies is described, for example, in international application WO 89/09622.

The present invention also relates to one or more polynucleotide(s) encoding the antibody, antigen-binding fragment or derivative thereof of the present invention or an immunoglobulin VH and VL thereof, preferably wherein the polynucleotide(s) is (are) cDNA.

In a preferred embodiment of the present invention, the polynucleotide comprises, consists essentially of, or consists of a nucleic acid having a polynucleotide sequence encoding the VH or VL chain of an anti-CRMP2 antibody as depicted in Table IV. In this respect, the person skilled in the art will readily appreciate that the polynucleotides encoding the light and/or heavy chain may be encoded by one or more polynucleotides. In one embodiment therefore, the polynucleotide comprises, consists essentially of, or consists of a nucleic acid having a polynucleotide sequence of the VH and the VL chain of an anti-CRMP2 antibody as depicted in Table IV.

In one embodiment of the present invention, the polynucleotide(s) are linked to a heterologous nucleic acid, for example expression control sequences such as a promoter, transcription and/or translation enhancer sequences, internal ribosome binding sites, nucleic acids encoding a peptide leader sequence for recombinant expression in a host and the like.

Accordingly, the present invention relates to a polynucleotide encoding an anti-CRMP2 antibody, in a preferred embodiment a human-derived recombinant anti-CRMP2 antibody or CRMP2 binding fragment, or derivative thereof, wherein the polynucleotide encodes
a VH chain comprising CDRs 1, 2, and 3, and/or a VL chain comprising VL CDRs 1, 2, and 3 as defined by Kabat, wherein
(a) VH-CDR1 comprises the amino acid sequence of SEQ ID NO: 3 as shown in Figure 1 and Table V or a variant thereof, wherein the variant comprises an amino acid sequence resulted from a partial alteration of SEQ ID NO: 3,
(b) VH-CDR2 comprises the amino acid sequence of SEQ ID NO: 4 as shown in Figure 1 and Table V or a variant thereof, wherein the variant comprises an amino acid sequence resulted from a partial alteration of SEQ ID NO: 4,
(c) VH-CDR3 comprises the amino acid sequence of SEQ ID NO: 5 as shown in Figure 1 and Table V or a variant thereof, wherein the variant comprises an amino acid sequence resulted from a partial alteration of SEQ ID NO: 5,
(d) VL-CDR1 comprises the amino acid sequence of SEQ ID NO: 8 as shown in Figure 1 and Table V or a variant thereof, wherein the variant comprises an amino acid sequence resulted from a partial alteration of SEQ ID NO: 8,
(e) VL-CDR2 comprises the amino acid sequence of SEQ ID NO: 9 as shown in Figure 1 and Table V or a variant thereof, wherein the variant comprises an amino acid sequence resulted from a partial alteration of SEQ ID NO: 9, and
(f) VL-CDR3 comprises the amino acid sequence of SEQ ID NO: 10 as shown in Figure 1 and Table V or a variant thereof, wherein the variant comprises an amino acid sequence resulted from a partial alteration of SEQ ID NO: 10.

In a preferred embodiment, the variant CDR comprises one or more amino acid substitutions, preferably one, two, three or four, preferably one or two amino acid substitutions, and most preferably one amino acid substitution. In a preferred embodiment, only the VL-CDR comprises one or more amino acid substitutions, preferably one, two, three or four, preferably one or two amino acid substitutions, most preferably one amino acid substitution. In a further preferred embodiment, the amino acid substitution is within the VL-CDR3, preferably at position 91 of the VL chain, and the amino acid substitution is most preferably D91E. In another preferred embodiment, the variant CDR comprises an amino acid sequence which is at least 90%, preferably 91%, preferably 92%, preferably 93%, preferably 94%, preferably 95%, preferably 96%, preferably 97%, preferably 98%, preferably 99% identical to the sequences set forth in SEQ ID NO: 3, SEQ ID NO: 4, SEQ ID NO: 5, SEQ ID NO: 8, SEQ ID NO: 9, and SEQ ID NO: 10, respectively. In a preferred embodiment, the variant CDR comprises an amino acid sequence which is at least 90%, preferably 91%, preferably 92%, preferably 93%, preferably 94%, preferably 95%, preferably 96%, preferably 97%, preferably 98%, preferably 99% identical to the sequences set forth in SEQ ID NO: 10.

The present invention further relates to a polynucleotide encoding an anti-CRMP2 antibody, in a preferred embodiment a human-derived recombinant anti-CRMP2 antibody or CRMP2 binding fragment, or derivative thereof, wherein the polynucleotide encodes
(i) a VH chain comprising CDRs 1, 2, and 3, and/or a VL chain comprising VL CDRs 1, 2, and 3 as defined by Kabat, wherein
   (a) VH-CDR1 comprises the amino acid sequence of SEQ ID NO: 3 as shown in Figure 1 and Table V or a variant thereof, wherein the variant comprises an amino acid sequence resulted from a partial alteration of SEQ ID NO: 3,
   (b) VH-CDR2 comprises the amino acid sequence of SEQ ID NO: 4 as shown in Figure 1 and Table V or a variant thereof, wherein the variant comprises an amino acid sequence resulted from a partial alteration of SEQ ID NO: 4,
   (c) VH-CDR3 comprises the amino acid sequence of SEQ ID NO: 5 as shown in Figure 1 and Table V or a variant thereof, wherein the variant comprises an amino acid sequence resulted from a partial alteration of SEQ ID NO: 5,
   (d) VL-CDR1 comprises the amino acid sequence of SEQ ID NO: 8 as shown in Figure 1 and Table V or a variant thereof, wherein the variant comprises an amino acid sequence resulted from a partial alteration of SEQ ID NO: 8,
   (e) VL-CDR2 comprises the amino acid sequence of SEQ ID NO: 9 as shown in Figure 1 and Table V or a variant thereof, wherein the variant comprises an amino acid sequence resulted from a partial alteration of SEQ ID NO: 9, and
   (f) VL-CDR3 comprises the amino acid sequence of SEQ ID NO: 61 as shown in Figure 1 and Table V or a variant thereof, wherein the variant comprises an amino acid sequence resulted from a partial alteration of SEQ ID NO: 61.

In a preferred embodiment, the variant CDR comprises one or more amino acid substitutions, preferably one, two, three or four, preferably one or two amino acid substitutions, and most preferably one amino acid substitution. In another preferred embodiment, the variant comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 3, SEQ ID NO: 4, SEQ ID NO: 5, SEQ ID NO: 8, SEQ ID NO: 9, and SEQ ID NO: 61, respectively.

In an additional or alternative embodiment, the present invention relates to a polynucleotide encoding an anti-CRMP2 antibody, in a preferred embodiment a human-derived recombinant anti-CRMP2 antibody or CRMP2 binding fragment, or derivative thereof, wherein the polynucleotide encodes
a VH chain and/or a VL chain, wherein
(a) the VH chain comprises the amino acid sequence depicted in SEQ ID NO: 2 as shown in Figure 1 or Table IV or a variant thereof, wherein the variant comprises an amino acid sequence resulted from a partial alteration of SEQ ID NO: 2; and/or
(b) the VL comprises the amino acid sequence depicted in SEQ ID NO: 7 as shown in Figure 1 or Table IV or a variant thereof, wherein the variant comprises an amino acid sequence resulted from a partial alteration of SEQ ID NO: 7.

In another preferred embodiment, the variant VH and VL, respectively, comprises one or more amino acid substitutions, preferably one, two, three, four, five, six, seven, eight, nine, or ten amino acid substitutions, preferably wherein the variant of the VL chain comprises one, two, three, four, or five, preferably two, four, or five amino acid substitutions, and/or the variant of the VH chain comprises one, two, three, four, five, six, seven, eight, nine, or ten, preferably two, three, four, five six, seven, eight, or ten amino acid substitutions. Most preferably, the variant VH and VL, respectively comprises one or two amino acid substitutions.

In one embodiment, the variant of the listed VH chain comprises the amino acid substitutions T7S, Q19R, P28T, Y29F, V40S, V40A, I71T, M80T, R82Y, H84Q, and/or Y86S. In particular, in a preferred embodiment, the VH chain variant comprises the amino acid substitutions:
(i) M80T and Y86S,
(ii) V40S, M80T, and Y86S,
(iii) I71T, M80T, and Y86S,
(iv) V40S, I71T, M80T, and Y86S,
(v) V40A, I71T, M80T, and Y86S,
(vi) T7S, Q19R, V40A, I71T, M80T, R82Y, H84Q, and Y86S,
(vii) Q19R, V40A, I71T, M80T, R82Y, H84Q, and Y86S,
(viii) T7S, V40A, I71T, M80T, R82Y, H84Q, and Y86S,
(ix) T7S, Q19R, V40A, I71T, M80T, H84Q, and Y86S,
(x) T7S, Q19R, V40A, I71T, M80T, R82Y, and Y86S,
(xi) V40A, I71T, M80T, R82Y, H84Q, and Y86S,
(xii) Q19R, V40A, I71T, M80T, H84Q, and Y86S,
(xiii) Q19R, V40A, I71T, M80T, R82Y, and Y86S,
(xiv) T7S, V40A, I71T, M80T, H84Q, and Y86S,
(xv) T7S, V40A, I71T, M80T, R82Y, and Y86S,
(xvi) T7S, Q19R, V40A, I71T, M80T, and Y86S,
(xvii) T7S, V40A, I71T, M80T, and Y86S,
(xviii) Q19R, V40A, I71T, M80T, and Y86S,
(ixx) V40A, I71T, M80T, R82Y, and Y86S,
(xx) V40A, I71T, M80T, H84Q, and Y86S,
(xxi) T7S, Q19R, V40S, I71T, M80T, R82Y, H84Q, and Y86S, or
(xxii) T7S, Q19R, P28T, Y29F, V40A, I71T, M80T, R82Y, H84Q, and Y86S.

In one embodiment, the variant of the listed VL chains comprises the amino acid substitutions L37Q, L69T, N75S, L78Q, and/or D91E. In particular, in a preferred embodiment, the VL variant comprises the amino acid substitutions:
(i) L69T and L78Q,
(ii) L37Q, L69T, N75S, and L78Q, or
(iii) L37Q, L69T, N75S, L78Q, and D91E.

As shown in Table VII, the mentioned amino acid substitutions are present in variants of the VH chain of antibody NI-504.3E7 (VH_3E7_var1, VH_3E7_var2, VH_3E7_var3, VH_3E7 _var4, VH_3E7 _var5, VH_3E7 _var6, VH_3E7_var6a, VH_3E7_var6b, VH_3E7 _var6c, VH_3E7_var6d, VH_3E7_var6ab, VH_3E7_var6ac, VH_3E7 _var6ad, VH_3E7 _var6bc, VH_3E7 _var6bd, VH_3E7_var6cd, VH_3E7_var6bcd, VH_3E7 _var6acd, VH_3E7 _var6abd, VH_3E7_var6abc, VH_3E7 _var7, VH_3E7_var8) and of variants in the VL chain of antibody NI-504.3E7 (VL_3E7_var1, VL_3E7_var2, VL_3E7_var3). A preferred assignment of variant VH and VL chains is depicted in Table VI.

In another preferred embodiment, the variant of the mentioned VH and VL chains, respectively comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 2 and SEQ ID NO: 7, respectively.

In one embodiment, the present invention relates to a polynucleotide encoding an anti-CRMP2 antibody, in a preferred embodiment a human-derived recombinant anti-CRMP2 antibody or CRMP2 binding fragment, or derivative thereof, wherein the polynucleotide encodes a VH chain and/or a VL chain, wherein
NI-504.3E7_V1
   (i) the VH chain comprises the amino acid sequence depicted in SEQ ID NO: 2 (VH_3E7) as shown in Figure 1 or Table IV or a variant thereof, wherein the variant comprises an amino acid sequence resulted from a partial alteration of SEQ ID NO: 2; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V2
   (i) the VH chain comprises the amino acid sequence depicted in SEQ ID NO: 2 (VH_3E7) as shown in Figure 1 or Table IV or a variant thereof, wherein the variant comprises an amino acid sequence resulted from a partial alteration of SEQ ID NO: 2; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V3
   (i) the VH chain comprises the amino acid sequence depicted in SEQ ID NO: 12 (VH_3E7_var1) as shown in Figure 1 or Table IV or a variant thereof, wherein the variant comprises an amino acid sequence resulted from a partial alteration of SEQ ID NO: 12; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7;
NI-504.3E7_V4
   (i) the VH chain comprises the amino acid sequence depicted in SEQ ID NO: 12 (VH_3E7_var1) as shown in Figure 1 or Table IV or a variant thereof, wherein the variant comprises an amino acid sequence resulted from a partial alteration of SEQ ID NO: 12; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V5
   (i) the VH chain comprises the amino acid sequence depicted in SEQ ID NO: 12 (VH_3E7_var1) as shown in Figure 1 or Table IV or a variant thereof, wherein the variant comprises an amino acid sequence resulted from a partial alteration of SEQ ID NO: 12; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V6
   (i) the VH chain comprises the amino acid sequence depicted in SEQ ID NO: 14 (VH_3E7_var2) as shown in Figure 1 or Table IV or a variant thereof, wherein the variant comprises an amino acid sequence resulted from a partial alteration of SEQ ID NO: 14; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7;
NI-504.3E7_V7
   (i) the VH chain comprises the amino acid sequence depicted in SEQ ID NO: 14 (VH_3E7_var2) as shown in Figure 1 or Table IV or a variant thereof, wherein the variant comprises an amino acid sequence resulted from a partial alteration of SEQ ID NO: 14; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V8
   (i) the VH chain comprises the amino acid sequence depicted in SEQ ID NO: 14 (VH_3E7_var2) as shown in Figure 1 or Table IV or a variant thereof, wherein the variant comprises an amino acid sequence resulted from a partial alteration of SEQ ID NO: 14; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V9
   (i) the VH chain comprises the amino acid sequence depicted in SEQ ID NO: 16 (VH_3E7_var3) as shown in Figure 1 or Table IV or a variant thereof, wherein the variant comprises an amino acid sequence resulted from a partial alteration of SEQ ID NO: 16; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7;
NI-504.3E7_V10
   (i) the VH chain comprises the amino acid sequence depicted in SEQ ID NO: 16 (VH_3E7_var3) as shown in Figure 1 or Table IV or a variant thereof, wherein the variant comprises an amino acid sequence resulted from a partial alteration of SEQ ID NO: 16; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V11
   (i) the VH chain comprises the amino acid sequence depicted in SEQ ID NO: 16 (VH_3E7_var3) as shown in Figure 1 or Table IV or a variant thereof, wherein the variant comprises an amino acid sequence resulted from a partial alteration of SEQ ID NO: 16; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V12
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7;
NI-504.3E7_V13
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V14
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V15
   (i) the VH chain comprises the amino acid sequence depicted in SEQ ID NO: 18 (VH_3E7_var4) as shown in Figure 1 or Table IV or a variant thereof, wherein the variant comprises an amino acid sequence resulted from a partial alteration of SEQ ID NO: 18; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60;
NI-504.3E7_V16
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 7 (VL_3E7) as shown in Figure 1 or Table IV or a variant thereof, wherein the variant comprises an amino acid sequence resulted from a partial alteration of SEQ ID NO: 7;
NI-504.3E7_V17
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V18
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V19
   (i) the VH chain comprises the amino acid sequence depicted in SEQ ID NO: 20 (VH_3E7_var5) as shown in Figure 1 or Table IV or a variant thereof, wherein the variant comprises an amino acid sequence resulted from a partial alteration of SEQ ID NO: 20; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60;
NI-504.3E7_V20
   (i) the VH chain comprises the amino acid sequence depicted in SEQ ID NO: 22 (VH_3E7_var6) as shown in Figure 1 or Table IV or a variant thereof, wherein the variant comprises an amino acid sequence resulted from a partial alteration of SEQ ID NO: 22; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V21
   (i) the VH chain comprises the amino acid sequence depicted in SEQ ID NO: 24 (VH_3E7_var6a) as shown in Figure 1 or Table IV or a variant thereof, wherein the variant comprises an amino acid sequence resulted from a partial alteration of SEQ ID NO: 24; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V22
   (i) the VH chain comprises the amino acid sequence depicted in SEQ ID NO: 26 (VH_3E7_var6b) as shown in Figure 1 or Table IV or a variant thereof, wherein the variant comprises an amino acid sequence resulted from a partial alteration of SEQ ID NO: 26; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V23
   (i) the VH chain comprises the amino acid sequence depicted in SEQ ID NO: 28 (VH_3E7_var6c) as shown in Figure 1 or Table IV or a variant thereof, wherein the variant comprises an amino acid sequence resulted from a partial alteration of SEQ ID NO: 28; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V24
   (i) the VH chain comprises the amino acid sequence depicted in SEQ ID NO: 30 (VH_3E7_var6d) as shown in Figure 1 or Table IV or a variant thereof, wherein the variant comprises an amino acid sequence resulted from a partial alteration of SEQ ID NO: 30; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V25
   (i) the VH chain comprises the amino acid sequence depicted in SEQ ID NO: 32 (VH_3E7_var6ab) as shown in Figure 1 or Table IV or a variant thereof, wherein the variant comprises an amino acid sequence resulted from a partial alteration of SEQ ID NO: 32; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V26
   (i) the VH chain comprises the amino acid sequence depicted in SEQ ID NO: 34 (VH_3E7_var6ac) as shown in Figure 1 or Table IV or a variant thereof, wherein the variant comprises an amino acid sequence resulted from a partial alteration of SEQ ID NO: 34; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V27
   (i) the VH chain comprises the amino acid sequence depicted in SEQ ID NO: 36 (VH_3E7_var6ad) as shown in Figure 1 or Table IV or a variant thereof, wherein the variant comprises an amino acid sequence resulted from a partial alteration of SEQ ID NO: 36; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V28
   (i) the VH chain comprises the amino acid sequence depicted in SEQ ID NO: 38 (VH_3E7_var6bc) as shown in Figure 1 or Table IV or a variant thereof, wherein the variant comprises an amino acid sequence resulted from a partial alteration of SEQ ID NO: 38; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V29
   (i) the VH chain comprises the amino acid sequence depicted in SEQ ID NO: 40 (VH_3E7_var6bd) as shown in Figure 1 or Table IV or a variant thereof, wherein the variant comprises an amino acid sequence resulted from a partial alteration of SEQ ID NO: 40; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V30
   (i) the VH chain comprises the amino acid sequence depicted in SEQ ID NO: 42 (VH_3E7_var6cd) as shown in Figure 1 or Table IV or a variant thereof, wherein the variant comprises an amino acid sequence resulted from a partial alteration of SEQ ID NO: 42; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V31
   (i) the VH chain comprises the amino acid sequence depicted in SEQ ID NO: 44 (VH_3E7_var6bcd) as shown in Figure 1 or Table IV or a variant thereof, wherein the variant comprises an amino acid sequence resulted from a partial alteration of SEQ ID NO: 44; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V32
   (i) the VH chain comprises the amino acid sequence depicted in SEQ ID NO: 46 (VH_3E7_var6acd) as shown in Figure 1 or Table IV or a variant thereof, wherein the variant comprises an amino acid sequence resulted from a partial alteration of SEQ ID NO: 46; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V33
   (i) the VH chain comprises the amino acid sequence depicted in SEQ ID NO: 48 (VH_3E7_var6abd) as shown in Figure 1 or Table IV or a variant thereof, wherein the variant comprises an amino acid sequence resulted from a partial alteration of SEQ ID NO: 48; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V34
   (i) the VH chain comprises the amino acid sequence depicted in SEQ ID NO: 50 (VH_3E7_var6abc) as shown in Figure 1 or Table IV or a variant thereof, wherein the variant comprises an amino acid sequence resulted from a partial alteration of SEQ ID NO: 50; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V35
   (i) the VH chain comprises the amino acid sequence depicted in SEQ ID NO: 22 (VH_3E7_var6) as shown in Figure 1 or Table IV or a variant thereof, wherein the variant comprises an amino acid sequence resulted from a partial alteration of SEQ ID NO: 22; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V36
   (i) the VH chain comprises the amino acid sequence depicted in SEQ ID NO: 22 (VH_3E7_var6) as shown in Figure 1 or Table IV or a variant thereof, wherein the variant comprises an amino acid sequence resulted from a partial alteration of SEQ ID NO: 22; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60;
NI-504.3E7_V37
   (i) the VH chain comprises the amino acid sequence depicted in SEQ ID NO: 52 (VH_3E7_var7) as shown in Figure 1 or Table IV or a variant thereof, wherein the variant comprises an amino acid sequence resulted from a partial alteration of SEQ ID NO: 52; and/or
   (ii) the VL comprises the amino acid sequence depicted in SEQ ID NO: 56 (VL_3E7_var1) as shown in Figure 1 or Table IV or a variant thereof, wherein the variant comprises an amino acid sequence resulted from a partial alteration of SEQ ID NO: 56;
NI-504.3E7_V38
   (i) the VH chain comprises the amino acid sequence depicted in SEQ ID NO: 52 (VH_3E7_var7) as shown in Figure 1 or Table IV or a variant thereof, wherein the variant comprises an amino acid sequence resulted from a partial alteration of SEQ ID NO: 52; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58;
NI-504.3E7_V39
   (i) the VH chain comprises the amino acid sequence depicted in SEQ ID NO: 52 (VH_3E7_var7) as shown in Figure 1 or Table IV or a variant thereof, wherein the variant comprises an amino acid sequence resulted from a partial alteration of SEQ ID NO: 52; and/or
   (ii) the VL comprises the amino acid sequence depicted in SEQ ID NO: 60 (VL_3E7_var3) as shown in Figure 1 or Table IV or a variant thereof, wherein the variant comprises an amino acid sequence resulted from a partial alteration of SEQ ID NO: 60;
NI-504.3E7_V40
   (i) the VH chain comprises the amino acid sequence depicted in SEQ ID NO: 54 (VH_3E7_var8) as shown in Figure 1 or Table IV or a variant thereof, wherein the variant comprises an amino acid sequence resulted from a partial alteration of SEQ ID NO: 54; and/or
   (ii) the VL comprises the amino acid sequence depicted in SEQ ID NO: 58 (VL_3E7_var2) as shown in Figure 1 or Table IV or a variant thereof, wherein the variant comprises an amino acid sequence resulted from a partial alteration of SEQ ID NO: 58.

In another preferred embodiment, the variant VH and VL, respectively, comprises one or more amino acid substitutions, preferably one, two, three, four, five, six, seven, eight, nine, or ten amino acid substitutions, preferably wherein the variant of the VL chain comprises one, two, three, four, or five, preferably two, four, or five amino acid substitutions, and/or the variant of the VH chain comprises one, two, three, four, five, six, seven, eight, nine, or ten, preferably two, three, four, five six, seven, eight, or ten amino acid substitutions. Most preferably, the variant VH and VL, respectively comprises one or two amino acid substitutions.

In another preferred embodiment, the variant of the mentioned VH and VL chains, respectively comprises an amino acid sequence which is at least 90 %, preferably 91%, preferably 92%, preferably 93%, preferably 94%, preferably 95%, preferably 96%, preferably 97%, preferably 98%, preferably 99% identical to the sequences set forth in SEQ ID NO: 2, SEQ ID NO: 7, SEQ ID NO: 12, and SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, respectively.

In addition, the present invention relates to a polynucleotide linked to a heterologous nucleic acid, wherein the polynucleotide is selected from the group consisting of:
(a) a polynucleotide encoding an immunoglobulin heavy chain or a fragment thereof comprising a heavy chain variable region (VH) comprising CDRs 1, 2, and 3 with the amino acid sequences set forth in SEQ ID NOs: 3, 4, and 5, respectively, and wherein the VH when paired with a light chain variable region (VL) comprising the amino acid sequence set forth in SEQ ID NO: 7, SEQ ID NO: 56, SEQ ID NO: 58, or SEQ ID NO: 60 binds to CRMP2;
(b) a polynucleotide encoding an immunoglobulin light chain or a fragment thereof comprising a VL comprising CDRs 1, 2, and 3 with the amino acid sequences set forth in SEQ ID NOs: 8, 9, and 10, respectively, or SEQ ID NOs: 8, 9, and 61, respectively and wherein the VL when paired with a VH comprising the amino acid sequence set forth in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, or SEQ ID NO: 54, binds to CRMP2;
   a polynucleotide encoding
   (i) an immunoglobulin heavy chain or a fragment thereof comprising a VH comprising CDRs 1, 2, and 3 with the amino acid sequences set forth in SEQ ID NOs: 3, 4, and 5, respectively; and
   (ii) an immunoglobulin light chain or a fragment thereof comprising a VL comprising CDRs 1, 2, and 3 with the amino acid sequences set forth in SEQ ID NOs: 8, 9, and 10, respectively or SEQ ID NOs: 8, 9, and 61, respectively;
(d) a polynucleotide encoding an immunoglobulin heavy chain or a fragment thereof comprising a VH comprising the amino acid sequence set forth in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, or SEQ ID NO: 54, wherein the VH when paired with a VL comprising the amino acid sequence set forth in SEQ ID NO: 7, SEQ ID NO: 56, SEQ ID NO: 58, or SEQ ID NO: 60 binds to CRMP2;
(e) a polynucleotide encoding an immunoglobulin light chain or a fragment thereof comprising a VL comprising the amino acid sequence set forth in SEQ ID NO: 7, SEQ ID NO: 56, SEQ ID NO: 58, or SEQ ID NO: 60, wherein the VL when paired with a VH comprising the amino acid sequence set forth in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, or SEQ ID NO: 54 binds to CRMP2;
(f) a polynucleotide encoding an immunoglobulin heavy chain or a fragment thereof comprising a VH comprising the amino acid sequence set forth in SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, or SEQ ID NO: 54 and an immunoglobulin light chain or a fragment thereof comprising a VL comprising the amino acid sequence set forth in SEQ ID NO: 7, SEQ ID NO: 56, SEQ ID NO: 58, or SEQ ID NO: 60;
(g) a polynucleotide as in any one of (a)-(f), wherein a CDR comprises one or more, preferably no more than two amino acid substitution and/or the variable region sequence is at least 90% identical to SEQ ID NO: 2, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, or SEQ ID NO: 54 or SEQ ID NO: 7, SEQ ID NO: 56, SEQ ID NO: 58, or SEQ ID NO: 60.

The assignment of the SEQ ID NOs to the corresponding CDRs, as well as to the VH and VL chains in provided in Tables V and IV, wherein the preferred combinations of the VH and VL chains are shown in Table VI.

In a preferred embodiment, the immunoglobulin may, when paired as VH and VL, bind CRMP2 as measured by direct or capture ELISA or an equivalent assay to the assay described in the Examples.

Furthermore, the present invention relates to a vector and vectors comprising one or more of those polynucleotides, preferably wherein the vector is an expression vector, and the one or more polynucleotide(s) are operably linked to expression control sequences.

The polynucleotides may be produced and, if desired manipulated using methods well known in the art for the manipulation of nucleotide sequences, *e.g.,* recombinant DNA techniques, site directed mutagenesis, PCR, etc. (see, for example, the techniques described in Molecular Cloning: A Laboratory Manual (Fourth Edition): Three-volume set; Green and Sambrook (2012) ISBN 10: 1936113422 / ISBN 13: 9781936113422 Cold Spring Harbor Laboratory Press; update (2014) ISBN 978-1-936113-42-2 and Ausubel et al., eds., Current Protocols in Molecular Biology, John Wiley & Sons, NY (1998) and updates, which are both incorporated by reference herein in their entireties), to generate antibodies having a different amino acid sequence, for example to create amino acid substitutions, deletions, and/or insertions.

Once a polynucleotide encoding an antibody molecule or a heavy or light chain of an antibody, or portion thereof (preferably containing the heavy or light chain variable domain), of the invention has been obtained, the vector for the production of the antibody molecule may be produced by recombinant DNA technology using techniques well known in the art. Thus, methods for preparing a protein by expressing a polynucleotide containing an antibody encoding nucleotide sequence are described herein. Methods which are well known to those skilled in the art can be used to construct expression vectors containing antibody coding sequences and appropriate transcriptional and translational control signals. These methods include, for example, *in vitro* recombinant DNA techniques, synthetic techniques, and *in vivo* genetic recombination. The invention, thus, provides replicable vectors comprising a nucleotide sequence encoding an antibody molecule of the invention, or a heavy or light chain thereof, or a heavy or light chain variable domain, operable linked to a promoter. Such vectors may include the nucleotide sequence encoding the constant region of the antibody molecule (*see, e.g.,* international applications WO 86/05807 A1 and WO 89/01036 A1; and US patent no. 5,122,464) and the variable domain of the antibody may be cloned into such a vector for expression of the entire heavy or light chain.

The term "vector" or "expression vector" is used herein to mean vectors used in accordance with the present invention as a vehicle for introducing into and expressing a desired gene in a host cell. In general, vectors compatible with the instant invention will comprise a selection marker, appropriate restriction sites to facilitate cloning of the desired gene and the ability to enter and/or replicate in eukaryotic or prokaryotic cells. The marker may provide for prototrophy to an auxotrophic host, biocide resistance (*e.g.,* antibiotics), or resistance to heavy metals such as copper. The selectable marker gene can either be directly linked to the DNA sequences to be expressed, or introduced into the same cell by co-transformation. Additional elements may also be needed for optimal synthesis of mRNA. These elements may include signal sequences, splice signals, as well as transcriptional promoters, enhancers, and termination signals. For the expression of double-chained antibodies, a single vector or vectors encoding both the heavy and light chains may be co-expressed in the host cell for expression of the entire immunoglobulin molecule, as detailed below.

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain is advantageously placed before the heavy chain to avoid an excess of toxic free heavy chain; *see* Proudfoot, Nature 322 (1986), 52; Kohler, Proc. Natl. Acad. Sci. USA 77 (1980), 2197. The coding sequences for the heavy and light chains may comprise cDNA or genomic DNA. The expression vector(s) is (are) transferred to a host cell by conventional techniques and the transfected cells are then cultured by conventional techniques to produce an antibody for use in the methods described herein. Accordingly, the present invention also relates to host cells comprising one or more polynucleotides or a vector or vectors of the present invention.

As used herein, "host cells" refers to cells which harbor vectors constructed using recombinant DNA techniques and encoding at least one heterologous gene. In descriptions of processes for isolation of antibodies from recombinant hosts, the terms "cell" and "cell culture" are used interchangeably to denote the source of antibody unless it is clearly specified otherwise. In other words, recovery of polypeptide from the "cells" may mean either from spun down whole cells, or from the cell culture containing both the medium and the suspended cells.

Antibodies used for laboratory research/diagnosis may be expressed in any suitable host, *e.g.* in mammalian cells, bacterial cells, yeasts, plant cells or insect cells. However, currently almost all therapeutic antibodies are still produced in mammalian cell lines in order to reduce the risk of immunogenicity due to altered, non-human glycosylation patterns. However, recent developments of glycosylation-engineered yeast, insect cell lines, and transgenic plants are promising to obtain antibodies with "human-like" post-translational modifications. Furthermore, smaller antibody fragments including bispecific antibodies without any glycosylation are successfully produced in bacteria and have advanced to clinical testing. The first therapeutic antibody products from a non-mammalian source can be expected in coming next years. A review on current antibody production systems that can be applied for preparing the human-derived recombinant anti-CRMP2 antibody or CRMP2 binding fragment or derivate thereof of the present invention including their usability for different applications is given in Frenzel et al., Front Immunol. 4 (2013), 217, published online on July 29, 2013 doi: 10.3389/fimmu.2013.00217 and transient expression of human antibodies in mammalian cells is described by Vazquez-Lombardi et al., Nature protocols 13 (2018), 99-117; and Hunter et al., Optimization of protein expression in mammalian cells. Current Protocols in Protein Science 95 (2019), e77. doi: 10.1002/cpps.77.

Once an antibody molecule of the invention has been recombinantly expressed, the whole antibodies, their dimers, individual light and heavy chains, or other immunoglobulin forms of the present invention can be purified according to standard procedures of the art, including for example, by chromatography (*e.g.,* ion exchange, affinity, particularly by affinity for the specific antigen after Protein A, and sizing column chromatography), centrifugation, differential solubility, *e.g.* ammonium sulfate precipitation, or by any other standard technique for the purification of proteins; see, *e.g.,* Scopes, "Protein Purification", Springer Verlag, N.Y. (1982) and Antibodies A Laboratory Manual 2nd edition, 2014 by Cold Spring Harbor Laboratory Press, Cold Spring Harbor, New York, USA. Thus, the present invention also relates to a method for preparing an anti-CRMP2 antibody and/or fragments thereof or immunoglobulin chain(s) thereof, said method comprising:
(a) culturing the host cell as defined hereinabove, which cell comprised the polynucleotide(s) or vector(s) as defined hereinbefore under conditions allowing for expression of the anti-CRMP2 antibody, CRMP2-binding fragment or immunoglobulin chain(s) thereof; and
(b) isolating the anti-CRMP2 antibody, CRMP2 binding fragment or immunoglobulin chain(s) thereof from the culture.

Furthermore, the present invention also relates to the anti-CRMP2, CRMP2 binding fragment, derivatives and immunoglobulin chain(s) thereof encoded by a polynucleotide of the present invention described hereinbefore and obtainable by the method for their recombinant production; *see supra.*

The antibody of the present invention may also be used in diagnostic approaches. Since the antibody of the present invention binds pCRMP2 and CRMP2, total CRMP2 can be detected. Accordingly, in one embodiment, the present invention relates to a method of diagnosing, wherein the method comprises determining the total level of CRMP2 (CRMP2 and pCRMP2) in a sample from the subject to be diagnosed with at least one antibody of the present invention or an CRMP2 binding fragment thereof, wherein an increased level of total CRMP2 in comparison to a control (*e.g.,* a sample of a healthy subject, or a sample of a subject which has a diagnosed disease associated with an increased level of total CRMP2) is indicative of a corresponding disease in said subject.

The subject being diagnosed with such a disease is administered an anti-CRMP2 antibody of the invention. Alternatively, the subject is diagnosed in accordance with the method of the present invention, the information is transmitted directly or indirectly to the subject or to a physician or medical institute, and if the subject has been diagnosed with a disease associated with CRMP2 pathology, the subject is treated with an agent which is capable of ameliorating, treating or reducing the progression of at least one symptom of the disease in the subject. Preferably, the agent is an anti-CRMP2 antibody, most preferably an antibody of the present invention.

The subject to be diagnosed may be asymptomatic or preclinical for the disease. In one embodiment, the subject to be diagnosed and the control subject(s) are age-matched. The sample to be analyzed may be any body fluid suspected to contain CRMP2/pCRMP2, for example blood, CSF, or tissue.

The level of CRMP2/pCRMP2 may be assessed by any suitable method known in the art comprising, *e.g.,* analyzing CRMP2/pCRMP2 by one or more techniques chosen from Western blot, immunoprecipitation, enzyme-linked immunosorbent assay (ELISA), radioimmunoassay (RIA), fluorescent activated cell sorting (FACS), two-dimensional gel electrophoresis, mass spectroscopy (MS), matrix-assisted laser desorption/ionization-time of flight-MS (MALDI-TOF), surface-enhanced laser desorption ionization-time of flight (SELDI-TOF), high performance liquid chromatography (HPLC), fast protein liquid chromatography (FPLC), multidimensional liquid chromatography (LC) followed by tandem mass spectrometry (MS/MS), laser densitometry, and IHC.

Furthermore, the anti-CRMP2 antibody or CRMP2-binding fragment or derivative thereof can be used for *in vivo* imaging of CRMP2/pCRMP2. Thus, in one embodiment, said *in vivo* imaging of CRMP2/pCRMP2 comprises positron emission tomography (PET), single photon emission tomography (SPECT), near infrared (NIR) optical imaging or magnetic resonance imaging (MRI).

In certain embodiments, the antibody polypeptide comprises an amino acid sequence or one or more moieties not normally associated with an antibody. Thus, the present invention further encompasses antibodies, or antigen-binding fragments, variants, or derivatives thereof of the invention conjugated to a diagnostic or therapeutic agent. The antibodies can be used diagnostically to, for example, demonstrate presence of a disease associated with abnormal level/location of pCRMP2/CRMP2 as described above, to indicate the risk of getting a disease associated with abnormal level/location of pCRMP2/CRMP2, to monitor the development or progression of a disease associated with abnormal level/location of pCRMP2/CRMP2, *e.g.,* as part of a clinical testing procedure to, *e.g.,* determine the efficacy of a given treatment and/or prevention regimen. Detection can be facilitated by coupling the antibody, or antigen-binding fragment, variant, or derivative thereof to a detectable substance. Exemplary modifications are described in more detail below. For example, the antibody or CRMP2-binding fragment thereof such a single-chain Fv antibody fragment of the invention may comprise a flexible linker sequence, or may be modified to add a functional moiety or detectable label (*e.g.,* PEG, a drug, a toxin, or a label such as a fluorescent, (chemo/bio)luminescent, radioactive, enzyme, nuclear magnetic, heavy metal, a tag, a flag and the like); see, *e.g.,* Antibodies A Laboratory Manual 2nd edition, 2014 by Cold Spring Harbor Laboratory Press, Cold Spring Harbor, New York, USA for general techniques; Dean and Palmer, Nat. Chem. Biol. 10 (2014), 512-523, for advances in fluorescence labeling strategies for dynamic cellular imaging; and Falck and Müller, Antibodies 7 (2018), 4; doi: 10.3390/antib7010004 for enzyme-based labeling strategies for antibody-drug conjugates and antibody mimetics.

Furthermore, the anti-CRMP2 antibody or CRMP2-binding fragments or derivatives thereof of the present invention can comprise an organ specific targeting entity, in particular a brain targeting entity and/or is contained in or conjugated to a vehicle such as an exosome or nanoparticle for delivery to the brain.

As it is well known in the art, the blood-brain barrier (BBB) restricts drug efficacy for central nervous system (CNS) diseases. For example, monoclonal antibodies do not cross the BBB efficiently, reaching a maximum of 0.11% at 1 h after injection (Banks et al. (2002), Peptides 23, 2223-2226). The BBB is a specialized structural, physiological and biochemical barrier and serves as the first interface between the changeable environment of blood and the extracellular fluid in the CNS. The BBB regulates the homeostasis of the nervous system by strictly controlling the movement of small molecules or macromolecules from the blood to the brain. It only permits selective transport of molecules that are essential for brain function. In detail, more than 98% of small molecule drugs and almost 100% of large molecule drugs are precluded from drug delivery to brain (Redzic (2011) Fluids Barriers CNS 8, 3; Pardridge (2005) NeuroRx, 2, 3-14). Thus, the polypeptide and the antibody, antigen-binding fragment thereof, variant or derivative thereof, respectively may be modified in order to be able to penetrate the BBB.

For example, said antibodies and binding fragments can be fused to cell-penetrating peptides (CPPs), which qualify as brain targeting entity and which are usually short cationic and/or amphipathic peptides that have the ability to transport the associated molecular cargo (*e.g.,* peptides, proteins, antibodies, *etc.*) across cellular membranes. However, also anionic CCPs have been reported. Examples are given in Sharma et al. (2016) Int. J. Mol. Sci. 17, 806 and instructions how to fuse an antibody with a CPP are for example provided in Gaston et al. (2019) Sci. Rep. 9, 18688 doi:10.1038/s41598-019-55091-0. Furthermore, polyamine modification has been shown to dramatically increase the penetration of *i.a.* antibodies across the BBB (Poduslo and Curran (1996) J. Neurochem. 66, 1599-1609). The most investigated method to deliver macromolecules into the brain is via receptor-mediated transcytosis (RMT) and the main RMT receptors that have been studied are the transferrin receptor (TfR) and insulin receptor (IR). Thus, RMT receptors are also brain targeting entities. For example, bispecific antibodies have emerged as promising scaffolds to deliver therapeutic antibodies to the brain via engineering the antibody to incorporate one arm with specificity against a BBB RMT receptor, which drives their transmission across the BBB, and the other arm against a CNS therapeutic agent. Essentially, bispecific antibodies can be generated by fusion of antibody fragments such as Fabs, scFv or single domain antibodies into the N- or C-terminal of a convention IgG molecule or by heterodimerization strategies such as the "knobs-into-holes" technology developed by Genentech; see for details Neves et al. (2016) Trends Biotech. 34, 36-48.

Thus, the anti-Tau antibody of the present invention can be a bispecific antibody binding to Tau and to a BBB RMT receptor.

In another approach, lipid nanoparticles / nanoexosomes can be used to deliver the antibodies or binding fragments of the present invention across the BBB or towards the brain or kidney. For example, dually decorated nanoliposomes with an anti-CRMP2 monoclonal antibody and an anti-RMT antibody, *e.g.* anti-TfR monoclonal antibody using biotin streptavidin conjugation can be used for improved delivery across the blood brain barrier. This principle is outlined in Markoutsa et al. (2012) Eur. J. Pharm. Biopharm. 81, 49-56) and the principle of delivery to the heart or kidney is outlined in Huang et al., Front. Bioeng. Biotechnol. 9 (2021), DOI: 10.3389/fbioe.2021.683247 and Yang et al., Adv. Drug Deliv. Rev. 160 (2020), 1-18.

Furthermore, as summarized in Tosi et al. (2013) (Curr. Med. Chem. 20, 2212-25), biodegradable nanoparticles formulated from poly(D,L-lactide-co-glycolide) (PLGA) have been extensively investigated for sustained and targeted delivery of different agents. Thus, the antibodies and binding fragments of the present invention are conjugated to nanoparticles and nanoexosomes, respectively.

The anti-CRMP2 antibody or CRMP2-binding fragment, synthetic derivative, or biotechnological derivative thereof, in particular the human-derived recombinant antibody, optionally as fusion protein and/or labeled as described hereinbefore is then provided for various applications in accordance with standard techniques known in the art; see, *e.g.,* Antibodies A Laboratory Manual 2nd edition, 2014 by Cold Spring Harbor Laboratory Press, Cold Spring Harbor, New York, USA. Current advancements in therapeutic antibody design, manufacture, and formulation are described in Sifniotis et al., Antibodies 2019, 8(2), 36; https://doi.org/10.3390/antib8020036, wherein also developments in computational approaches for the strategic design of antibodies with modulated functions are discussed.

The present invention relates to compositions comprising the aforementioned CRMP2-binding molecule of the present invention, *e.g.,* antibody or CRMP2-binding fragment, variant or biotechnological derivative thereof, or the polynucleotide(s), vector(s) or cell of the invention as defined hereinbefore. In one embodiment, the composition of the present invention is a pharmaceutical composition and further comprises a pharmaceutically acceptable carrier.

The present invention also provides the pharmaceutical and diagnostic composition, respectively, in form of a pack or kit comprising one or more containers filled with one or more of the above-described ingredients, *e.g.,* anti-CRMP2 antibody, CRMP2-binding fragment, biotechnological derivative or variant thereof, polynucleotide, vector or cell of the present invention. Associated with such container(s) can be a notice in the form prescribed by a governmental agency regulating the manufacture, use or sale of pharmaceuticals or biological products, which notice reflects approval by the agency of manufacture, use or sale for human administration. In addition, or alternatively the kit comprises reagents and/or instructions for use in appropriate immuno-based diagnostic assays. The composition, *e.g.* kit of the present invention is of course particularly suitable for the risk assessment, diagnosis, prevention and treatment of a disease or disorder which is accompanied with the presence of CRMP2, and in particular applicable for the treatment of disorders generally associated with CRMP2 as discussed herein above.

The pharmaceutical compositions of the present invention can be formulated according to methods well known in the art; see for example, Remington: The Science and Practice of Pharmacy (2000) by the University of Sciences in Philadelphia, ISBN 0-683-306472. Examples of suitable pharmaceutical carriers are well known in the art and include phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be effected by different ways, *e.g.,* by intravenous, intraperitoneal, subcutaneous, intramuscular, intranasal, topical or intradermal administration or spinal or brain delivery. Aerosol formulations such as nasal spray formulations include purified aqueous or other solutions of the active agent with preservative agents and isotonic agents. Such formulations are preferably adjusted to a pH and isotonic state compatible with the nasal mucous membranes.

The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently; see for example Bai et al., Clinical Pharmacokinetics 51 (2012), 119-135.

As explained in detail above, the antibody of the present invention has been shown to improve the cognitive function in aged mice and to reduce amyloid beta plaque load in the cortex and hippocampus of mice. Accordingly, the present invention provides for the first time a therapeutically useful anti-CRMP2 antibody. Thus, the present invention relates in one embodiment to the antibody or antigen-binding fragment, derivative or variant thereof for use as a medicament.

The experiments showing the improvement of cognitive function in aged mice and the reduction of amyloid beta load have been performed in an AD disease mouse model. Furthermore, it is known that pCRMP2 is co-localized with pTau in neurofibrillary tangles and experiments performed within the scope of the present invention confirmed that pCRMP2 co-aggregates with pTau. In addition, staining of hippocampal tissue of patients with different tauopathies with the antibody of the present invention was observed. Thus, it is prudent to expect that the antibodies of the present invention and corresponding CRMP2 binding fragments thereof are not only useful for the treatment of AD, but also useful for the treatment of further neurodegenerative diseases and in particular for the treatment of further diseases associated with Tau and/or Aβ pathology, more specifically with cognitive disorders, diseases associated with loss of memory and learning ability and of a neurodegenerative disease in general.

The antibody of the present invention may reduce or eliminate at least one symptom of a neurodegenerative disease in a subject. The symptom may be hyperphosphorylation of CRMP2, accumulation of pCRM2, in particular in neurofibrillary tangles, the depletion of CRMP2, co-aggregation of pCRMP2 and pTau, and/or the accumulation of amyloid beta plaques in the brain of a subject.

Neurodegenerative disorders are chronic disorders characterized by progressive loss of neurons in the brain and spinal cord, wherein a prominent and most prevalent group represents dementia-associated tauopathies. Neurodegenerative tauopathies share a common pathologic lesion consisting of intracellular aggregates of abnormal filaments that are mainly composed of pathologically hyperphosphorylated Tau in neurons and/or glial cells. Clinical features of the tauopathies are heterogeneous and characterized by dementia and/or motor syndromes. The progressive accumulation of filamentous Tau inclusions may cause neuronal and glial degeneration in combination with other deposits as, *e.g.,* beta-amyloid in AD. Because of the heterogeneity of their clinical manifestations a potentially non-exhaustive list of tauopathic diseases which are also associated with loss of memory may be provided including AD, amyotrophic lateral sclerosis and parkinsonism-dementia complex (ALS-PDC), argyrophilic grain disease (AGD), British type amyloid angiopathy, cerebral amyloid angiopathy, Creutzfeldt-Jakob Disease (CJD), dementia pugilistica, diffuse neurofibrillary tangles with calcification, FTD, FTDP-17, frontotemporal lobar degeneration (FLD), Hallervorden-Spatz disease, PiD, prion protein cerebral amyloid angiopathy, progressive subcoitical gliosis, PSP, tangle only dementia, and multi-infarct dementia; see for a review, *e.g.,* Lee et al. (2001) Annu. Rev. Neurosci. 24, 1121-1159 in which Table 1 catalogs the unique members of tauopathies or Sergeant et al. (2005), Bioch. Biophy. Acta 1739, 179-97, with a list in Figure 2 therein.

In one embodiment, the present invention relates to the antibody or corresponding CRMP2 binding fragments for use in the treatment of a disorder of the nervous system, preferably of a disease associated with Tau and/or Aβ pathology, in particular with cognitive disorders, diseases associated with loss of memory and learning ability and of a neurodegenerative disease in general. In a preferred embodiment, the present invention relates to the antibody or corresponding CRMP2 binding fragments for use in the treatment of a disorder of the nervous system, including but not limited to AD, ALS-PDC, AGD, British type amyloid angiopathy, cerebral amyloid angiopathy, CJD, dementia pugilistica, diffuse neurofibrillary tangles with calcification, FTD, FTDP-17, FLD, Hallervorden-Spatz disease, PiD, prion protein cerebral amyloid angiopathy, progressive subcoitical gliosis, PSP, tangle only dementia, multi-infarct dementia, Lewy body disease, mild cognitive impairment, cognitive impairment, dementia with Lewy bodies (DLB), Parkinson's disease, ALS, spinal cord injury, Traumatic brain injury, Huntington's disease, multiple sclerosis, corticobasal degeneration (CBD), stroke, cerebrovascular disease, neurological disorders, CNS disorders, neuropathic pain, chronic pain, lower back pain, schizophrenia, depression, anxiety disorder, bipolar disorder, autism spectrum disorder, attention deficit/hyperactivity disorder, learning disabilities, movement disorders, obsessive-compulsive disorder, personality disorder, sleeping disorder, delirium, developmental disorders, intellectual disability, and post-traumatic stress disorder. In a further preferred embodiment, the present invention relates to the antibody or corresponding CRMP2 binding fragments for use in the treatment of a tauopathy, including the diseases mentioned above, preferably AD, ALS-PDC, AGD, British type amyloid angiopathy, cerebral amyloid angiopathy, CJD, dementia pugilistica, diffuse neurofibrillary tangles with calcification, FTD, FTDP-17, FLD, Hallervorden-Spatz disease, PiD, prion protein cerebral amyloid angiopathy, progressive subcoitical gliosis, PSP, tangle only dementia, and multi-infarct dementia, most preferably AD, PSP, PiD, FTD, and FLD, and most preferably of AD.

Hence, the present invention also relates to a method of treating a disease associated with Tau and/or Aβ pathology, in particular cognitive disorders, diseases associated with loss of memory and learning ability and of a neurodegenerative disease in general, which method comprises administering to a subject in need thereof a therapeutically effective amount of any one of the afore-described CRMP2-binding molecules, in particular human-derived antibodies of the instant invention. The present invention provides a method of treating a neurodegenerative disease in a subject by administering a therapeutically effective amount of any one of the anti-CRMP2 binding molecules of the invention, wherein the administration of the anti-CRMP2 antibody ameliorates, treats or reduces the progression of at least one symptom of the neurodegenerative disease in the subject. Preferably, the method is used for the treatment of a neurodegenerative disease, preferably of a tauopathy, including the diseases mentioned above, preferably of AD, PSP, PiD, FTD, and FLD, and most preferably of AD.

In addition, in one embodiment the present invention relates to peptides having an epitope of (p)CRMP2 specifically recognized by any antibody of the present invention, preferably by antibody NI-504.3F4 in which one or more amino acids are substituted, deleted and/or added, wherein the peptide is recognized by any antibody of the present invention. In a preferred embodiment, the peptide of the present invention consists of 10 to 25 contiguous amino acids of CRMP2 and comprises the amino acid sequence ASSAK (SEQ ID NO: 64), optionally wherein the peptide further comprises a heterologous amino acid sequence or functional moiety. Such heterologous amino acid sequence or functional moiety are defined hereinbefore. Most preferably, the peptide is the one set forth in SEQ ID NO: 62 or 63.

In one embodiment of this invention, such a peptide may be used for antibody screening, for example to screen for CRMP2 binding molecules equivalent to antibody NI-504.3E7 and/or for antibodies competing with the antibodies of the present invention, or such peptides may be used as an immunogen to raise antibodies for diagnostic use, for example in mice or rabbits. The peptide of the present invention may be formulated in an array, a kit and composition, respectively, as described hereinbefore. In this context, the present invention also relates to a kit as mentioned before, but further comprising the peptide of the present invention.

Several documents are cited throughout the text of this specification. The contents of all cited references (including literature references, issued patents, published patent applications as cited throughout this application including the background section and manufacturer's specifications, instructions, etc.) are hereby expressly incorporated by reference; however, there is no admission that any document cited is indeed prior art as to the present invention.

A more complete understanding can be obtained by reference to the following specific examples which are provided herein for purposes of illustration only and are not intended to limit the scope of the invention.

### EXAMPLES

In the following, the results of experiments performed with the original antibody NI-504.3E7 are shown. However, these results have been confirmed with one or more of the variants, *i.e.,* with one or more of any one of NI-504.3E7_V1 to NI-504.3E7_V40.

### Example 1: Isolation, identification, cloning and recombinant expression of anti-CRMP2 antibodies

In attempt to identify and isolate potential CRMP and CRMP2 specific antibodies that may have a protective effect in aged humans, cDNA sequences encoding human antibodies binding recombinant human CRMP2 and pCRMP2 were derived from a de-identified blood lymphocyte library collected from healthy elderly subjects applying the proprietary Reverse Translational Medicine^{™} (RTM) technology platform, Neurimmune AG, 8952 Schlieren / Zurich, Switzerland. Positive hits were counter-screened to exclude clones cross-reacting with unrelated proteins. Selected CRMP2-reactive B-cell clones were subjected to cDNA cloning of IgG heavy and light-chain variable region sequences (for selected antibodies see Table IV), and sub-cloned into expression vectors encoding human IgG1 constant domain sequences. Expression constructs encoding IgG1 heavy and light chains were transiently expressed in ExpiCHO cell line and purified by protein A affinity chromatography. The result of the first screen is summarized in Table I.

**Table VI: Overview of 21 human CRMP2 antibodies tested in ELISA assays for their binding affinity (EC₅₀ value) to full length CRMP2 and to CRMP2 peptides, in Western Blot assays (WB) for their binding towards full length CRMP2 and phosphorylated CRMP2 (pCRMP2), and in immunohistochemistry assays (IHC) for their binding in human hippocampal tissue. "n.d." = not detectable; "n.a." = not assessed.**

| **Antibody** | **CRMP2-protein [EC₅₀]** | **CRMP2-peptide [EC₅₀]** | **Western Blot** | **IHC** |
|---|---|---|---|---|
| NI-504.1 | n.d. | <20 nM | CRMP2/pCRMP2 | n.d. |
| NI-504.2 | n.d. | <20 nM | n.d. | n.d. |
| NI-504.3 | n.d. | <20 nM | pCRMP2/pCRMP4 | n.d. |
| NI-504.4 | n.d. | <20 nM | CRMP2/pCRMP2 | n.d. |
| **NI-504-3E7** | **<20 nM** | **<20 nM** | **CRMP2/pCRMP2** | **signal at 0.2 µg/mL** |
| NI-504.5 | n.d. | n.a. | low CRMP2/pCRMP2 | n.d. |
| NI-504.6 | n.d. | n.d. | n.a. | n.d. |
| NI-504.7 | n.d. | n.d. | n.a. | n.d. |
| NI-504.8 | n.d. | <20 nM | n.a. | n.a. |
| NI-504.9 | n.d. | >20 nM | n.a. | n.a. |
| NI-504.10 | <20 nM | <20 nM | CRMP2/pCRMP2/CRMP1 | low signal at 5 µg/mL |
| NI-504.11 | >20 nM | <20 nM | CRMP1/pCRMP1/CRMP2 /pCRMP2 | n.a. |
| NI-504.12 | <20 nM | <20 nM | CRMP2/pCRMP2/pCRMP4 | signal at 0.2 µg/mL |
| NI-504.13 | n.d. | n.d. | n.d. | n.a. |
| NI-504.14 | n.d. | n.a. | n.a. | n.a. |
| NI-504.15 | >20 nM | n.a. | n.a. | n.a. |
| NI-504.16 | n.d. | n.a. | n.a. | n.a. |
| NI-504.17 | >20 nM | n.a. | n.a. | n.a. |
| NI-504.18 | n.d. | n.a. | n.a. | n.a. |
| NI-504.19 | n.d. | n.a. | n.a. | n.a. |
| NI-504.20 | n.d. | n.a. | n.a. | n.a. |

### Example 2: NI-504.3E7 selectively binds to human CRMP2

Antibody NI-504.3E7 was tested for its binding specificity to full length CRMP1, CRMP2, CRMP3, CRMP4, and CRMP5 via direct ELISA and Western Blot.

### ELISA assay

For direct ELISA, 96-well microplates (Costar) were coated with the recombinant human CRMP1-5 proteins (CRMP1 (Origene), CRMP2 (Origene), CRMP3 (Origene), CRMP4 (Origene), and CRMP5 (Origene)) in PBS (Gibco) at 5 µg/mL and incubated overnight at 4°C.

Plates were blocked in blocking buffer (2% BSA (Sigma-Aldrich) in PBS-T (phosphate buffered saline, Fisher Bioreagents, Cat.BP399; containing 0.1% Tween20 (Sigma-Aldrich) for 1h at room temperature. After 3 washing steps with PBS-T using a plate washer (Bio Tek Microplate staker 3), the plates were incubated with 30 µL/well serial dilutions of antibody NI-504.3E7 for 1h at room temperature, followed by 3 more washing steps in PBS-T. Bound antibody NI-504.3E7 was detected using HRP-conjugated anti-human Fc secondary antibody (Jackson ImmunoResearch, Cat.709-036-098) for 1h at room temperature, followed by 3 washing steps with PBS-T and conversion of chromogenic substrate TMB (Clinical Science) for absorbance measurement at 450nm using a plate reader (Thermo Fisher Scientific). For estimation of half-maximal concentration EC₅₀ log transformed absorbance values were fitted using a log(agonist) vs. response - variable slope (four parameters) curve fitting applying formula Y=Bottom + (Top-Bottom)/(1+10^((LogEC50-X)^{∗}HillSlope)) was used in GraphPad Prism Version 9.3. Data is displayed as mean + SEM.

### Western Blot

For Western Blot analysis, 0.2 µg of recombinant human CRMP1 (Origene), CRMP2 (Origene), CRMP3 (Origene), CRMP4 (Origene), and CRMP5 (Origene) were denatured in LDS sample buffer (NuPAGE) + 4% β-Mercapto-Ethanol (Sigma-Aldrich)for 5min at 95°C. Samples were loaded onto a 4-12% Bis-Tris Protein Gel (NuPAGE) and gel was run for 45min at 200V in 1x MOPS SDS Running Buffer (NuPAGE). Proteins were transferred onto PVDF membrane (NuPAGE) using iBlot for 7min and 20V. Membrane was blocked with 2% BSA (Sigma-Aldrich) in PBS-T (phosphate buffered saline, Fisher Bioreagents, Cat.BP399; containing 0.1% Tween20 (Sigma-Aldrich)) for 1h at room temperature and antibody NI-504.3E7 was incubated in 2% BSA in PBS-T at 5 µg/mL for 1h at room temperature. After 3 washing steps with PBS-T, the membrane was incubated with HRP-conjugated anti-human-Fc secondary antibody (Jackson ImmunoResearch, Cat.709-036-098) for 1h at room temperature and washed again 3 times with PBS-T and incubated with ECL (Amersham) for 2min. The chemiluminescence signal was detected using Image Quant LAS 4000 (Amersham).

### Results

In the ELISA assays, preferential binding of antibody NI-504.3E7 to CRMP2 over CRMP1, CRMP3, CRMP4, and CRMP5 was observed. In particular, antibody NI-504.3E7 has been shown to bind with high affinity, *i.e.,* with an EC₅₀ of 4 nM to CRMP2, wherein only weak binding to CRMP1 with an EC₅₀ of 69 nM was observed; see Figure 2A. Furthermore, even weaker binding to CRMP3, CRMP4, and CRMP5 was observed. Same results were obtained by Western Blot analysis. In particular, antibody NI-504.3E7 displays strong binding to CRMP2, only very weak binding to CRMP1, and no binding to CRMP3, CRMP4, and CRMP5; see Figure 2B. Thus, antibody NI-504.3E7 binds selectively to CRMP2.

### Example 3: NI-504.3E7 binds to a linear epitope at the C-terminus of CRMP2

To assess the binding epitope of the antibody NI-504.3E7, epitope mapping was performed using a cellulose membrane array with overlapping peptides of 15 amino acid length and 11 amino acid overlap, covering the full length of the CRMP2 protein. The membrane was produced by JPT Membrane Technologies (Germany). The membrane was blocked with 1x RotiBlock for 1h (Roth) at room temperature and antibody NI-504.3E7 was incubated in 1x RotiBlock at 0.04 µg/mL overnight at 4°C. After 3 washing steps with 1x TBS-T (TBS 20x, Biocave Medical, Cat. TWB945M; containing 0.1% Tween20, Sigma-Aldrich), membrane was incubated with HRP-conjugated anti-human H+L secondary antibody (Jackson ImmunoResearch, Cat.709-036-149,) for 1h at room temperature and washed 3 times with TBS-T. Detection of antibody NI-504.3E7 bound to membrane was performed with ECL (Amersham) for 2min and chemiluminescence signal was detected using Image Quant LAS 4000 (Amersham).

As shown in Figure 3, by using the overlapping peptide array, antibody NI-504.3E7 has been found to bind the two linear peptides 128 and 129 (SEQ ID NOs: 62 and 63) with the consensus sequences VTPASSAKTSP (SEQ ID NO: 65). Furthermore, the minimal epitope of antibody NI-504.3E7 comprises the amino acid sequence 516-ASSAK-520 (SEQ ID NO: 64).

### Example 4: NI-504.3E7 binds to non-phosphorylated CRMP2 and phosphorylated CRMP2

ELISA assays have been performed to determine the binding specificity of antibody NI-504.3E7 and of 40 variants thereof to CRMP2 and pCRMP2. The variants comprise one or more amino acid substitutions within the VH chain, the VL chain, and/or the CDRs. The ELISA assay has been performed as described in Example 2 with CRMP2 and pCRMP2. EC₅₀ values for antibody NI-504.3E7 as well as for the antibody variants N1-504.3E7_V1 to V40 are listed in Table VIII.

**Table VIII: Determination of the binding affinities of antibody NI-504.3E7 and variants thereof to full length recombinant CRMP2 and pCRMP2 by ELISA assay.**

| **Antibody** | **EC50 [nM] (CRMP2)** | **EC50 [nM] (pCRMP2)** |
|---|---|---|
| NI-504.3E7 | 0.5 | 4.7 |
| NI-504.3E7_V1 | 0.5 | 0.7 |
| NI-504.3E7_V2 | 0.3 | 0.8 |
| NI-504.3E7_V3 | 0.5 | 1.2 |
| NI-504.3E7_V4 | 0.5 | 1.3 |
| NI-504.3E7_V5 | 0.5 | 1.1 |
| NI-504.3E7_V6 | 0.5 | 1.5 |
| NI-504.3E7_V7 | 1 | 3.6 |
| NI-504.3E7_V8 | 0.5 | 1 |
| NI-504.3E7_V9 | 1.2 | 3.2 |
| NI-504.3E7_V10 | 0.9 | 2.2 |
| NI-504.3E7_V11 | 0.7 | 1.2 |
| NI-504.3E7_V12 | 0.6 | 1.9 |
| NI-504.3E7_V13 | 0.9 | 3 |
| NI-504.3E7_V14 | 0.6 | 2.5 |
| NI-504.3E7_V15 | 0.4 | 0.6 |
| NI-504.3E7_V16 | 0.3 | 0.7 |
| NI-504.3E7_V17 | 0.5 | 1.6 |
| NI-504.3E7_V18 | 0.5 | 1.5 |
| NI-504.3E7_V19 | 0.9 | 1.8 |
| NI-504.3E7_V20 | 1.2 | 13.7 |
| NI-504.3E7_V21 | 2.0 | 32.5 |
| NI-504.3E7_V22 | 2.3 | 47.0 |
| NI-504.3E7 V23 | 1.1 | 5.1 |
| NI-504.3E7_V24 | 1.4 | 46.4 |
| NI-504.3E7_V25 | 1.5 | 26.7 |
| NI-504.3E7_V26 | 0.6 | 3.5 |
| NI-504.3E7_V27 | 1.4 | 67.4 |
| NI-504.3E7_V28 | 1.1 | 8.1 |
| NI-504.3E7_V29 | 1.8 | 101.4 |
| NI-504.3E7_V30 | 0.2 | 0.6 |
| NI-504.3E7_V31 | 1.0 | 22.5 |
| NI-504.3E7_V32 | 0.3 | 0.9 |
| NI-504.3E7_V33 | 1.0 | 53.4 |
| NI-504.3E7_V34 | 1.6 | 8.9 |
| NI-504.3E7_V35 | 2.9 | 32.1 |
| NI-504.3E7_V36 | 2.8 | 99.9 |
| NI-504.3E7_V37 | 1.9 | 74.9 |
| NI-504.3E7_V38 | 1.2 | 52.4 |
| NI-504.3E7_V39 | 4.0 | 84.0 |
| NI-504.3E7_V40 | 4.1 | 89.2 |

As shown in Table VIII, antibody NI-504.3E7 specifically recognizes recombinant human full length CRMP2 and pCRMP2 with an EC₅₀ of 0.5 nM and 4.7 nM, respectively. As can be seen, all variants still bind to CRMP2 and pCRMP2, thus confirming that one or more amino acid substitutions do not substantially alter the binding affinity of the antibody.

### Example 5: NI-504.3E7 reduces the level of pCRMP2 in a concentration-dependent manner

To elucidate if the phosphorylation of CRMP2 is altered in the presence of increasing concentrations of the antibody of the present invention or an isotype control, a phosphorylation assay in combination with Western Blot analysis was performed. This assay has also been exemplarily performed with antibody NI-504.3E7_V20 to further support the fact that the variant antibodies retain the binding specificity and activity of the original antibody. In particular, this experimental setup was performed to observe the levels of T509 pCRMP2 (Figure 4 A-E), of T514 pCRMP2 (Figure 4 F-J), and of S522 pCRMP2 (Figure 4 K-O), when subjecting CRMP2 and either antibody NI-504.3E7_V20, or an anti-CRMP2 control antibody (NI-504.B) or an isotype control antibody to a phosphorylation assay.

The CRMP2 protein (Alexotech) was incubated for 1h in presence of different concentrations (3x less, equimolar or 3x more antibody compared to CRMP2) of CRMP2 antibodies (NI-504.3E7 and NI-504.B) as well as one human IgG control (isotype control antibody). Phosphorylation of CRMP2 was performed at 30°C for 1h or 24h using a phosphorylation mix containing phosphorylation buffer (50 mM Tris-HCl (Sigma-Aldrich), 10 mM MgCl2 (Sigma-Aldrich), 1.5 mM DTT (Sigma-Aldrich)), 0.25µg GSK3β (SignalChem), 0.25µg CDK5/p35 (SignalChem), and 0.7 mM ATP (SignalChem). Analysis of the phosphorylation levels of CRMP2 was performed via Western Blot. 200ng of phosphorylated CRMP2 protein was denatured in LDS sample buffer + 4% β-Mercapto-Ethanol (NuPAGE) for 10min at 95°C. Samples were loaded onto a 4-12% Bis-Tris Protein Gel (NuPAGE) and gel was run for 45min at 200V in 1x MES SDS Running Buffer (NuPAGE). Proteins were transferred onto nitrocellulose membranes (NuPAGE) using iBlot for 7min and 20V. Membranes were blocked with 2% BSA (Sigma-Aldrich) in PBS-T (phosphate buffered saline, Fisher Bioreagents, Cat.BP399; containing 0.1% Tween20 (Sigma-Aldrich)) overnight at 4°C. The next day, membranes were incubated with anti-CRMP2 antibody C2993 (Sigma-Aldrich), pCRMP2 (Thr-514) antibody (Cell Signaling, Cat.9397S), pCRMP2 (Thr-509) antibody (Thermo Fisher Scientific, Cat.PA5-37551), or pCRMP2 (Ser-522) antibody (ECM Bioscience, Cat.CP2191) for 1h at room temperature and washed 3 times with PBS-T. Membranes were incubated with HRP-conjugated goat anti-rabbit IgG (H+L) (Jackson ImmunoResearch, Cat.111-035-045) for 1h at room temperature. After 3 times washing with PBS-T, membranes were incubated with ECL (Amersham) for 1min and chemiluminescence signal was detected using Image Quant LAS 4000 (Amersham). Analysis of the signal band density was performed with the Java-based image processing program ImageJ.

The antibody C2993 was used to analyze samples for total CRMP2 levels to normalize the levels of pCRMP2, wherein it was shown that total CRMP2 levels were comparable in all samples as well as in the untreated loading control from Alexotech (200 ng/well). The graphs shown in Figure 4, C, E, H, J, M, and O have been normalized on those total CRMP2 levels.

As can be seen in the Figures, antibody NI-504.3E7_V20 reduces T509 pCRMP2 levels in a concentration-dependent manner after phosphorylation for 1h or 24h. Furthermore, antibody NI-504.3E7_V20 reduces T514 pCRMP2 levels after phosphorylation for 1h or 24h compared to antibody NI-504.B and the isotype control. Antibody NI-504.3E7_V20 reduces S522 pCRMP2 levels after phosphorylation for 1h in comparison to antibody NI-504.B and the isotype control. These results show that antibody NI-504.3E7_V20 reduces the level of pCRMP2 in a concentration-dependent manner. Furthermore, more repeats of this study have been performed, which confirm the results and also show concentration dependent reduction for S522 after 24h.

To ensure that the binding affinity of antibodies is not diminished or modified by the phosphorylation procedure for up to 24h, a separate phosphorylation experiment has been performed in the absence of CRMP2 protein. Antibody NI-504.3E7_V20, and antibody NI-504.B as well as isotype control antibodies have been incubated for 24h at standard phosphorylation conditions. Binding affinity was assessed with a direct CRMP2 ELISA. In conclusion, all antibodies display comparable binding before and after phosphorylation and, regarding their CRMP2 binding, are not affected by the phosphorylation procedure.

### Example 6: NI-504.3E7 specifically binds CRMP2 and pCRMP2 peptides

To assess if antibody NI-504.3E7 binds CRMP2 in its phosphorylated stage, four different peptides (Bachem) covering the antibody's epitope containing either no phosphorylation site or phosphorylation at specific sites (T509, T514, S522) have been tested in an ELISA format. The sequences of the peptides are the following:
Peptide 4152960: VCEVSVTPKTVTPASSAKTSPAKQQA (SEQ ID NO: 72)
Peptide 4152961: VCEVSV**(p)T₅₀₉**PKTVTPASSAKTSPAKQQA (SEQ ID NO: 72)
Peptide 4152962: VCEVSVTPKTV**(p)T₅₁₄**PASSAKTSPAKQQA (SEQ ID NO: 72)
Peptide 4152963: VCEVSVTPKTVTPASSAKT**(p)S₅₂₂**PAKQQA (SEQ ID NO: 72)

For direct ELISA, 96-well microplates (Costar) were coated with 30 µL/well of 5 µg/mL of each peptide in PBS and incubated overnight at 4°C. ELISA plates were blocked in 2% BSA (Sigma-Aldrich) in PBS-T (phosphate buffered saline, Fisher Bioreagents, Cat.BP399; containing 0.1% Tween20 (Sigma-Aldrich)) for 1h at room temperature, incubated with 30 µL/well serial dilutions of antibody NI-504.3E7 for 1h at room temperature, followed by 3 washing steps with PBS-T. Bound antibody was detected using HRP-conjugated anti-human Fc secondary antibody (Jackson ImmunoResearch, Cat. 709-036-098) and conversion of chromogenic substrate TMB (Clinical Science) for absorbance measurement at 450nm using a plate reader (Thermo Fisher Scientific). For estimation of half-maximal concentration EC₅₀ log transformed absorbance values were fitted using a log(agonist) vs. response - variable slope (four parameters) curve fitting applying formula Y=Bottom + (Top-Bottom)/(1+10^((LogEC50-X)^{∗}HillSlope)) was used in GraphPad Prism Version 9.3. Data is displayed as mean + SEM.

As shown in Figure 5, the affinity of antibody NI-504.3E7 to the CRMP2 peptide with phosphorylation at position T514 (Peptide 4152962) is ~300-fold lower than the non-phosphorylated peptide and the peptides with phosphorylation at positions T509 (Peptide 4152961) and S522 (Peptide 4152963). Phosphorylation at T514, seems to partially impair binding of antibody NI-504.3E7 potentially due to the antibody epitope.

### Example 7: NI-504.3E7 is capable of binding human, murine and rat recombinant CRMP2 full length protein

To determine the binding of antibody NI-504.3E7 on different species of CRMP2, 0.5µg of recombinant human SUMO-CRMP2 (Boston Biochem), recombinant mouse CRMP2-His₆ (MyBioSource, Cat.MBS11185591) and recombinant rat CRMP2-His₆ (MyBioSource, Cat.MBS961395) were denatured in LDS sample buffer + 4% β-Mercapto-Ethanol (NuPAGE) for 10min at 70°C. Samples were loaded onto a 4-12% Bis-Tris Protein Gel (NuPAGE) and gel was run for 60min at 200V in 1x MES SDS Running Buffer (NuPAGE). Proteins were transferred onto nitrocellulose membrane (NuPAGE) using iBlot for 7min and 20V. Membrane was blocked with 5% Milk in 1x TBS-T (TBS 20x, Biocave Medical, Cat. TWB945M; containing 0.1% Tween20, Sigma-Aldrich), for 1h at room temperature and antibody NI-504.3E7 was incubated in 1% Milk in TBS-T at 5 µg/mL overnight at 4°C. After 3 washing steps with TBS-T, membrane was incubated with HRP-conjugated anti-human H+L secondary antibody (Jackson ImmunoResearch, Cat.709-036-149) for 1h at room temperature and washed 3 times with TBS-T. Membrane was incubated with ECL (Amersham) for 2min and chemiluminescence signal was detected using Image Quant LAS 4000 (Amersham).

As shown in Figure 6, antibody NI-504.3E7 specifically binds all analyzed CRMP2 species, *i.e*., human, murine, and rat CRMP2 compared to the isotype IgG1 and human IgG1 control. Accordingly, antibody NI-504.3E7 is capable of binding human, murine and rat recombinant CRMP2 full length protein.

### Example 8: Co-aggregation of recombinant (p)Tau and (p)CRMP2 in a ThioT assay

Based on Takata, et al., Am J. Pathol. 175 (2009), 17-24 where it was shown in Figure 1 that hyperphosphorylated Tau was co-localized with phosphorylated CRMP2, a Thioflavin-T (ThioT, Sigma-Aldrich) assay was performed to detect whether recombinant (p)Tau and (p)CRMP2 proteins are not only co-localized but also co-aggregate. ThioT is known to bind to diverse fibrils, despite their distinct amino acid sequences, strongly suggesting that ThioT recognizes a structural feature common among fibrils. The ThioT assay measures changes of fluorescence intensity of ThioT upon binding to fibrils. It does not bind to the corresponding monomeric structures. In the present case, CRMP2, pCRMP2, Tau + pCRMP2, and pTau + pCRMP2 were subjected to the ThioT assay and the fluorescence signal was measured over 240h (exposure time was 5min). The Tau protein was obtained from rPeptide, Watkinsville, USA and human CRMP2 is a custom-made protein obtained from Boston Biochem, Inc. (Cambridge USA) including a SUMO tag at N-terminus, expressed in *E. coli*). In particular, amyloid fibril formation of CRMP2 (CRMP2, 6.4 µM, Boston Biochem), phosphorylated CRMP2 (pCRMP2, 6.4 µM), combined samples of Tau (Tau, 4.35 µM, Tau-441 full length protein (rPeptide, Cat.T-1001)) and phosphorylated CRMP2 (CRMP2, 6.4 µM), or phosphorylated Tau (pTau, 4.35 µM) and phosphorylated CRMP2 (pCRMP2, 6.4 µM) was measured by increase of ThioT fluorescence. For CRMP2 and pCRMP2 samples, 50 µM ThioT was used, while for the combined samples (Tau+pCRMP2, pTau+pCRMP2) 50 µM ThioT and 50 µM heparin were used. The aggregation assay was performed in plate format at room temperature, shaking at 300 rpm. Before each measurement, the plate was shaken for 5sec at 600 rpm, and fluorescence subsequently was measured at emission wavelength of 490nm after excitation at 456nm. Data is displayed as mean + SEM of duplicate values measured at indicated time points.

As can be seen in Figure 7, only weak binding of ThioT to CRMP2 (as determined by the ThioT fluorescence) was shown indicating that CRMP2 does not aggregate. Slightly higher binding of ThioT was observed during incubation of pCRMP2 and during incubation of pCRMP2 together with Tau the ThioT fluorescence signal increased, indicating that more aggregates were generated. The highest signal was obtained when incubating pCRMP2 together with pTau, showing that pCRMP2 and pTau co-aggregate.

### Example 9: Antibody NI-504.3E7 reverses the increases of spine density as induced by CRMP2 in a hippocampal slice culture model

The effect of antibody NI-504.3E7 on neuronal spine density driven by CRMP2/pCRMP2, *i.e.,* pCRMP2 aggregates and CRMP2 monomers has been analyzed in an *ex vivo* hippocampal slice culture (HSC) model. Organotypic hippocampal slices from C57BL/6J wildtype mouse pups 5 days postnatal (N=8), were prepared. Pups were decapitated, brain was removed, the two brain hemispheres separated, midbrain was dislodged, and hippocampus removed on both sides. Hippocampi were cut into slices of 400µm thickness with a tissue chopper. The experimental procedure was done according to publication Suendermann et al., Methods Mol Biol (2012), 277-293. Each condition was tested in duplicates. A total of 2 x 5 slices per condition were transferred into Multidish 6-well plates (Nunclon^{™}) containing culture medium and Millicell cell culture inserts (Millipore, Cat.PICM0RG50). Hippocampal slices were cultured, treated with the respective combination of target protein and antibody NI-504.3E7, infected, and fixed. Hippocampal slices were prepared on day 0, cultured for 10 days with medium exchange every 2-3 days, followed by treatment with pCRMP2 aggregates or CRMP2 monomers in combination with or without antibody NI-504.3E7 at the indicated concentrations. Antibody NI-504.3E7 was diluted in NB medium containing N1 supplement at day 10 for 4 days and at day 14 for additional 2 days after medium change and infection. Monomeric yeast CRMP2 was diluted in NB medium containing N1 supplement, while pCRMP2 aggregates were obtained after recombinant CRMP2 phosphorylation at 37°C for 1 day in phosphorylation buffer containing Tris-HCl (Sigma-Aldrich), MgCl2 (Sigma-Aldrich), DTT (Sigma-Aldrich), GSK3β (SignalChem), CDK5/p35 (SignalChem), and ATP (SignalChem). Aggregation of phosphorylated CRMP2 was performed in 5 mM Tris-HCl, pH 7.4 containing 5 mM heparin for 10 days at 1 mg/mL at room temperature.

On day 14, slice cultures were treated with the GFP Sinbis virus (Sindbis Expression System Manual, Invitrogen Cat.K750-01) applied with the droplet method with 1 µL/slice of the hSyn1-EGFP AAV9 construct diluted 1:3 from supernatant. On day 16, hippocampal slices were fixed with fixation solution (phosphate buffered saline (Gibco), 25% paraformaldehyde (Sigma-Aldrich, Cat.158127), 25% sucrose (Sigma-Aldrich)) for 2h at +4°C, subsequently mounted onto a glass slide, embedded in confocal matrix, and imaged by confocal laser scanning microscope Zeiss LSM 510. Semi-automated threshold analysis of spine density was performed by determining the number of spines per µm of dendrite length. A total of 29-58 images per treatment condition were analyzed, detecting between 11-52 spines on dendritic segments per image. Data is displayed as mean + SEM. Analysis by one-way ANOVA: *** p<0.01 ** p<0.01; Kruskal-Wallis-Test post-hoc vs. untreated.

As shown in Figure 8, neuronal spine density was increased by pCRMP2 aggregates or CRMP2 monomers in comparison to an untreated/aggregation buffer pool. This increase of spine density driven by CRMP2/pCRMP2 was reversed by antibody NI-504.3E7 at concentrations of 7.5 µM and 0.7 µM antibody. Accordingly, antibody NI-504.3E7 is capable of reversing the increase of spine density induced by aggregated pCRMP2 and monomeric CRMP2 in a hippocampal slice culture model.

### Example 10: NI-504.3E7 detects neuronal cells in AD hippocampal sections

The binding of antibody NI-504.3E7 on tissue sections was analyzed by immunohistochemistry (IHC). For the binding analysis of antibody NI-504.3E7 in human Alzheimer's disease (AD) tissue, deparaffinized and rehydrated FFPE brain sections from a human AD donor were boiled in citrate buffer (20mM Sodium citrate tribasic dihydrate, Sigma-Aldrich, Cat.C7129 and 80mM Sodium citrate tribasic dihydrate, Sigma-Aldrich, Cat.71405) for 15min, washed with water and endogenous peroxidase was blocked by incubating sections with methanol containing 3% hydrogen peroxide (Sigma-Aldrich), followed by additional washing with water. Sections were blocked in blocking buffer (5% goat serum (Vector Laboratories); 5% horse serum (Vector Laboratories); 4% bovine serum albumin (Sigma-Aldrich) in PBS (Gibco)) for 1h at room temperature. Antibody NI-504.3E7 was diluted in 2% BSA in PBS-T (phosphate buffered saline, Fisher Bioreagents, Cat.BP399; containing 0.1% Tween20 (Sigma-Aldrich)) at a concentration of 6.67 nM and incubated on sections overnight at 4°C, followed by 3 washing steps with water. Detection was performed using Biotin-SP AffiniPure goat anti-human IgG (H+L) (Jackson ImmunoResearch, Cat. 109-065-088) for 1h at room temperature, followed by the Vectastain ABC kit (Vector Laboratories) for 20min at room temperature, and diaminobenzidine (DAB, Thermo Fisher Scientific, Cat.1856090) and nuclei staining with hematoxylin (Roth, Cat.T865).

As shown in Figure 9, antibody NI-504.3E7 detects neuronal cells in the hippocampus of AD patients. Similar observations were made in the hippocampal tissue of patients with PSP, PiD, FTD-PiD, and FTLD-TDP, wherein the results are summarized in the following Table IX.

**Table IX: Analyses of histological binding of antibody NI-504.3E7 on tauopathy patient tissue.**

| | **Progressive Supranuclear Palsy** | **Pick's disease** | **Frontal Temporal Dementia and Pick's disease** | **Frontotemporal Lobar Degeneration -TDP** | **Alzheimer's Disease** |
|---|---|---|---|---|---|
| **NI-504.3E7** | uniform staining | cellular + uniform staining | cellular + strong uniform staining | weak cellular staining | cellular + weak uniform staining |

### Example 11: NI-504.3E7 reduces amyloid-beta plaques in a transgenic mouse model of Alzheimer's disease

The *in vivo* effect of antibody NI-504.3E7 has been analyzed in the Tau tubulin kinase-1 (TTBK1)/ amyloid precursor protein (APP) double transgenic mouse model for Alzheimer's disease. In particular, the *in vivo* efficacy of antibody NI-504.3E7 was assessed by weekly i.p. injections at 30 mg/kg into female APPPS1/TTBK1 transgenic mice (APPPS1 #034829-JAX, Jackson Laboratories; TTBK1 #B6/129-TTBK1-Tg Line 141, Tsuneya Ikezu, Nebraska University) for a period of 9 months (from 3 to 12 months of age). To allow repeated injections into the mouse model, a chimeric murine IgG2a form of the antibody was formulated in PBS and utilized for the injections. Antibody and vehicle (PBS) volume were adjusted by animal weight. At the end of their in-life phase, mice were transcardially perfused with ice-cold PBS, mouse brain was postfixed in paraffin and sagittal sectioning was performed at 3µm.

For Amyloid plaque analysis deparaffinized and rehydrated FFPE mouse brain sections, covering cortex and hippocampus, were boiled in citrate buffer for 15min and washed with water. Endogenous peroxidase was blocked by incubating sections with 3% hydrogen peroxide solution (Sigma-Aldrich), followed by additional washing with water. Sections were blocked in 5% goat serum (Vector Laboratories), 5% horse serum (Vector Laboratories) and 4% bovine serum albumin (Sigma-Aldrich) in PBS. Sections were subsequently incubated with anti-β-Amyloid antibody 6E10 (Biolegend) at 6.67 nM, followed by washing with water and detection by secondary antibody Biotin-SP AffiniPure Goat Anti-Mouse IgG (H+L) (Jackson ImmunoResearch, Cat.115-065-003) at 2.3 ug/ml in combination with the Vectastain ABC kit (Vector Laboratories), diaminobenzidine (Thermo Fisher Scientific, Cat.1856090) and nucleus staining with hematoxylin (Roth, Cat.T865). For image analysis, 3 sections of 3µm thickness located 60µm apart were used for detection. The plaque number and size in the depicted brain regions was assessed with threshold determination with the Java-based image processing program ImageJ. Amyloid-beta plaque load represents the % area covered by plaques stained with the respective antibody. Plaque size represents the average plaques size of all the plaques in the cortex or hippocampus. Data is displayed as mean + SEM. Group size: n=7, Statistical analysis: Unpaired T-test ≤ 0.0001.

As shown in Figure 10 A and C, mice receiving antibody NI-504.3E7 showed 63% reduced amyloid plaque load in cortex and hippocampus. Furthermore, not only the amyloid plaque load was reduced, but also the plaque size in the cortex and the hippocampus (Figure 10 B and C). In addition, in the cortex, a 50% reduction in the amount of plaques/area in female TTBK1/APP mice receiving antibody NI-504.3E7 was observed (area is defined by the whole cortex). The reduction in average plaque size in female TTBK1/APP mice receiving antibody NI-504.3E7 was 30% in the cortex (Figure 10B) and even 68% in the hippocampus (Figure 10D).

### Example 12: NI-504.3E7 is capable of binding CRMP2 in fully differentiated SH-SY5Y cells

To further analyze the mechanism of action of antibody NI-504.3E7, its binding in differentiated cells has been observed. For this analysis, SH-SY5Y cells were maintained in MEM medium (Thermo Fisher Scientific, Cat.41090028) supplemented with 1% MEM NEAA (Gibco), 10mM HEPES (Gibco), 1mM Sodium pyruvate (Gibco), 1% Penicillin-Streptomycin (Gibco) and 10% heat-inactivated FBS (Gibco). Differentiation of SH-SY5Y cell was performed in two steps, with a 7-day pre-differentiation, during which 10µM10 µM all-trans retinoic acid (Sigma-Aldrich, Cat.R2625) was added to the medium. Medium was changed every 2-3 days. Pre-differentiated cells were plated on Poly-D-Lysine coated 8-well 15µ-Slides (Ibidi, Cat.80825) at a density of 50'000 cells/well and cultured for full differentiation into MEM medium (Thermo Fisher Scientific, Cat.41090028) supplemented with 1% MEM NEAA (Gibco), 10mM HEPES (Gibco), 1mM Sodium pyruvate (Gibco), 1% Penicillin-Streptomycin (Gibco), 50 ng/ml BDNF (Sigma-Aldrich, Cat. SRP3014), 10 ng/ml NGF (RnD Systems) and 24 nM Cholecalciferol (Sigma-Aldrich, Cat.C9756) for 10 days. Medium was changed every 3-4 days. Fully differentiated cells were washed with PBS and fixed with 4% PFA (Sigma-Aldrich) for 20min at room temperature. After 2 washing steps with PBS, cells were permeabilized and blocked for 1h in PBS containing 10% FBS and 0.2% Triton-X-100 (Sigma-Aldrich). Primary antibody incubation was performed overnight at 4°C with 50 nM human IgG control (isotype control antibody), 50 nM NI-504.3E7, 1 µg/ml anti-β-III-tubulin (Abcam, Cat.ab78078) or 1% anti-CRMP2 (Abcam, Cat.ab129082) diluted in blocking buffer (PBS containing 10% FBS). After 2 washing steps with PBS, cells were stained for 1h at room temperature with secondary antibodies anti-mouse-Cy3 (Jackson ImmunoResearch, Cat.715-165-150), anti-rabbit-Cy3 (Jackson ImmunoResearch, Cat.711-165-152) or anti-human-Cy3 (Jackson ImmunoResearch, Cat.709-165-149) at 2.7 ug/ml each in blocking buffer. Nuclei were stained for 5min with DAPI (Thermo Fisher Scientific) in PBS and imaging was performed by inverted confocal microscopy (20x/dry objective, Leica DMI6000 AFC, Model SP8).

As shown in Figure 11, antibody NI-504.3E7 binds differentiated SH-SY5Y cells, wherein no signal was observed with human IgG control and with an anti-human control. These results demonstrate target, *i.e.,* CRMP2, engagement in the cells.

### Example 13: NI-504.3E7 improves Long-Term Potentiation (LTP) in aged mice

In addition of its capability of reducing amyloid plaque load in a transgenic mouse model of Alzheimer's disease as shown in Example 11, the ability of the NI-504.3E7 antibody family to improve long term potential (LTP) has been confirmed by one of its variants. In particular, like the phosphorylation assay described in Example 5, the present assay has also been exemplarily performed with antibody NI-504.3E7_V20 to further support the fact that the variant antibodies retain the activity of the original antibody.

The effect of antibody NI-504.3E7_V20 has been analyzed in aged C57B1/6J male mice (90 weeks-old). A chimeric murine IgG2a form of the antibody was formulated in PBS and used for injections. Antibody and vehicle (PBS) volume were adjusted by animal weight and intraperitoneal injections were delivered once a week for 5 weeks, wherein different doses of the antibody were delivered, *i.e.,* 1 mg/kg, 3 mg/kg, 10 mg/kg, and 30 mg/kg. At the end of their in-life phase, mice were anesthetized with 5% isoflurane and then decapitated. The brain was dissected out of the cranium and immediately immersed in ice-cold freshly prepared artificial cerebrospinal fluid (aCSF) containing: 124 mM NaCl, 3.75 mM KCl, 2 mM MgSO₄, 2 mM CaCl₂, 26.5 mM NaHCO₃, 1.25 mM NaH₂PO₄, 10 mM glucose, continuously oxygenated (95% O₂, 5% CO₂) (pH 7.4) for a total duration of 3-4 minutes. Acute slices (350 µm thick) were prepared using a vibratome (VT 1000S; Leica Microsystems, Bannockburn, IL). Sections were incubated in standard aCSF (124 mM NaCl, 3.75 mM KCl, 2 mM MgSO₄, 2 mM CaCl₂, 26.5 mM NaHCO₃, 1.25 mM NaH₂PO₄, 10 mM glucose) at room temperature for at least 1h before recordings. For electrophysiological recordings, a single slice was placed in the recording chamber (room temperature), submerged and continuously superfused with gassed aCSF (95% O₂, 5% CO₂; pH 7.4) at a constant rate (2 mL min⁻¹) for the remaining experiment. Extracellular field excitatory postsynaptic potentials (fEPSPs) were recorded in the CA1 stratum radiatum using a glass micropipette filled with aCSF. fEPSPs were evoked by the electrical stimulation of Schaffer collateral-commissural pathway at 0.1 Hz *(i.e.,* a single pulse every 10 s) with a glass stimulating electrode (borosilicate capillary glass with filament, standard wall; OD:1.5 mm; ID:0.86 mm; Length: 75 mm; ref: W3 30-0060 from Harvard Apparatus) placed in the stratum radiatum. Stable baseline fEPSPs were then recorded during 10 minutes by stimulating at 30 % of maximal field amplitude (single pulse every 10 s, *i.e.,* 0,1 Hz). After 10 min stablilization, a conditioning stimulus consisting in 1 train of 100-Hz stimulation was delivered to assess LTP. Following the conditioning stimulus, a 1 h test period was recorded where responses were again elicited by a single stimulation every 10 s (0,. Hz) at the same stimulus intensity. Signals were amplified with an Axopatch 200B amplifier (Molecular Devices, Union City, CA) digitized by a Digidata 1322A interface (Axon Instruments, Molecular Devices, US) and sampled at 10 kHz. Recordings were acquired using Clampex (Molecular Devices) and analyzed with Clampfit (Molecular Devices). LTP was calculated as percent of baseline fEPSP slope recorded over a 10-minute period before LTP induction. The average slope response during 40-60 min post-LTP induction was used for analysis. Data is displayed as mean + SEM. Group size: n=8, Statistical analysis: two-way ANOVA, * p<0.05, *** p<0.001 vs PBS group.

As shown in Figure 12, in aged mice, the LTP was decreased compared to the young mice; see PBS control. However, after administering the antibody to the aged mice, the LTP was increased in comparison to the LTP of the PBS control and nearly reached the score of the LTP in young mice, in particular after administration of 3 mg/kg, 10 mg/kg and 30 mg/kg of the antibody. Thus, it has been shown that antibody NI-504.3E7_V20 improved the LTP in aged mice already at a dose of 3 mg/kg.

### Example 14: NI-504.3E7 improves cognitive function in aged mice

In addition of its capability of reducing amyloid plaque load in a transgenic mouse model of Alzheimer's disease as shown in Example 11, and its capability to improve LTP in aged mice as shown in Example 13, the ability of the NI-504.3E7 antibody family to improve cognitive function in aged mice has been confirmed by one of its variants. In particular, like the phosphorylation assay described in Example 5, and the LTP assay as described in Example 13, the present assay has also been exemplarily performed with antibody NI-504.3E7_V20 to further support the fact that the variant antibodies retain the activity of the original antibody.

The effect of antibody NI-504.3E7_V20 on the cognitive function has been analyzed in aged C57B1/6J male mice. Seven-week-old (young) and 73-week-old (aged) mice were used for the experiment. Antibody and vehicle (PBS) were intraperitoneally injected once a week for 5 weeks. Cognitive function was evaluated using the novel object recognition test (NOR). During the acclimation to the experimental apparatus on the first day, each mouse was placed in a test chamber and was allowed free moving for 10 min and, thereafter, returned to the home cage. The acclimation was repeated twice with an interval of at least 4 hours. In the acquisition trial on the second day, two objects with the same color and shape were placed in a test chamber, and each mouse was similarly allowed free moving for 10 min. In the evaluation trial on the third day, one of the objects used for the acquisition trial (familiar objects) was replaced with a novel object with different color and shape, and each mouse was similarly allowed free moving for 10 min and recorded using a video camera. The evaluation trial was initiated at 24 h after acquisition trial. Based on the video image recorded during the evaluation trial, the time spent exploring each object was measured using a stopwatch. A discrimination index was calculated using the following formula: (time exploring the novel object - time exploring the familiar object)/ (time exploring the novel object + time exploring the familiar object). Data is displayed as mean + SEM. Group size: n=11 to 12, Statistical analysis: ** p <0.01 compared to the Young group (t-test), ### p <0.001 compared to the PBS group (Dunnett test).

As shown in Figure 13, in aged mice, the discrimination index was decreased compared to the young mice; see PBS control. However, after administering the antibody at doses of 1 mg/kg, 3 mg/kg, and 10 mg/kg, the discrimination index was significantly increased. Thus, it has been shown that antibody NI-504.3E7_V20 improved the discrimination index in aged mice already at 1 mg/kg and these results demonstrate that antibody NI-504.3E7 improves the cognitive function in aged mice.

## Claims

1. A human-derived monoclonal antibody or antigen-binding fragment thereof that selectively binds to human Collapsin Response Mediator Protein 2 (CRMP2), wherein the antibody binds to an epitope which comprises the amino acid sequence ASSAK (SEQ ID NO: 64), preferably wherein the antibody is capable of binding CRMP2 peptides which consist of the amino acid sequence TPKTVTPASSAKTSP (SEQ ID NO: 62) or VTPASSAKTSPAKQQ (SEQ ID NO: 63).

2. The antibody or antigen-binding fragment of claim 1, wherein the antibody is capable of binding full length non-phosphorylated CRMP2 and phosphorylated CRMP2 (pCRMP2), preferably wherein the antibody is capable of reducing the level of pCRMP2 in a concentration-dependent manner when subjecting the antibody and CRMP2 to a phosphorylation assay and/or wherein the antibody is capable of binding human, murine and rat recombinant CRMP2 full length protein, preferably wherein the antibody binds to full length human CRMP2 with an EC50 of about ≤ 10.0 nM and/or pCRMP2 with an EC50 of about ≤ 100.0 nM as determined by ELISA.

3. A human-derived monoclonal antibody or antigen-binding fragment thereof that selectively binds to human Collapsin Response Mediator Protein 2 (CRMP2) and which comprises in its variable region a heavy chain variable (VH) region and a light chain variable (VL) region, wherein the VH region comprises complementarity determining regions (CDRs) 1, 2, and 3 and the VL region comprises CDRs 1, 2, and 3, wherein
(a) VH-CDR1 comprises the amino acid sequence of SEQ ID NO: 3 as shown in Table V,
(b) VH-CDR2 comprises the amino acid sequence of SEQ ID NO: 4 as shown in Table V,
(c) VH-CDR3 comprises the amino acid sequence of SEQ ID NO: 5 as shown in Table V,
(d) VL-CDR1 comprises the amino acid sequence of SEQ ID NO: 8 as shown in Table V,
(e) VL-CDR2 comprises the amino acid sequence of SEQ ID NO: 9 as shown in Table V, and
(f) VL-CDR3 comprises the amino acid sequence of SEQ ID NO: 10 or 61 as shown in Table V; or
wherein one or more of the CDRs may comprise one or more amino acid substitutions, preferably wherein the antibody or antigen-binding fragment comprises in its variable region an amino acid sequence of the VH region and the VL region selected from
(0) SEQ ID NO: 2 and SEQ ID NO: 7,
(1) SEQ ID NO: 2 and SEQ ID NO: 56,
(2) SEQ ID NO: 2 and SEQ ID NO: 58,
(3) SEQ ID NO: 12 and SEQ ID NO: 7,
(4) SEQ ID NO: 12 and SEQ ID NO: 56,
(5) SEQ ID NO: 12 and SEQ ID NO: 58,
(6) SEQ ID NO: 14 and SEQ ID NO: 7,
(7) SEQ ID NO: 14 and SEQ ID NO: 56,
(8) SEQ ID NO: 14 and SEQ ID NO: 58,
(9) SEQ ID NO: 16 and SEQ ID NO: 7,
(10) SEQ ID NO: 16 and SEQ ID NO: 56,
(11) SEQ ID NO: 16 and SEQ ID NO: 58,
(12) SEQ ID NO: 18 and SEQ ID NO: 7,
(13) SEQ ID NO: 18 and SEQ ID NO: 56,
(14) SEQ ID NO: 18 and SEQ ID NO: 58,
(15) SEQ ID NO: 18 and SEQ ID NO: 60,
(16) SEQ ID NO: 20 and SEQ ID NO: 7,
(17) SEQ ID NO: 20 and SEQ ID NO: 56,
(18) SEQ ID NO: 20 and SEQ ID NO: 58,
(19) SEQ ID NO: 20 and SEQ ID NO: 60,
(20) SEQ ID NO: 22 and SEQ ID NO: 58,
(21) SEQ ID NO: 24 and SEQ ID NO: 58,
(22) SEQ ID NO: 26 and SEQ ID NO: 58,
(23) SEQ ID NO: 28 and SEQ ID NO: 58,
(24) SEQ ID NO: 30 and SEQ ID NO: 58,
(25) SEQ ID NO: 32 and SEQ ID NO: 58,
(26) SEQ ID NO: 34 and SEQ ID NO: 58,
(27) SEQ ID NO: 36 and SEQ ID NO: 58,
(28) SEQ ID NO: 38 and SEQ ID NO: 58,
(29) SEQ ID NO: 40 and SEQ ID NO: 58,
(30) SEQ ID NO: 42 and SEQ ID NO: 58,
(31) SEQ ID NO: 44 and SEQ ID NO: 58,
(32) SEQ ID NO: 46 and SEQ ID NO: 58,
(33) SEQ ID NO: 48 and SEQ ID NO: 58,
(34) SEQ ID NO: 50 and SEQ ID NO: 58,
(35) SEQ ID NO: 22 and SEQ ID NO: 56,
(36) SEQ ID NO: 22 and SEQ ID NO: 60,
(37) SEQ ID NO: 52 and SEQ ID NO: 56,
(38) SEQ ID NO: 52 and SEQ ID NO: 58,
(39) SEQ ID NO: 52 and SEQ ID NO: 60, and
(40) SEQ ID NO: 54and SEQ ID NO: 58, or
a pair of VH and VL amino acid sequences, wherein the VH and/or VL may comprise one or more amino acid substitutions and/or deletions, preferably wherein the VH and/or VL are at least 90% identical to the amino acid sequences of any one of (0) to (40).

4. The antibody or antigen-binding fragment of any one of claims 1 to 3 which is capable of reversing the increase of neuronal spine density induced by CRMP2 as determined in an *ex vivo* hippocampal slice culture model, preferably wherein neuronal spine density is increased by pCRMP2 aggregates or CRMP2 monomers.

5. The antibody or antigen-binding fragment of any one of claims 1 to 4, wherein the antibody or antigen-binding fragment
(a) comprises a constant region, preferably wherein the constant region is of the IgG type, preferably of the IgG1 class or isotype;
(b) comprises a heterologous polypeptide, optionally wherein the antibody or antigen binding fragment is a chimeric murine-human antibody, preferably wherein the antibody comprises murine IgG2a constant region; and/or
(c) is selected from the group consisting of a single chain Fv fragment (scFv), an F(ab') fragment, an F(ab) fragment, and an F(ab')₂ fragment, an Fd, an Fv, a single-chain antibody, a disulfide-stabilized Fv (dsFv), and a nanobody.

6. An antibody or an antigen-binding molecule which competes with an antibody of any one of claims 1 to 5 for specific binding to human CRMP2 and/or pCRMP2, wherein the antibody
- preferentially recognizes human CRMP2 over CRMP1, CRMP3, CRMP4 and CRMP5,
- is capable of reducing the level of phosphorylated CRMP2 (pCRMP2) levels in a concentration-dependent manner when subjecting CRMP2 to a phosphorylation assay, and/or
- is capable of reversing the increase of neuronal spine density induced by CRMP2 as determined in an *ex vivo* hippocampal slice culture model.

7. One or more polynucleotide(s) encoding the antibody or antigen-binding fragment of any one of claims 1 to 6 or an immunoglobulin VH or VL thereof, preferably wherein the polynucleotide is a cDNA and/or operably linked to a heterologous nucleic acid, preferably expression control sequences.

8. One or more vector(s) comprising the polynucleotide(s) of claim 7.

9. A host cell comprising the polynucleotide(s) of claim 7 or the vector(s) of claim 8.

10. A method for preparing an anti-CRMP2 antibody, or an antigen-binding fragment or immunoglobulin chains thereof, said method comprising:
(a) culturing the cell of claim 9; and
(b) isolating the antibody, or antigen-binding fragment or immunoglobulin chain(s) thereof from the culture.

11. An antibody, antigen-binding fragment or immunoglobulin chain encoded by the polynucleotide(s) of claim 9, or obtainable by the method of claim 10.

12. The antibody or antigen-binding fragment of any one of claims 1 to 6 or 11, which
(i) is detectably labeled with a label selected from the group consisting of an enzyme, a radioisotope, a fluorescent compound, a chemiluminescent compound, a bioluminescent compound, a tag, a flag and a heavy metal;
(ii) is attached to a drug;
(iii) comprises polyethylene glycol;
(iv) comprises an organ specific targeting entity; and/or
(v) is contained in or conjugated to a vehicle such as an exosome or nanoparticle for delivery to a specific organ.

13. A peptide having an epitope of (p)CRMP2 specifically recognized by an antibody of any one of the claims 1 to 6 or 11, wherein the peptide consists of 10 to 25 contiguous amino acids of CRMP2 and comprises the amino acid sequence ASSAK (SEQ ID NO: 64), optionally wherein the peptide further comprises a heterologous amino acid sequence or functional moiety.

14. A composition comprising the antibody or antigen-binding fragment of any one of claims 1 to 6, 11 or 12, the polynucleotide(s) of claim 7, the vector(s) of claim 8 or the host cell of claim 9, preferably wherein the composition is:
(i) a pharmaceutical composition and further comprises a pharmaceutical acceptable carrier; or
(ii) a diagnostic composition and designed as a kit, optionally further comprising reagents conventionally used in immuno-based diagnostic methods.

15. The antibody or antigen-binding fragment of any one of claims 1 to 6, 11 or 12, or the composition of claim 14, for use in a method of treating a neurodegenerative disorder, such as Alzheimer's disease and tauopathy.

16. A molecule capable of interfering with Collapsin Response Mediator Protein 2 (CRMP2) signaling for use as a medicament, preferably for use in a method for the prevention, delay of progression or the treatment of a neurodegenerative disorder, cognitive disorder and/or for improving memory and learning ability.
